(19) Europäisches Patentamt
European Patent Office
Office européen des brevets

(11) EP 3 519 417 B1

(12) # EUROPÄISCHE PATENTSCHRIFT

(45) Veröffentlichungstag und Bekanntmachung des Hinweises auf die Patenterteilung:
**08.09.2021 Patentblatt 2021/36**

(21) Anmeldenummer: **17781424.1**

(22) Anmeldetag: **28.09.2017**

(51) Int Cl.:
***C07D 491/04*** (2006.01) ***C07D 495/04*** (2006.01)
***H01L 51/50*** (2006.01)

(86) Internationale Anmeldenummer:
**PCT/EP2017/074585**

(87) Internationale Veröffentlichungsnummer:
**WO 2018/060307 (05.04.2018 Gazette 2018/14)**

(54) **VERBINDUNGEN MIT DIAZADIBENZOFURAN- ODER DIAZADIBENZOTHIOPHEN-STRUKTUREN**

COMPOUNDS WITH DIAZADIBENZOFURANE OR DIAZADIBENZOTHIOPHENE STRUCTURES

COMPOSÉS COMPRENANT DES STRUCTURES DIAZADIBENZOFURANE OU DIAZADIBENZOTHIOPHÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2016 EP 16191703**

(43) Veröffentlichungstag der Anmeldung:
**07.08.2019 Patentblatt 2019/32**

(73) Patentinhaber: **Merck Patent GmbH**
**64293 Darmstadt (DE)**

(72) Erfinder:
- **PARHAM, Amir**
  **60486 Frankfurt am Main (DE)**
- **EBERLE, Thomas**
  **76829 Landau (DE)**
- **JATSCH, Anja**
  **60489 Frankfurt am Main (DE)**
- **GROSSMANN, Tobias**
  **64297 Darmstadt (DE)**
- **KROEBER, Jonas**
  **60311 Frankfurt am Main (DE)**

(56) Entgegenhaltungen:
**JP-A- 2011 084 531 US-A1- 2015 207 082**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 519 417 B1

## Beschreibung

Die vorliegende Erfindung beschreibt Diazadibenzofuran- oder Diazadibenzothiophenderivate, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen sowie als Elektronentransportmaterialien häufig heteroaromatische Verbindungen eingesetzt, wie zum Beispiel Diazadibenzofuranderivate. Ferner werden als Matrixmaterialien auch Carbazolderivate verwendet. Bekannt für diese Funktion sind beispielsweise Diazadibenzofuranderivate, welche mit Carbazolgruppen substituiert sind, wie in JP 5604848 B2, WO 2015/182872 A1 offenbart. Ferner sind in WO 2014/157599 A1 und WO 2015/182872 A1 Diazadibenzofuranderivate beschrieben, die mit Fluoren- Phenanthren-, Triphenylengruppen oder Dibenzothiophengruppen substituiert sind. Allerdings zeigen die Diazadibenzofurangruppen nicht zwingend eine doppelte Substitution in dem Diazaphenylrest der Diazadibenzofurangruppe mit Aryl- oder Heteroarylgruppen. Ferner zeigen einige der dargelegten Verbindungen zwei Diazaphenylreste in der Diazadibenzofurangruppe.

US2015/0207082 A1, JP2011084531A und EP3056498 A1 offenbaren Azadibenzofurane und Azadibenzothiophene und deren Verwendung in elektronichen Vorrichtungen.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen.

Ferner kann eine weitere Aufgabe der vorliegenden Erfindung darin gesehen werden, Verbindungen bereitzustellen, welche sich für den Einsatz in einer phosphoreszierenden oder fluoreszierenden OLED insbesondere als Elektronentransportmaterialien eignen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausfürhungsformen sind daher Gegenstand der vorliegenden Erfindung.

Gegenstand der vorliegenden Erfindung ist daher eine Verbindung, gemäß der folgenden Formel (A),

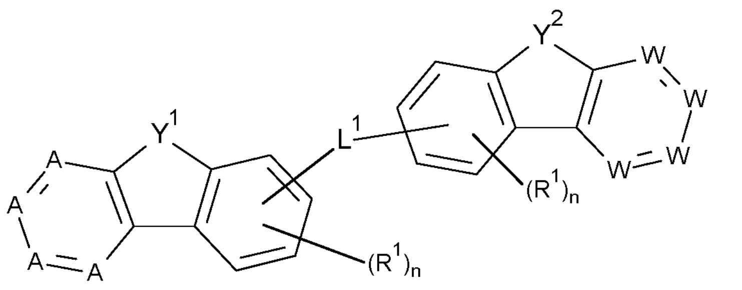

Formel (A)

wobei für die verwendeten Symbole gilt:

$Y^1$ ist O oder S;

$Y^2$ ist N(Ar), O, oder $C(R^1)_2$ ;

W ist bei jedem Auftreten gleich oder verschieden N oder $CR^1$, vorzugsweise $CR^1$, mit der Maßgabe, dass nicht mehr als zwei der Gruppen W in einem Cyclus für N stehen;

$L^1$ ist eine Bindung oder ein aromatisches oder heteroaromatisches Ringsystem mit 5 bis 30 aromatischen Ringatomen, welches durch einen oder mehrere Reste $R^1$ substituiert sein kann;

A ist bei jedem Auftreten gleich oder verschieden N, $CAr^8$ oder $CAr^b$, wobei genau zwei A für N stehen, die durch mindestens eine Gruppe $CAr^8$ oder $CAr^b$ getrennt sind, mit der Maßgabe, dass A für $CAr^b$ steht, falls zu diesem A zwei N benachbart sind;

Ar ist bei jedem Auftreten gleich oder verschieden ein aromatisches oder heteroaromatisches Ringsystem mit 5 bis 30 aromatischen Ringatomen, welches durch einen oder mehrere Reste $R^1$ substi-tuiert sein kann;

$Ar^a$ ist bei jedem Auftreten gleich oder verschieden ein aromatisches oder heteroaromatisches Ringsystem mit 5 bis 30 aromatischen Ringatomen, welches durch einen oder mehrere Reste $R^1$ substituiert sein kann;

$Ar^b$ ist bei jedem Auftreten gleich oder verschieden ein aromatisches oder heteroaromatisches Ringsystem mit 5 bis 30 aromatischen Ringatomen, welches durch einen oder mehrere Reste $R^1$ substituiert sein kann;

$R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere Substituenten $R^1$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis

30 aromatischen Ringatomen, das mit einem oder mehreren nicht-aromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

n ist 0, 1, 2 oder 3, vorzugsweise 0, 1 oder 2, besonders bevorzugt 0 oder 1, speziell bevorzugt 0;

mit der Maßgabe, dass
falls die Gruppe $Y^2$ N(Ar) oder O darstellt, der Rest $Ar^a$ keine Carbazol-Gruppe umfasst, wobei dies Substituenten $R^1$, $R^2$ und $R^3$, die an den Rest $Ar^a$ gebunden sein können, einschließt.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

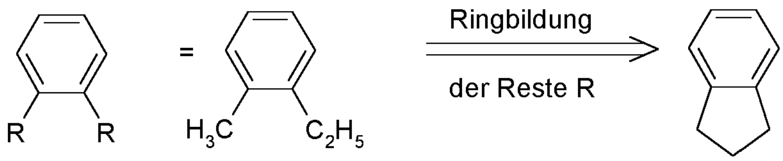


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

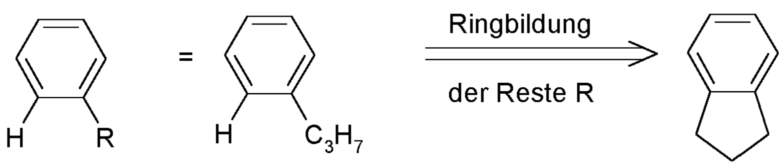


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausgestaltung können die erfindungsgemäßen Verbindungen eine Struktur gemäß der Formel (I), (II) oder (III) aufweisen

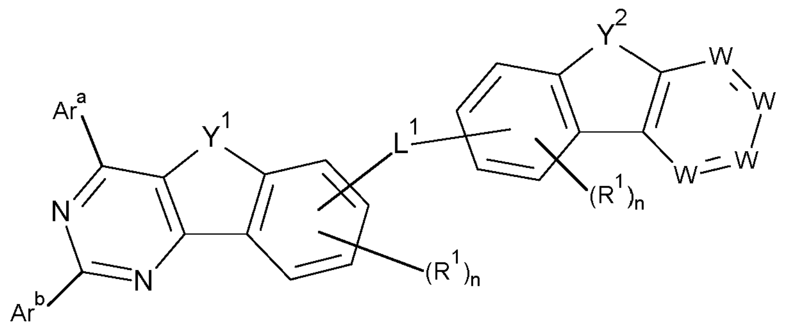

Formel (I)

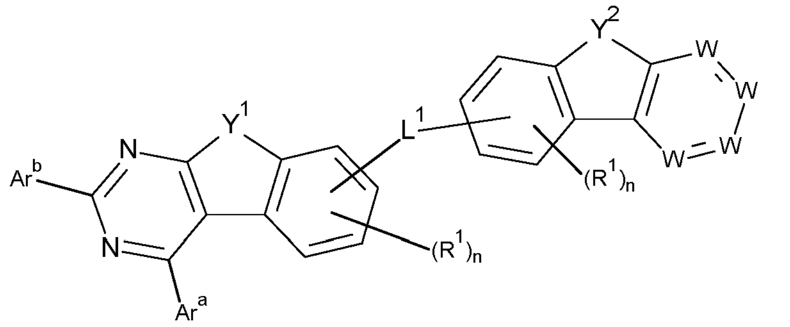

Formel (II)

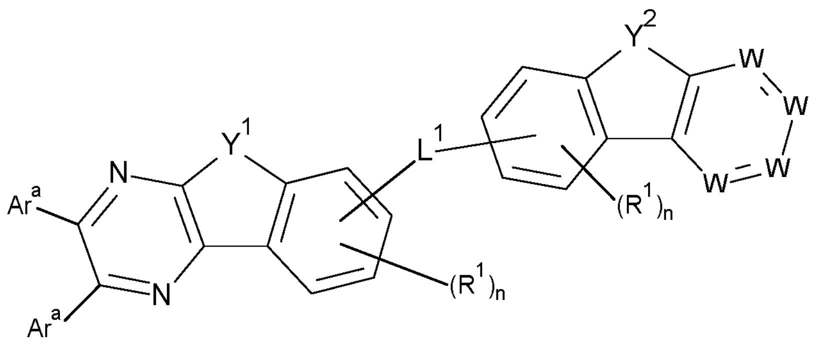

Formel (III)

worin die Symbole $Ar^a$, $Ar^b$, $Y^1$, $L^1$, $Y^2$, $R^1$, n und W die zuvor für Formel (A) dargelegte Bedeutung aufweisen. Hierbei sind Verbindungen mit Strukturen der Formeln (I) und/oder (II) bevorzugt.

Vorzugsweise weisen die erfindungsgemäßen Verbindungen Strukturen gemäß Formeln (Ia), (IIa) und/oder (IIIa) auf

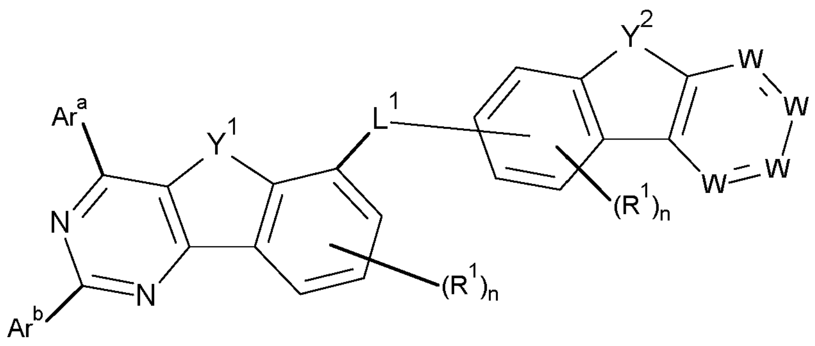

Formel (Ia)

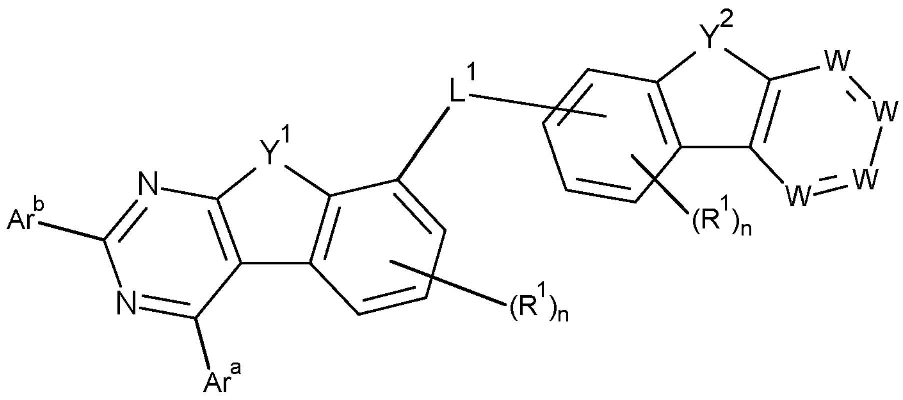

Formel (IIa)

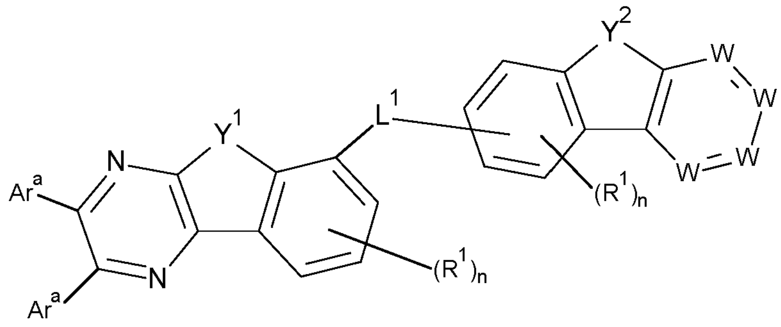

Formel (IIIa)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Ia) und/oder (IIa) bevorzugt sind.

Vorzugsweise weisen die erfindungsgemäßen Verbindungen Strukturen gemäß einer der Formeln (Ib), (IIb) und/oder (IIIb) auf

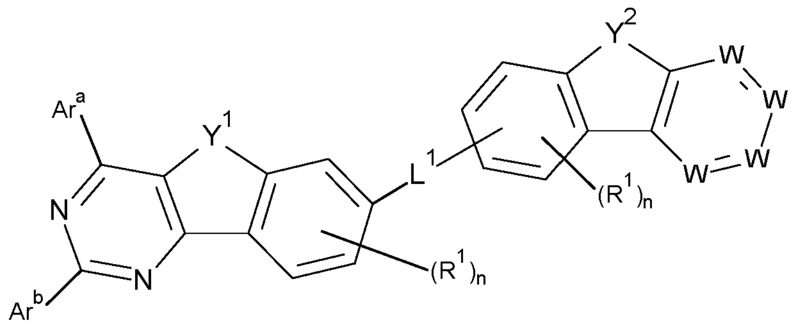

Formel (Ib)

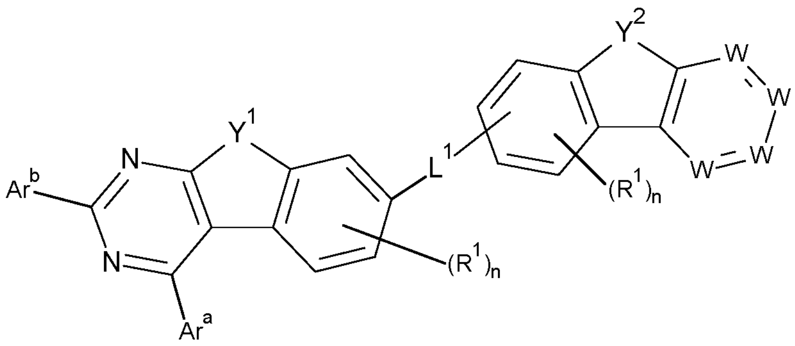

Formel (IIb)

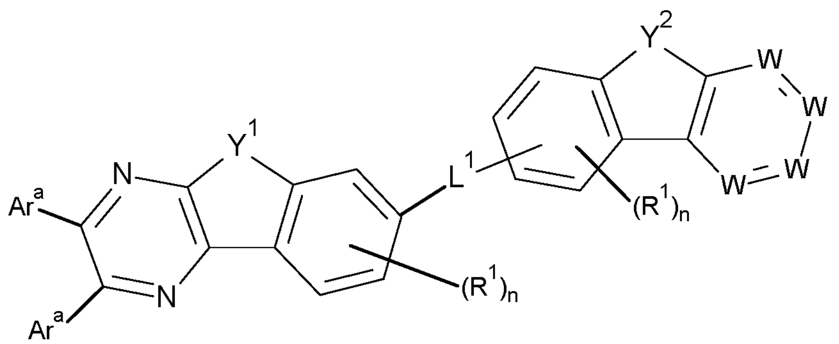

Formel (IIIb)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Ib) und/oder (IIb) bevorzugt sind.

Vorzugsweise weisen die erfindungsgemäßen Verbindungen Strukturen der Formeln (Ic), (IIc) und/oder (IIIc) auf

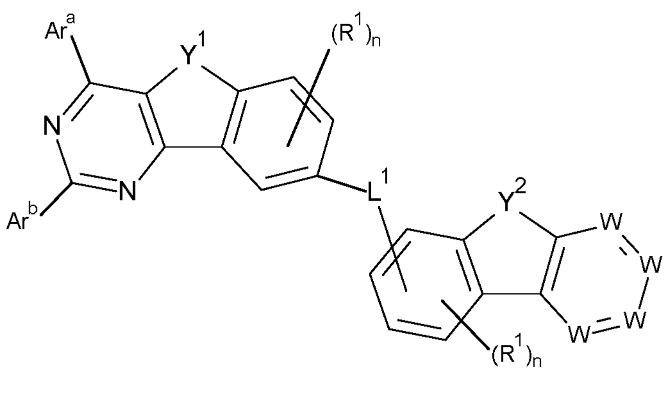

Formel (Ic)

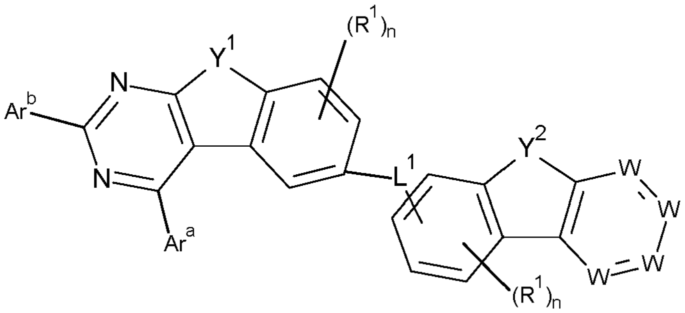

Formel (IIc)

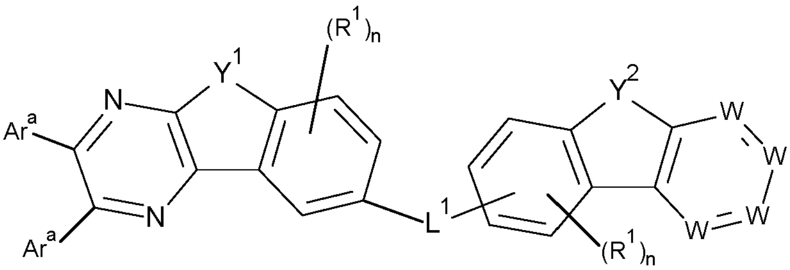

Formel (IIIc)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Ic) und/oder (IIc) bevorzugt sind.

Ferner kann vorgesehen sein, dass dass die Verbindung eine der Strukturen der Formeln (Id), (IId) und/oder (IIId) aufweist

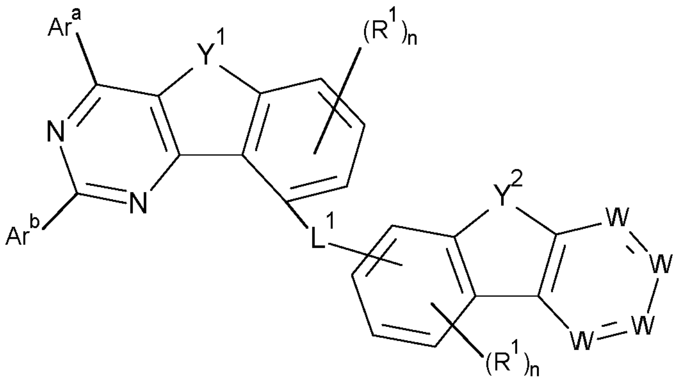

Formel (Id)

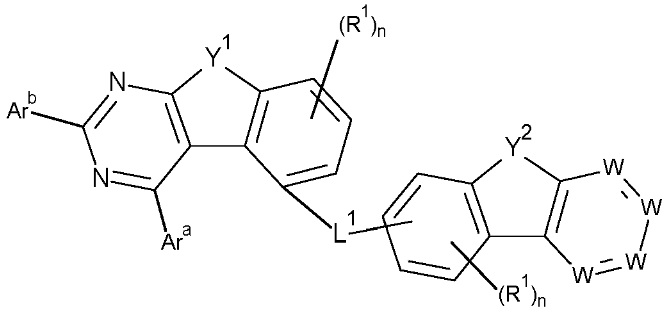

Formel (IId)

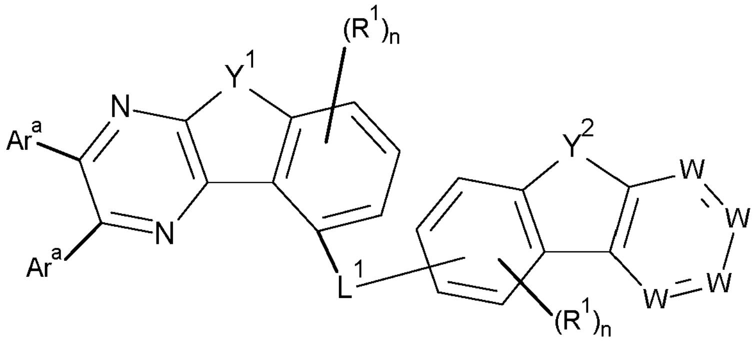

Formel (IIId)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Id) und/oder (IId) bevorzugt sind.

Von den zuvor dargelegten Verbindungen gemäß der Formeln (Ia) bis (IIId) sind solche Verbindungen bevorzugt, die die Strukturen der Formeln (Ic), (IIc), (IIIc), (Id), (IId) und/oder (IIId) enthalten, wobei Verbindungen mit Strukturen der Formeln (Ic), (IIc) und/oder (IIIc) besonders bevorzugt sind.

Weiterhin kann vorgesehen sein, dass dass die Verbindung eine der Strukturen der Formeln (Ie), (IIe) und/oder (IIIe) aufweist

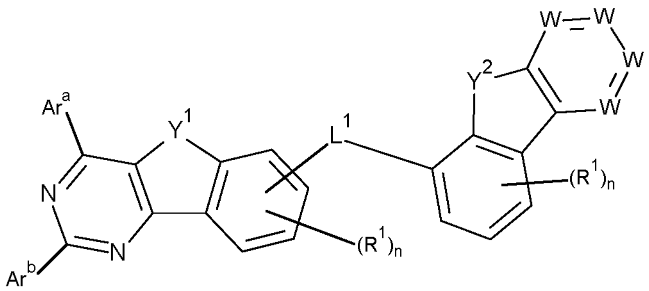

Formel (Ie)

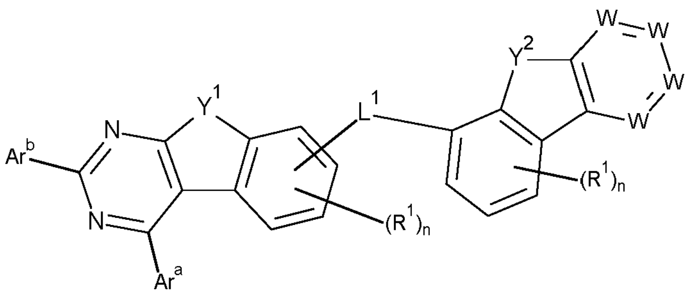

Formel (IIe)

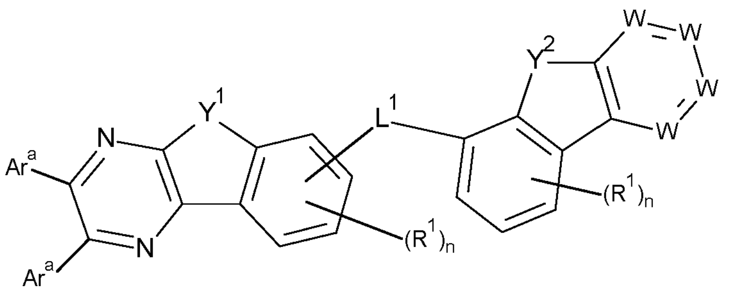

Formel (IIIe)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Ie) und/oder (IIe) bevorzugt sind.

Darüber hinaus kann vorgesehen sein, dass die Verbindung eine der Strukturen der Formeln (If), (IIf) und/oder (IIIf) aufweist

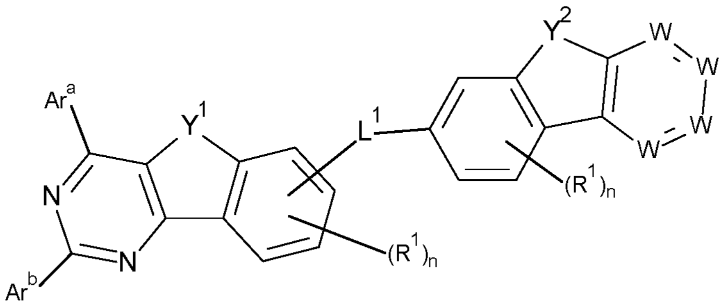

Formel (If)

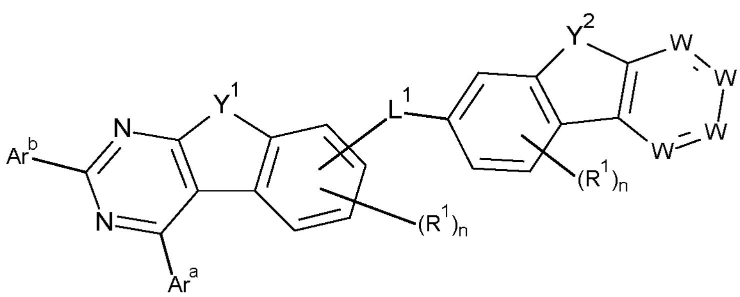

Formel (IIf)

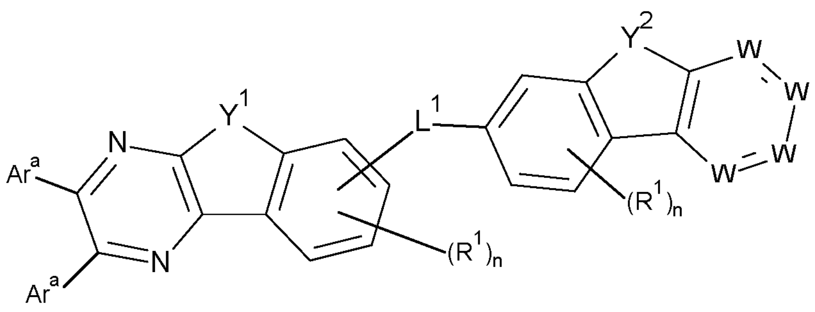

Formel (IIIf)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (If) und/oder (IIf) bevorzugt sind.

Vorzugsweise weisen die erfindungsgemäßen Verbindungen Strukturen gemäß mindestens eine der Formeln (Ig), (IIg) und/oder (IIIg) auf

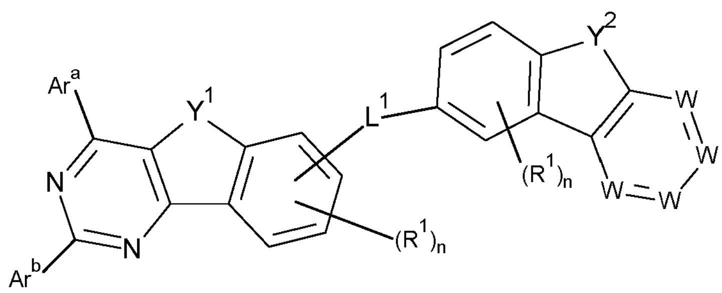

Formel (Ig)

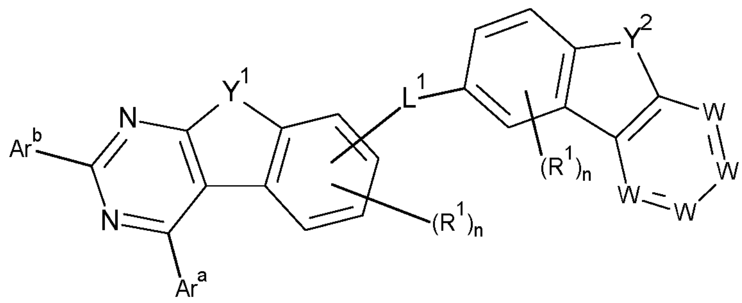

Formel (IIg)

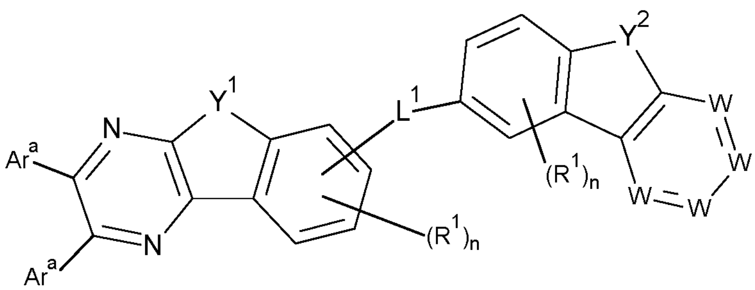

Formel (IIIg)

worin die Symbole $Ar^a$, $Ar^b$, $Y^1$, $L^1$, $Y^2$, $R^1$, n und W die zuvor insbesondere für Formel (A), (I), (II) oder (III) dargelegte

Bedeutung aufweisen, wobei Strukturen der Formel (Ig) und/oder (IIg) bevorzugt sind.

Ferner kann vorgesehen sein, dass die erfindungsgemäße Verbindung eine der Strukturen der Formeln (Ih), (IIh) und/oder (IIIh) aufweist

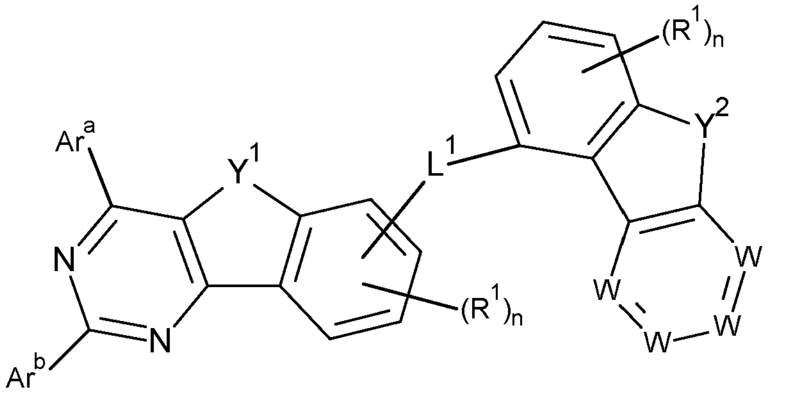

Formel (Ih)

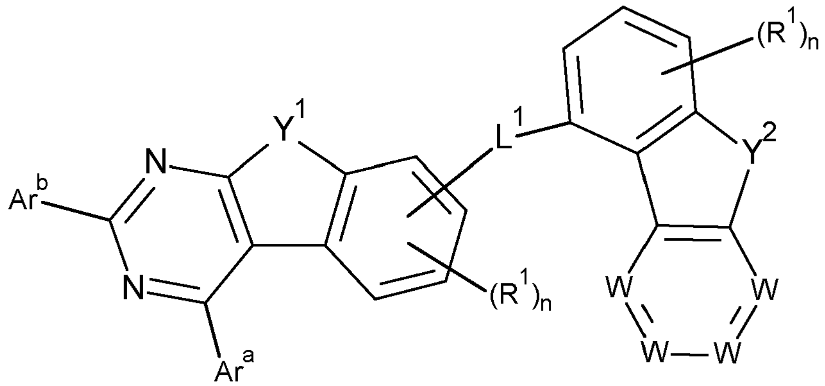

Formel (IIh)

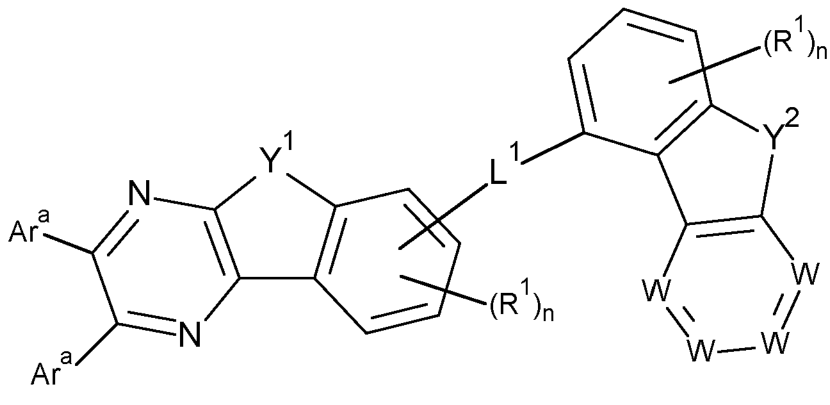

Formel (IIIh)

worin die Symbole $Ar^a$, $Ar^b$, $Y^1$, $L^1$, $Y^2$, $R^1$, n und W die zuvor insbesondere für Formel (A), (I), (II) oder (III) dargelegte Bedeutung aufweisen, wobei Strukturen der Formel (Ih) und/oder (IIh) bevorzugt sind.

Von den zuvor dargelegten Verbindungen mit denStrukturen der Formeln (Ie) bis (IIIh) sind solche Verbindungen bevorzugt, die die Strukturen der Formeln (Ig), (IIg), (IIIg), (Ih), (IIh) und/oder (IIIh) aufweisen, wobei Verbindungen mit Strukturen der Formeln (Ig), (IIg) und/oder (IIIg) besonders bevorzugt sind.

Weiterhin kann vorgesehen sein, dass die Substitutenten $R^1$ der Strukturen gemäß einer der Formeln (A), (I),

(II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh), die nicht Teil einer durch das Symbol W dargestellten Gruppe $CR^1$ sind, mit den Ringatomen der jeweiligen Ringstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substitutenten $R^1$ der Strukturen gemäß einer der Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh), die nicht Teil einer durch das Symbol W dargestellten Gruppe $CR^1$ sind, mit den Ringatomen der jeweiligen Ringstruktur kein Ringsystem bilden. Dies schließt die Bildung eines Ringsystems mit möglichen Substituenten $R^2$, $R^3$ ein, die an die Reste $R^1$ gebunden sein können.

Ferner kann vorgesehen sein, dass die Summe der Indices n in den Strukturen der Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) jeweils höchstens 3, vorzugsweise höchstens 2 und besonders bevorzugt höchstens 1 beträgt

In den Strukturen der Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) können zwei benachbarte Gruppen W jeweils für $CR^1$ stehen und zusammen eine Gruppe der Formel (W-1) bilden

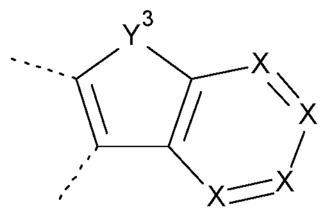

Formel (W-1)

worin

- $Y^3$ N(Ar), O, S oder $C(R^2)_2$, vorzugsweise $C(R^2)_2$ ist,
- X bei jedem Auftreten gleich oder verschieden N oder $CR^2$, vorzugsweise $CR^2$ ist, mit der Maßgabe, dass nicht mehr als zwei der Gruppen X in einem Cyclus für N stehen, wobei Ar und $R^2$ die zuvor, insbesondere für Formel (A) genannte Bedeutung aufweisen können, und

die gestrichelten Linien die Bindungen zu den benachbarten Atomen darstellen. Vorzugsweise weisen die Verbindungen der Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) vorzugsweise höchstens eine Gruppe der Formel (W-1) pro Struktur auf.

Darüber hinaus sind Verbindungen bevorzugt, die dadurch gekennzeichnet sind, dass in Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) nicht mehr als zwei, vorzugsweise nicht mehr als eine Gruppe W für N steht, vorzugsweise alle W für $CR^1$ stehen, wobei vorzugsweise höchstens 4, besonders bevorzugt höchstens 3 und speziell bevorzugt höchstens 2 der Gruppen $CR^1$ für die W steht, ungleich der Gruppe CH ist.

Ferner kann vorgesehen sein, dass die Reste $R^1$ der Gruppen W in den Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) mit den Ringatomen der Ringstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ der Gruppen W in den Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) mit den Ringatomen der Ringstruktur kein Ringsystem bilden. Dies schließt die Bildung eines Ringsystems mit möglichen Substituenten $R^2$, $R^3$ ein, die an die Reste $R^1$ gebunden sein können.

In einer bevorzugten Ausgestaltung der erfindungsgemäßen Verbindungen, der Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh), steht das Symbol $Y^2$ für $C(R^1)$ und $R^1$ jeweils gleich oder verschieden ein aromatisches oder heteroaromatisches Ringsystem mit 5 bis 40 aromatischen Ringatomen ist, das jeweils durch einen oder mehrere Reste $R^2$ substituiert sein kann.

Weiterhin kann vorgesehen sein, dass in den Formel (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) das Symbol $Y^2$ für eine

Gruppe der Formel ($Y^2$-1) steht

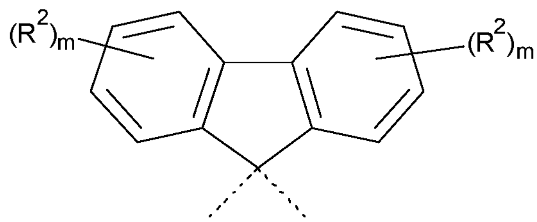

Formel ($Y^2$-1)

worin die gestrichelten Linien die Bindungen zu den benachbarten Atomen darstellen, $R^2$ die zuvor, insbesondere für Formel (A) genannte Bedeutung aufweist und m 0, 1, 2, 3 oder 4, vorzugsweise 0, 1 oder 2 ist.

Ferner kann vorgesehen sein, dass in den Formel (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) das Symbol $Y^2$ für eine Gruppe der Formel ($Y^2$-2) steht

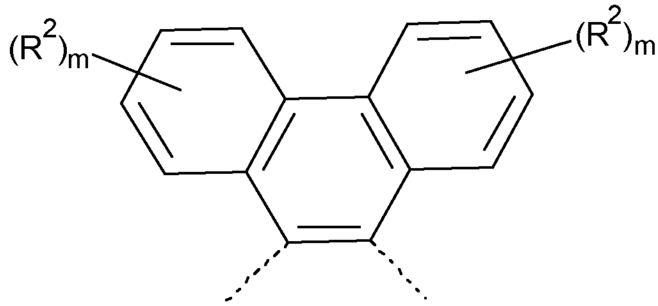

Formel ($Y^2$-2)

worin die gestrichelten Linien die Bindungen zu den benachbarten Atomen darstellen, $R^2$ die zuvor, insbesondere für Formel (A) genannte Bedeutung aufweist und m 0, 1, 2, 3 oder 4, vorzugsweise 0, 1 oder 2 ist.

Vorzugsweise kann vorgesehen sein, dass die Summe der Indices m in den Strukturen der Formel ($Y^2$-1) und/oder ($Y^2$-2) jeweils höchstens 3, vorzugsweise höchstens 2 und besonders bevorzugt höchstens 1 beträgt.

Ferner kann vorgesehen sein, dass die Reste $R^1$ in den Strukturen der Formel ($Y^2$-1) und/oder ($Y^2$-2) mit den Ringatomen der Ringstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ in den Strukturen der Formel ($Y^2$-1) und/oder ($Y^2$-2) mit den Ringatomen der Ringstruktur kein Ringsystem bilden. Dies schließt die Bildung eines Ringsystems mit möglichen Substituenten $R^2$, $R^3$ ein, die an die Reste $R^1$ gebunden sein können.

In einer weiteren, bevorzugten Ausgestaltung kann in Strukturen gemäß Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) das Symbol $Y^1$ für O oder S und das Symbol $Y^2$ für N(Ar) stehen.

Vorzugsweise kann in Strukturen gemäß Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) eine der Gruppen Ar, $Ar^a$ und/oder $Ar^b$ höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können. Speziell bevorzugt weisen die Gruppen Ar, $Ar^a$ und/oder $Ar^b$ kein Heteroatom auf, wobei dies Substituenten $R^1$, $R^2$ und $R^3$ einschließt, die an diese Gruppen gebunden sein können.

Ferner kann vorgesehen sein, dass die Gruppen Ar, $Ar^a$ und/oder $Ar^b$ in Strukturen gemäß Formeln (A), (I), (II), (III), (Ia), (IIa), (Iila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) insgesamt höchstens 5, vorzugsweise höchstens 3 und besonders bevorzugt höchstens 1 Heteroatom aufweisen, wobei dies Substituenten $R^1$, $R^2$ und $R^3$ einschließt, die an diese Gruppen gebunden sein können.

Weiterhin kann vorgesehen sein, dass die Gruppen Ar, $Ar^a$ und/oder $Ar^b$ in Strukturen gemäß Formeln (A), (I),

(II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) insgesamt höchstens 50, vorzugsweise höchstens 40 und besonders bevorzugt höchstens 22 aromatische Ringatome aufweisen, wobei dies Substituenten $R^1$, $R^2$ und $R^3$ einschließt, die an diese Gruppen gebunden sein können.

Darüber hinaus kann vorgesehen sein, dass der Rest Ar in Strukturen gemäß Formeln (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) keine Carbazol-Gruppe umfasst, wobei dies Substituenten $R^1$, $R^2$ und $R^3$, die an den Rest Ar gebunden sein können, einschließt.

Vorzugsweise umfasst der R est $Ar^a$ in Strukturen gemäß Formeln (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) keine Carbazol-Gruppe umfasst, wobei dies Substituenten $R^1$, $R^2$ und $R^3$, die an den Rest $Ar^a$ gebunden sein können, einschließt.

In einer weiterhin bevorzugten Ausgestaltung kann die Gruppe $Ar^b$ in Strukturen gemäß Formeln (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) eine Gruppe der Formel ($Ar^b$-1) darstellen

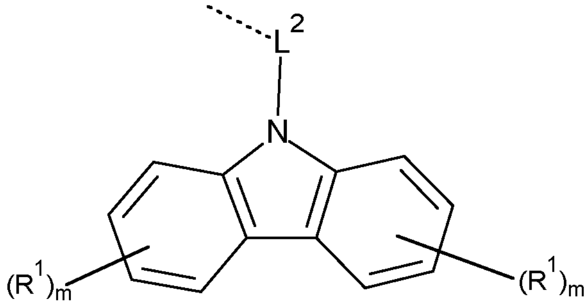

Formel ($Ar^b$-1)

worin $L^2$ eine Bindung oder ein aromatisches oder heteroaromatisches Ringsystem mit 5 bis 30 aromatischen Ringatomen ist, welches durch einen oder mehrere Reste $R^1$ substituiert sein kann, das Symbol $R^1$ die zuvor, insbesondere für Formel (A) genannte Bedeutung aufweist, m 0, 1, 2, 3 oder 4, vorzugsweise 0, 1 oder 2 ist und die gestrichelte Linie die Bindung zur Diazadibenzofuran- oder Diazadibenzothiophen-Gruppe darstellt.

Bevorzugt sind unter anderem Verbindungen der Formel (IV) und/oder (V)

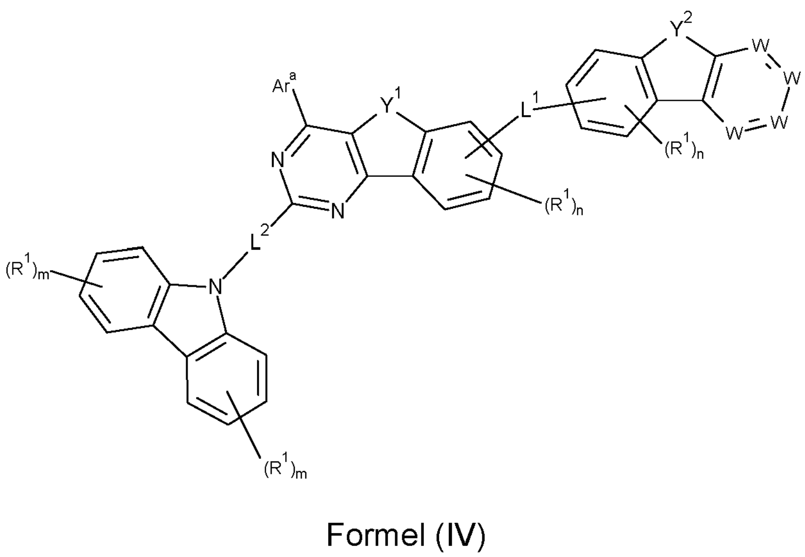

Formel (IV)

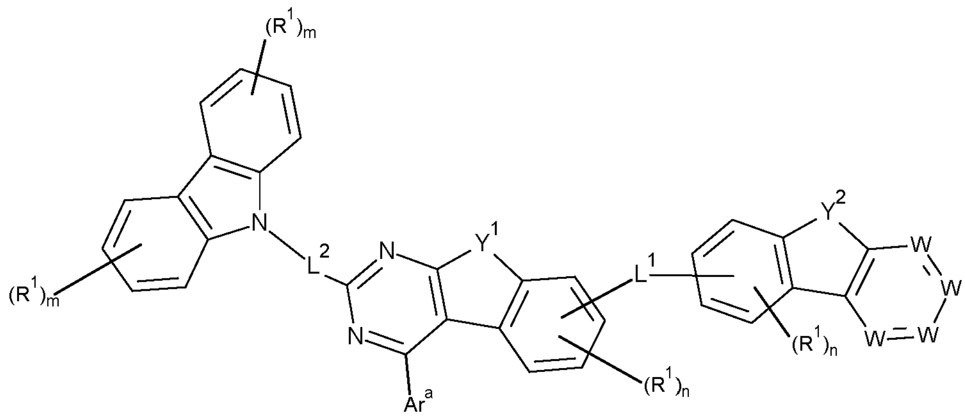

Formel (V)

worin $L^2$ eine Bindung oder ein aromatisches oder heteroaromatisches Ringsystem mit 5 bis 30 aromatischen Ringatomen ist, welches durch einen oder mehrere Reste $R^1$ substituiert sein kann, die Symbole $Ar^a$, $Y^1$, $L^1$, $Y^2$, $R^1$, n und W die zuvor, insbesondere für Formel (A) genannte Bedeutung aufweisen, m 0, 1, 2, 3 oder 4 ist, vorzugsweise 0, 1 oder 2, und die gestrichelte Linie die Bindung darstellt.

Vorzugsweise kann vorgesehen sein, dass die Summe der Indices m in den Strukturen der Formeln ($Ar^b$-1), (IV) und/oder (V) jeweils höchstens 3, vorzugsweise höchstens 2 und besonders bevorzugt höchstens 1 beträgt.

Ferner kann vorgesehen sein, dass die Reste $R^1$ in den Strukturen der Formeln ($Ar^b$-1), (IV) und/oder (V) mit den Ringatomen der Ringstruktur, an die der Rest $R^1$ bindet, kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ in den Strukturen der Formeln ($Ar^b$-1), (IV) und/oder (V) mit den Ringatomen der Ringstruktur kein Ringsystem bilden. Dies schließt die Bildung eines Ringsystems mit möglichen Substituenten $R^2$, $R^3$ ein, die an die Reste $R^1$ gebunden sein können.

Für Strukturen der Formeln (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh) und/oder (IIIh) in denen $Y^2$ für $C(R^1)_2$ oder $-R^1C=CR^1$-steht, kann der Rest $Ar^a$ ebenfalls für eine Gruppe der Formel ($Ar^b$-1) stehen.

Ferner kann vorgesehen sein, dass der Rest Ar, $Ar^a$ und/oder $Ar^b$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, Spirobifluorenyl, Fluorenyl, Dibenzofuranyl, Dibenzothiophenyl, Anthracenyl, Phenanthrenyl und/oder Triphenylenyl, die jeweils durch einen oder mehrere Reste $R^2$ substituiert sein können, bevorzugt aber unsubstituiert sind, wobei Spirobifluoren-, Fluoren-, Dibenzofuran-, Dibenzothiophen-, Anthracen-, Phenanthren-, TriphenylenGruppen besonders bevorzugt sind.

In einer weiteren bevorzugten Ausführungsform enthalten weder $Ar^a$ noch $Ar^b$ in den oben genannten Verbindungen eine Carbazol-, Dibenzofuran- oder Dibenzothiophengruppe. Ganz bevorzugt ist, wenn weder $Ar^a$ noch $Ar^b$ in den oben genannten Verbindungen eine kondensierte heteroaromatische Gruppe enthalten. Besonders bevorzugt ist, wenn weder $Ar^a$ noch $Ar^b$ in den oben genannten Verbindungen eine heteroaromatische Gruppe enthalten.

Gemäß einer bevorzugten Ausgestaltung sind Verbindungen der Formel (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V) durch Strukturen der Formel (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V) darstellbar. Vorzugsweise weisen Verbindungen der Formel (A), (I), (II), (III), (Ia), (IIa), (IiIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn W für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann; wobei $Ar^1$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (A) genannte Bedeutung aufweisen. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (A), (I), (II), (III), (Ia), (IIa), (lila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V) mindestens ein Rest $R^1$, Ar, $Ar^1$, $Ar^a$ oder $Ar^b$ für eine Gruppe steht, die ausgewählt ist aus den Formeln ($R^1$-1) bis ($R^1$- 87)

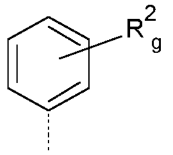

Formel ($R^1$-1)

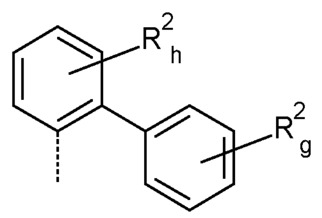

Formel ($R^1$-2)

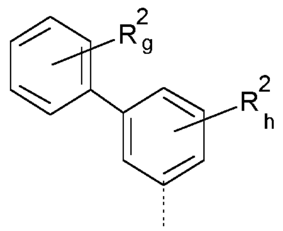

Formel ($R^1$-3)

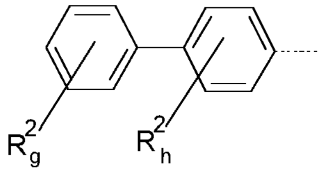

Formel ($R^1$-4)

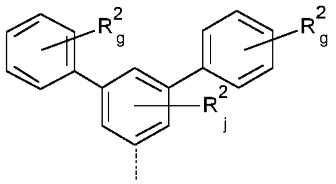

Formel ($R^1$-5)

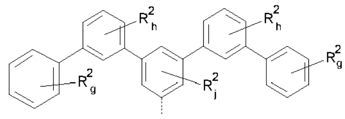

Formel ($R^1$-6)

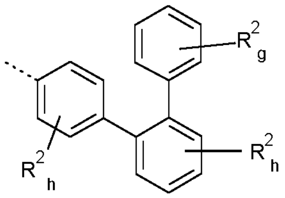

Formel ($R^1$-7)

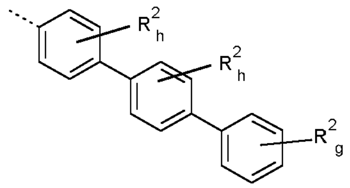

Formel ($R^1$-8)

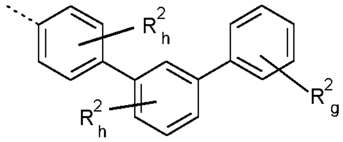

Formel ($R^1$-9)

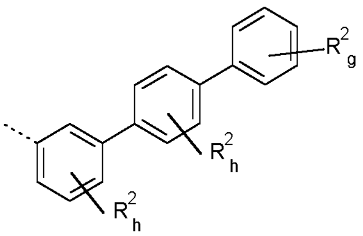

Formel ($R^1$-10)

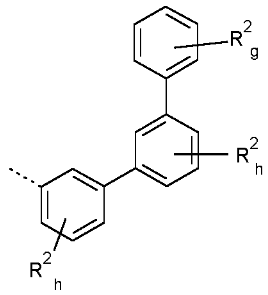

Formel ($R^1$-11)

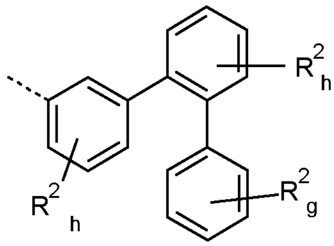

Formel ($R^1$-12)

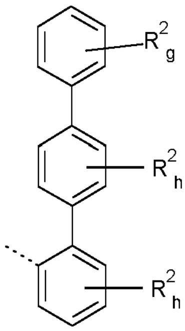

Formel ($R^1$-13)

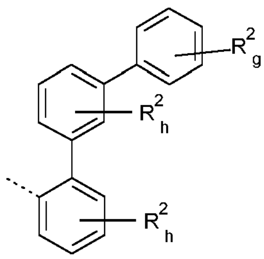

Formel ($R^1$-14)

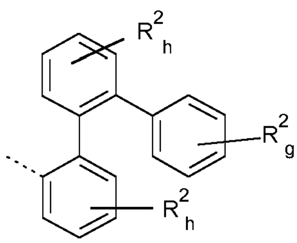

Formel ($R^1$-15)

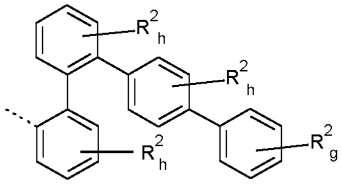

Formel ($R^1$-16)

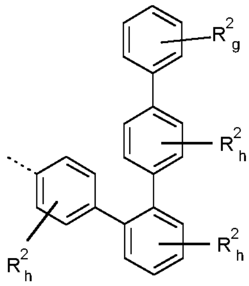

Formel ($R^1$-17)

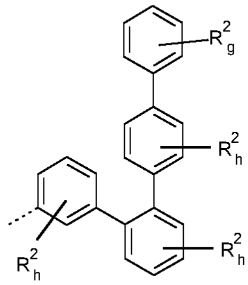

Formel ($R^1$-18)

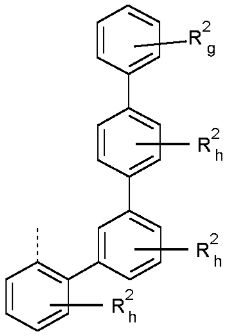

Formel ($R^1$-19)

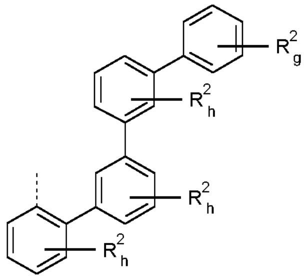

Formel ($R^1$-20)

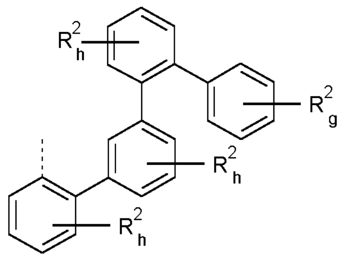

Formel ($R^1$-21)

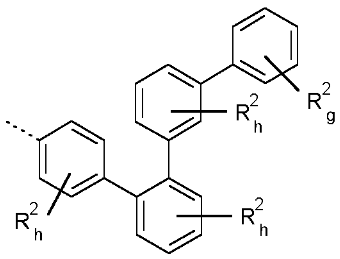

Formel ($R^1$-22)

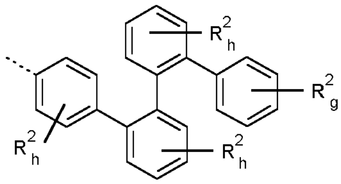

Formel ($R^1$-23)

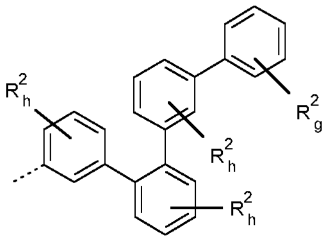

Formel ($R^1$-24)

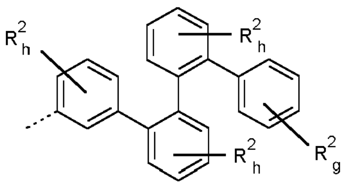

Formel ($R^1$-25)

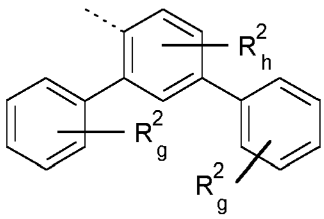

Formel ($R^1$-26)

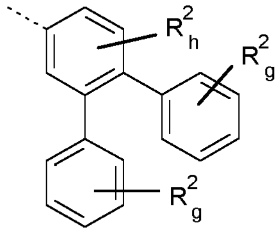

Formel ($R^1$-27)

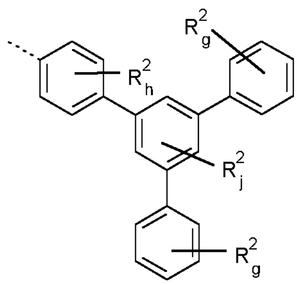

Formel ($R^1$-28)

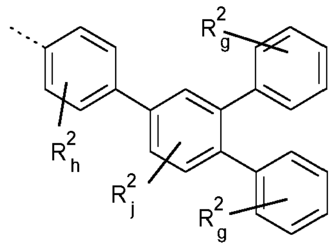

Formel ($R^1$-29)

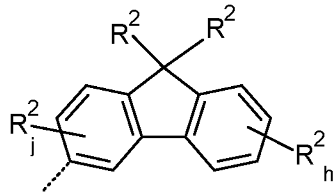

Formel ($R^1$-30)

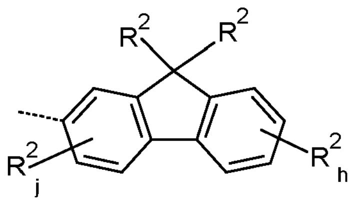

Formel ($R^1$-31)

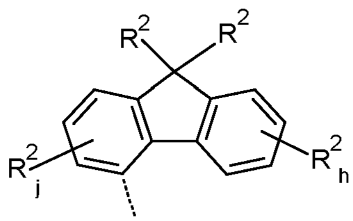

Formel ($R^1$-32)

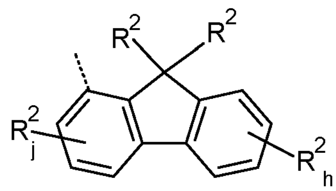

Formel ($R^1$-33)

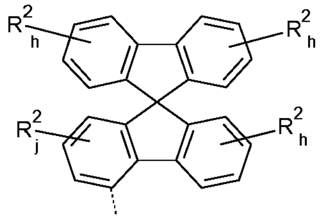

Formel ($R^1$-34)

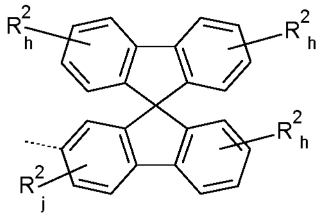

Formel ($R^1$-35)

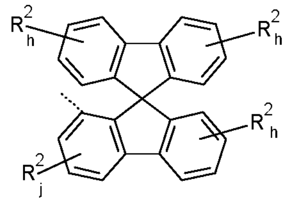

Formel ($R^1$-36)

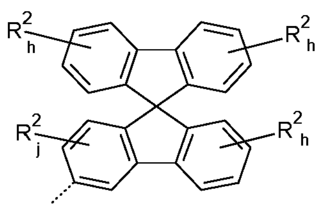

Formel ($R^1$-37)

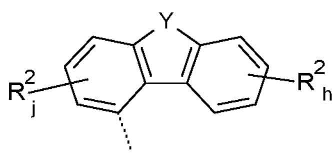

Formel ($R^1$-38)

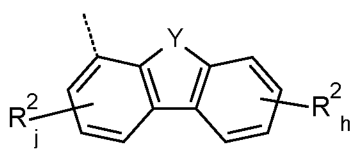

Formel ($R^1$-39)

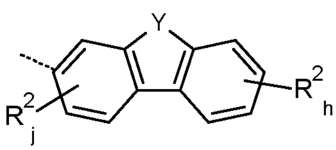

Formel ($R^1$-40)

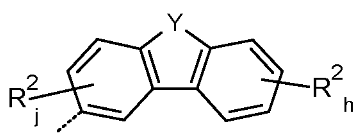

Formel ($R^1$-41)

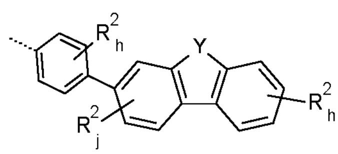

Formel ($R^1$-42)

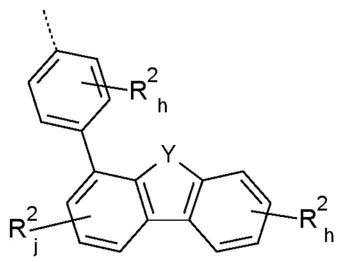

Formel ($R^1$-43)

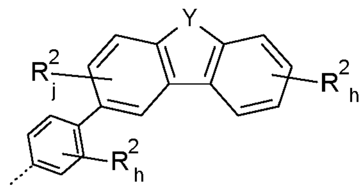

Formel ($R^1$-44)

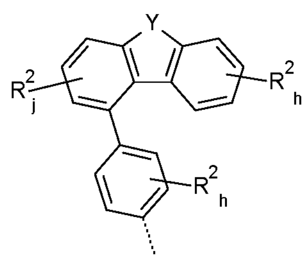

Formel ($R^1$-45)

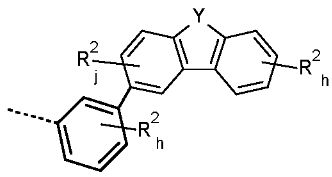

Formel ($R^1$-46)

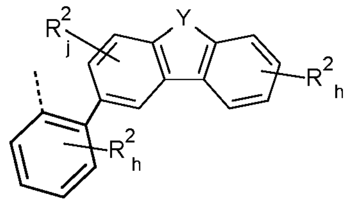

Formel ($R^1$-47)

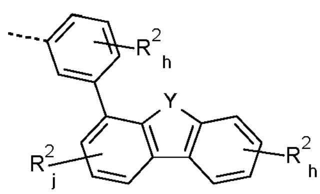

Formel ($R^1$-48)

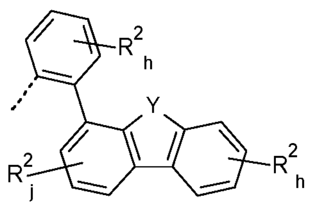

Formel ($R^1$-49)

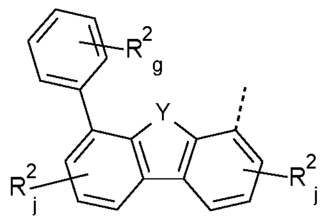

Formel ($R^1$-50)

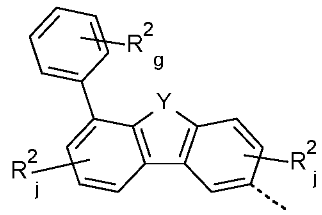

Formel ($R^1$-51)

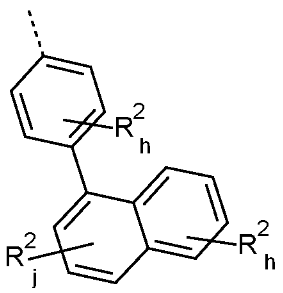

Formel ($R^1$-52)

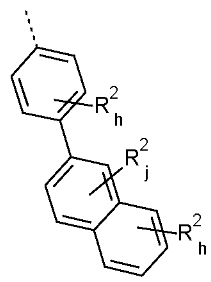

Formel ($R^1$-53)

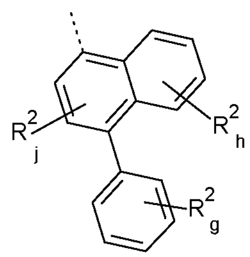

Formel ($R^1$-54)

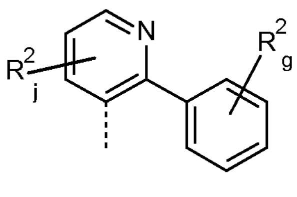

Formel ($R^1$-55)

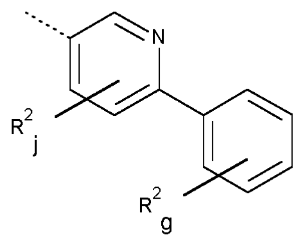

Formel ($R^1$-56)

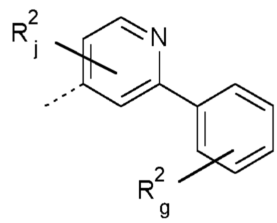

Formel ($R^1$-57)

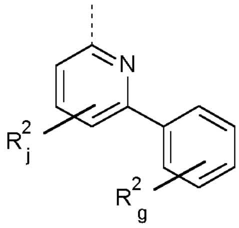
Formel ($R^1$-58)

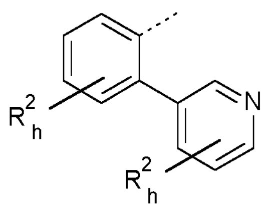
Formel ($R^1$-59)

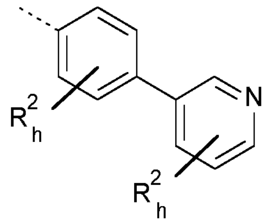
Formel ($R^1$-60)

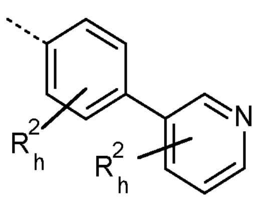
Formel ($R^1$-61)

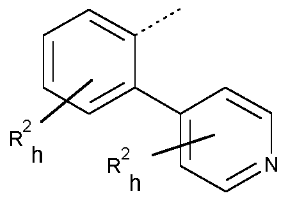
Formel ($R^1$-62)

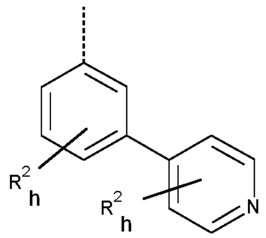
Formel ($R^1$-63)

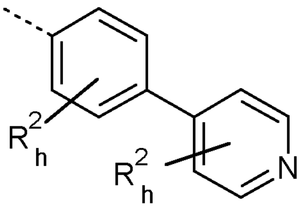
Formel ($R^1$-64)

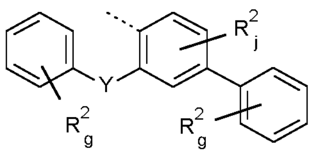
Formel ($R^1$-65)

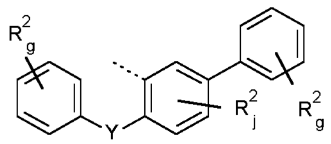
Formel ($R^1$-66)

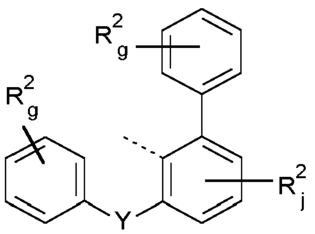
Formel ($R^1$-67)

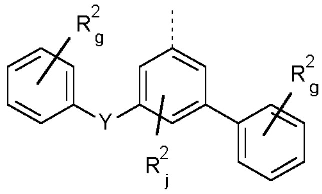
Formel ($R^1$-68)

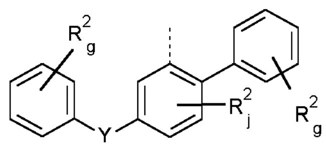
Formel ($R^1$-69)

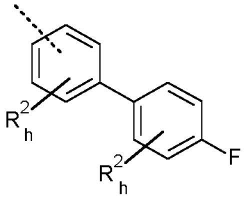
Formel ($R^1$-70)

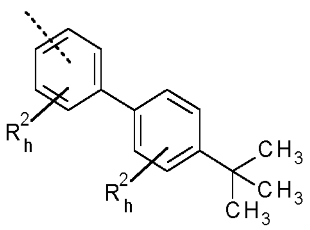
Formel ($R^1$-71)

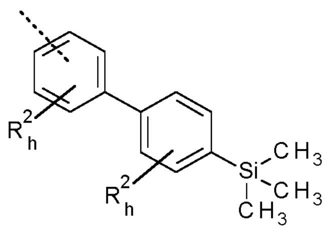
Formel ($R^1$-72)

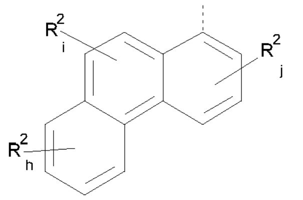

Formel ($R^1$-73)

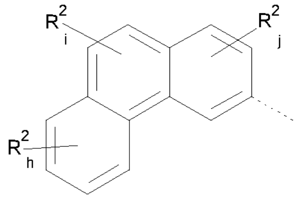

Formel ($R^1$-74)

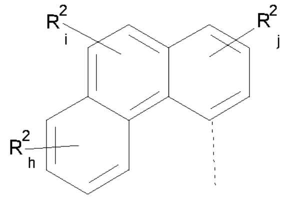

Formel ($R^1$-75)

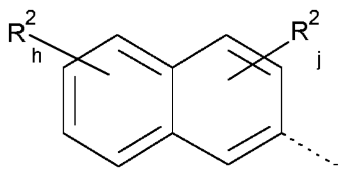

Formel ($R^1$-76)

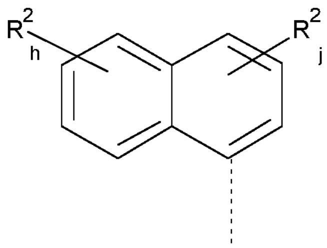

Formel ($R^1$-77)

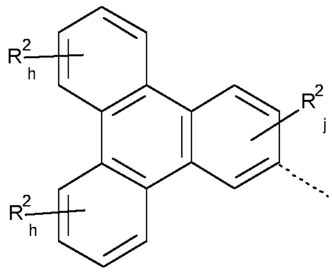

Formel ($R^1$-78)

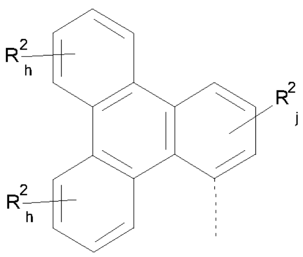

Formel ($R^1$-79)

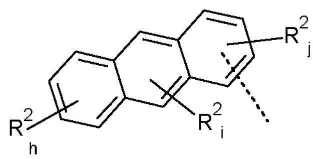

Formel ($R^1$-80)

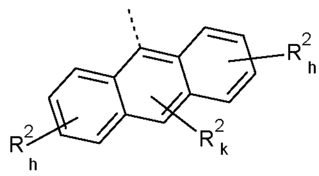

Formel ($R^1$-81)

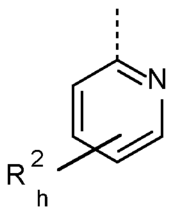

Formel ($R^1$-82)

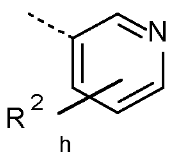

Formel ($R^1$-83)

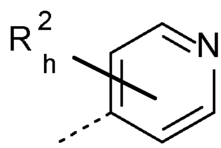

Formel ($R^1$-84)

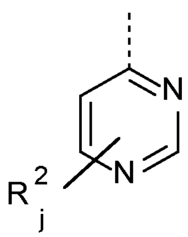

Formel ($R^1$-85)

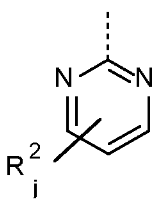

Formel ($R^1$-86)

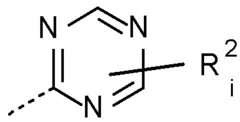

Formel ($R^1$-87)

wobei für die verwendeten Symbole gilt:

Y ist O, S oder $NR^2$, vorzugsweise O oder S;

k ist bei jedem Auftreten unabhängig 0 oder 1

i ist bei jedem Auftreten unabhängig 0, 1 oder 2, bevorzugt 0 oder 1;

j ist bei jedem Auftreten unabhängig 0, 1, 2 oder 3, vorzugsweise 0, 1 oder 2, besonders bevorzugt 0 oder 1;

h ist bei jedem Auftreten unabhängig 0, 1, 2, 3 oder 4, vorzugsweise 0, 1 oder 2;

g ist bei jedem Auftreten unabhängig 0, 1, 2, 3, 4 oder 5, vorzugsweise 0, 1 oder 2;

$R^2$ kann die zuvor genannte, insbesondere für Formel (A) genannte Bedeutung aufweisen und

die gestrichelte Bindung markiert die Anbindungsposition.

Vorzugsweise kann vorgesehen sein, dass die Summe der Indices k, i, j, h und g in den Strukturen der Formel ($R^1$-1) bis ($R^1$-87) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-87) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Besonders bevorzugt sind die Reste $Ar^a$ und/oder $Ar^b$ in den Formeln (A), (I), (II), (III), (Ia), (IIa), (IIIa), (Ib), (IIb), (IIIb), (Ic), (IIc), (IIIe), (Id), (IId), (IIId), (Ie), (IIe), (IIIe), (If), (IIf), (IIIf), (Ig), (IIg), (IIIg), (Ih), (IIh), (IIIh), (IV) und/oder (V) ausgewählt aus einer Gruppe gemäß den Formeln ($R^1$-1) bis ($R^1$-48) und ($R^1$-73) bis ($R^1$-87), speziell bevorzugt ($R^1$-1), ($R^1$-38) bis ($R^1$-48) und ($R^1$-73) bis ($R^1$-81). Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

Besonders bevorzugt sind die Reste Ar und/oder $Ar^1$ in den Formeln (A), (I), (II), (III), (Ia), (IIa), (IIIa), (Ib), (IIb), (IIIb), (Ic), (IIc), (IIIe), (Id), (IId), (IIId), (Ie), (IIe), (IIIe), (If), (IIf), (IIIf), (Ig), (IIg), (IIIg), (Ih), (IIh), (IIIh), (IV) und/oder (V) ausgewählt aus einer Gruppe gemäß den Formeln ($R^1$-1) bis ($R^1$-54), insbesondere bevorzugt ($R^1$-1) bis ($R^1$-51), speziell bevorzugt ($R^1$-1) bis ($R^1$-37), wobei Reste gemäß ($R^1$-1) ganz besonders bevorzugt sind. Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

Bevorzugt kann die Gruppe $L^1$ oder $L^2$ mit den beiden Aryl- oder Heteroarylgruppen, an den die Gruppe $L^1$ oder $L^2$ gemäß Formel (A), (I), (II), (III), (Ia), (IIa), (IIIa), (Ib), (IIb), (IIIb), (Ic), (IIc), (IIIe), (Id), (IId), (IIId), (Ie), (IIe), (IIIe), (If), (IIf), (IIIf), (Ig), (IIg), (IIIg), (Ih), (IIh), (IIIh), ($Ar^b$-1), (IV) und/oder (V) gebunden ist, eine durchgängige Konjugation ausbilden. Eine durchgängige Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen den beiden Aryl- oder Heteroarylgruppen liegt. Im Gegensatz hierzu kann bei einer zweiten Spirobifluorenstruktur eine durchgängie Konjugation ausgebildet werden, falls die Verbindung zwischen den beiden Aryl- oder Heteroarylgruppen über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen den beiden Aryl- oder Heteroarylgruppen über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen.

In einer bevorzugten Ausführungsform der Erfindung steht $L^1$ und/oder $L^2$ für eine Bindung.

In einer weiteren bevorzugten Ausführungsform der Erfindung steht $L^1$ und/oder $L^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (A) genannte Bedeutung aufweisen kann. Besonders bevorzugt steht $L^1$ und/oder $L^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor, insbesondere für Formel (A) genannte Bedeutung aufweisen kann.

Weiterhin bevorzugt steht das unter anderem in den Strukturen gemäß Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), ($Ar^b$-1), (IV) und/oder (V) dargelegte Symbol $L^1$ und/oder $L^2$ gleich oder verschieden bei jedem Auftreten für einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh),

(IIIh), ($Ar^b$-1), (IV) und/oder (V) dargelegte Gruppe $L^1$ und/oder $L^2$ ein aromatisches Ringsystem mit höchstens zwei kondensierten aromatischen und/oder heteroaromatischen Ringen, vorzugsweise kein kondensiertes aromatisches oder heteroaromatisches Ringsystem umfasst. Demgemäß sind Naphtylstrukturen gegenüber Antracenstrukturen bevorzugt. Weiterhin sind Fluorenyl-, Spirobifluorenyl-, Dibenzofuranyl- und/oder Dibenzothienyl-Strukturen gegenüber Naphtylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme $L^1$ und/oder $L^2$ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^2$ substituiert sein können, bevorzugt aber unsubstituiert sind.

Ferner kann vorgesehen sein, dass die unter anderem in den Strukturen gemäß Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), ($Ar^b$-1), (IV) und/oder (V) dargelegte Gruppe $L^1$ und/oder $L^2$ höchstens 1 Stickstoffatom, bevorzugt höchstens 2 Heteroatome, insbesondere bevorzugt höchstens ein Heteroatom und besonders bevorzugt kein Heteroatom aufweist.

Bevorzugt sind Verbindungen der Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V), in denen die Gruppe $L^1$ und/oder $L^2$ gemäß Formeln (A), (I), (II), (III), (Ia), (IIa), (lila), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), ($Ar^b$-1), (IV) und/oder (V) für eine Gruppe steht, die ausgewählt ist aus den Formeln ($L^1$-1) bis ($L^1$-108)

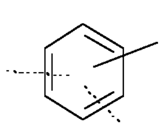

Formel ($L^1$-1)

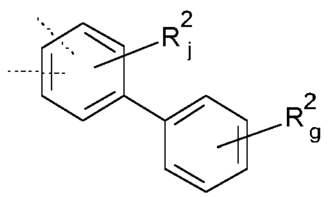

Formel ($L^1$-2)

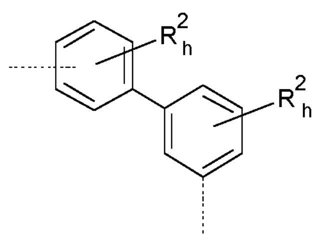

Formel ($L^1$-3)

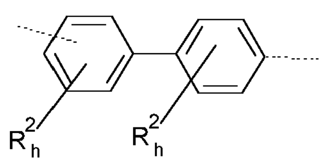

Formel ($L^1$-4)

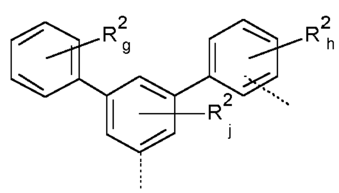

Formel ($L^1$-5)

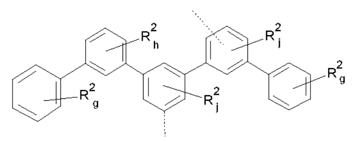

Formel ($L^1$-6)

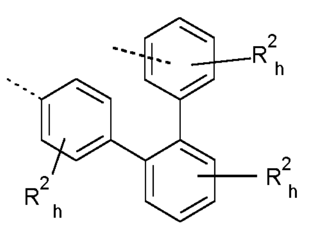

Formel ($L^1$-7)

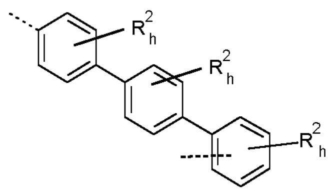

Formel ($L^1$-8)

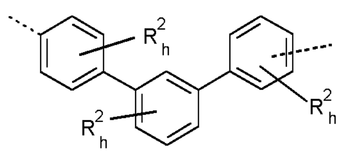

Formel ($L^1$-9)

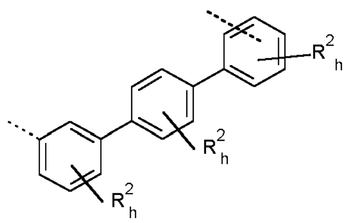

Formel ($L^1$-10)

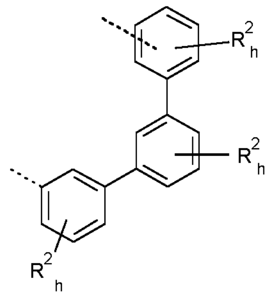

Formel ($L^1$-11)

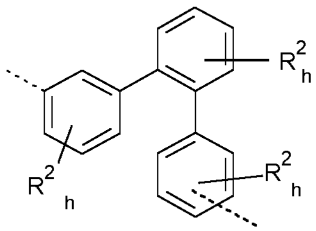

Formel ($L^1$-12)

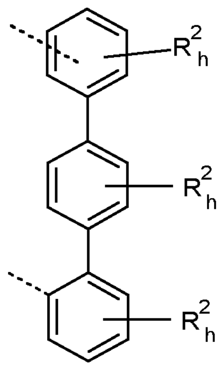

Formel ($L^1$-13)

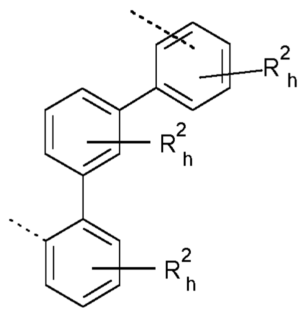

Formel ($L^1$-14)

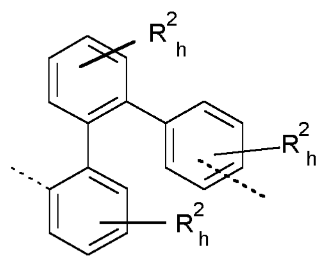

Formel ($L^1$-15)

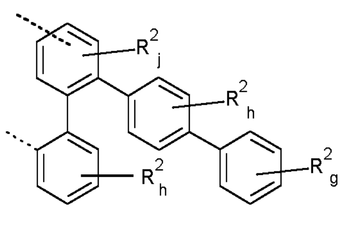

Formel ($L^1$-16)

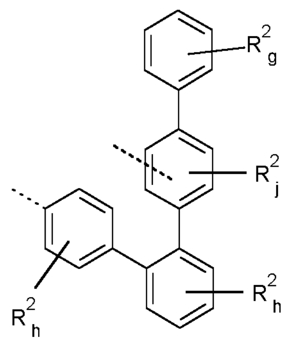

Formel ($L^1$-17)

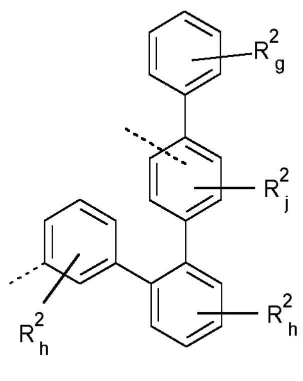

Formel ($L^1$-18)

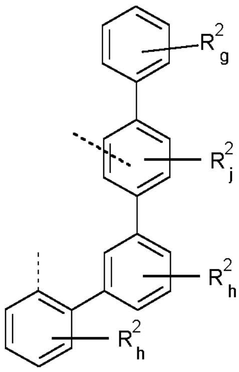

Formel ($L^1$-19)

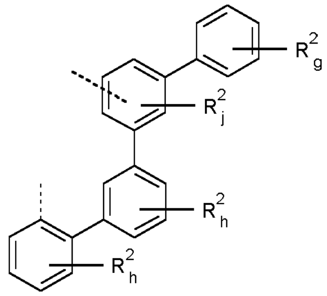

Formel ($L^1$-20)

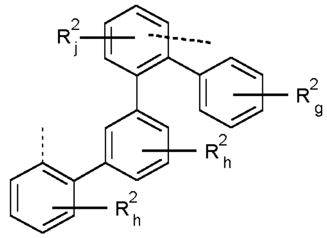

Formel ($L^1$-21)

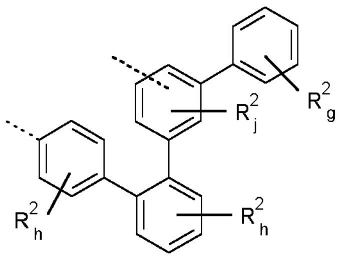

Formel ($L^1$-22)

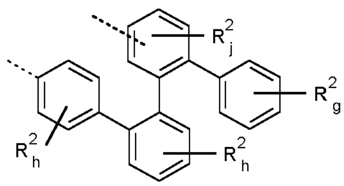

Formel ($L^1$-23)

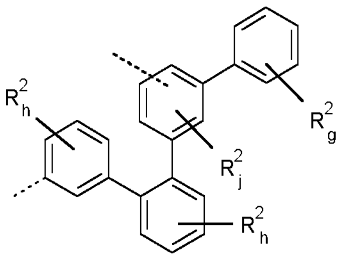

Formel ($L^1$-24)

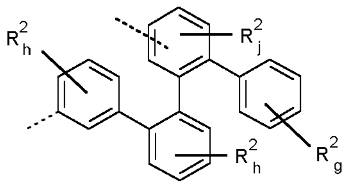

Formel ($L^1$-25)

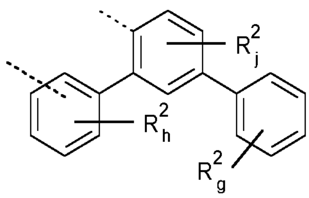

Formel ($L^1$-26)

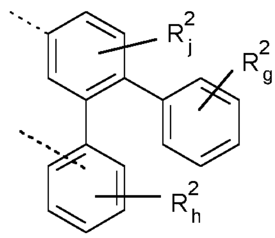

Formel ($L^1$-27)

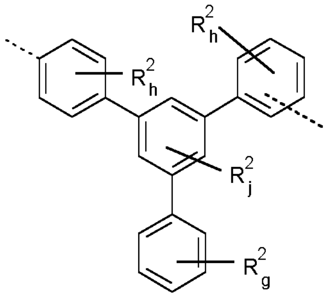

Formel ($L^1$-28)

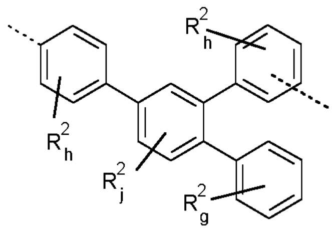

Formel ($L^1$-29)

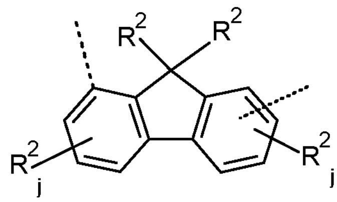

Formel ($L^1$-30)

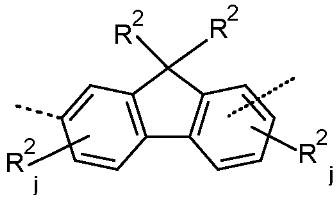
Formel ($L^1$-31)

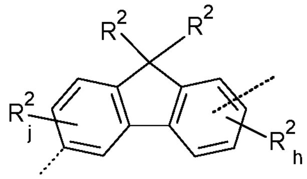
Formel ($L^1$-32)

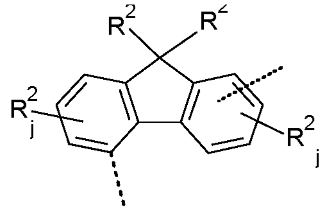
Formel ($L^1$-33)

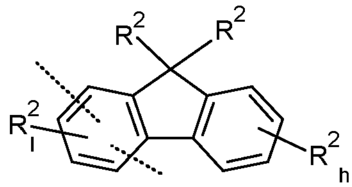
Formel ($L^1$-34)

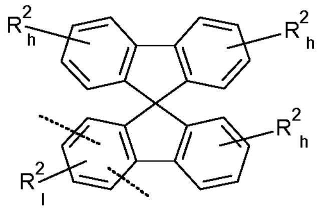
Formel ($L^1$-35)

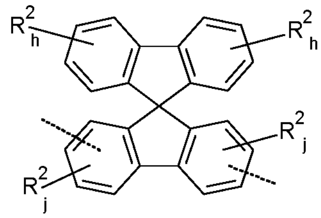
Formel ($L^1$-36)

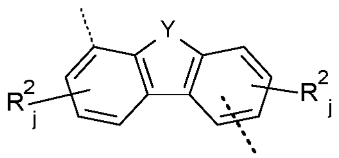
Formel ($L^1$-37)

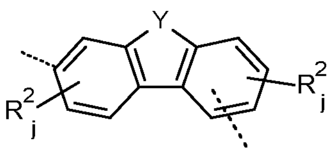
Formel ($L^1$-38)

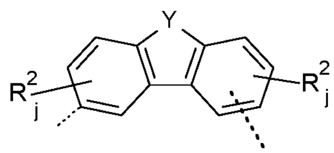
Formel ($L^1$-39)

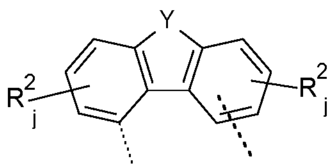
Formel ($L^1$-40)

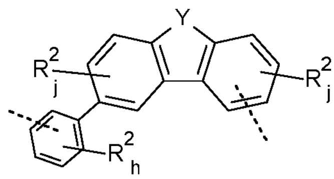
Formel ($L^1$-41)

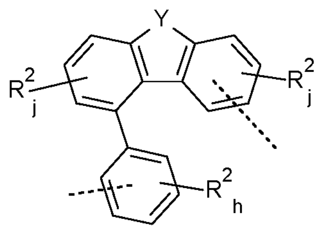
Formel ($L^1$-42)

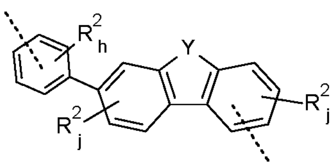
Formel ($L^1$-43)

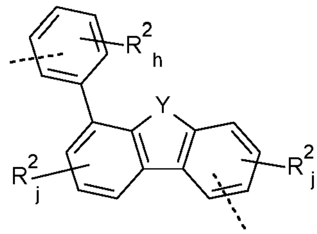
Formel ($L^1$-44)

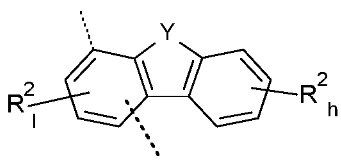
Formel ($L^1$-45)

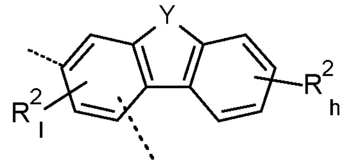

Formel ($L^1$-46)

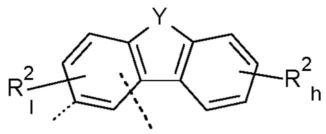

Formel ($L^1$-47)

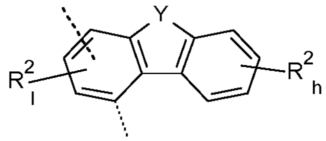

Formel ($L^1$-48)

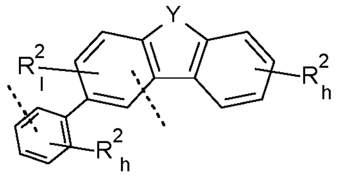

Formel ($L^1$-49)

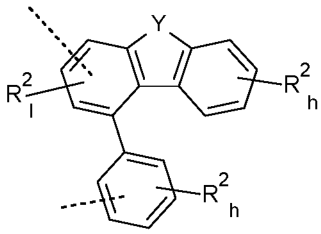

Formel ($L^1$-50)

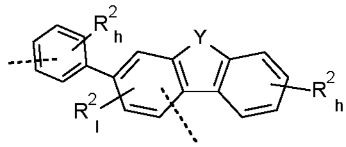

Formel ($L^1$-51)

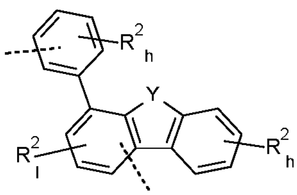

Formel ($L^1$-52)

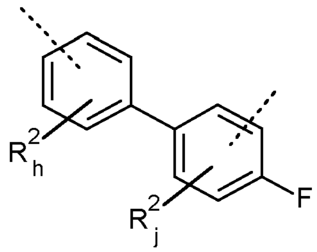

Formel ($L^1$-53)

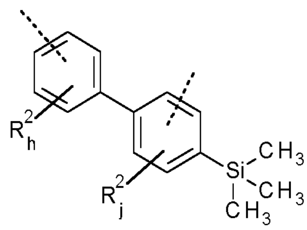

Formel ($L^1$-54)

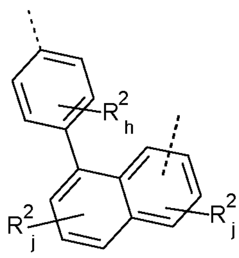

Formel ($L^1$-55)

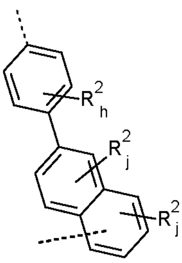

Formel ($L^1$-56)

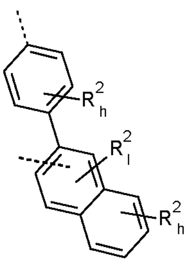

Formel ($L^1$-57)

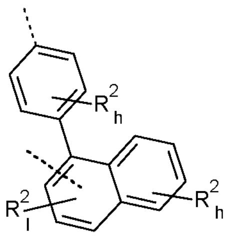

Formel ($L^1$-58)

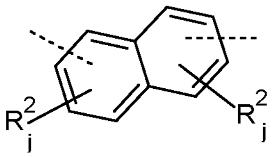

Formel ($L^1$-59)

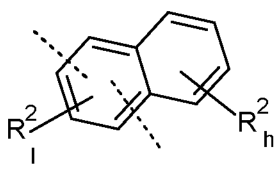

Formel ($L^1$-60)

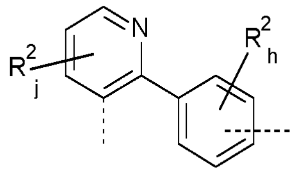
Formel ($L^1$-61)

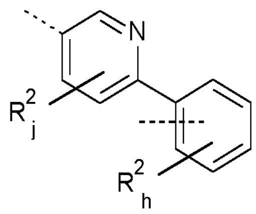
Formel ($L^1$-62)

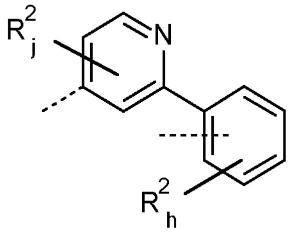
Formel ($L^1$-63)

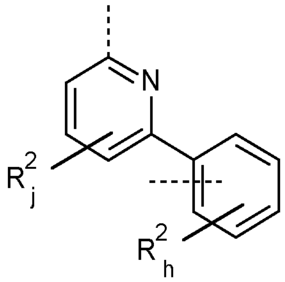
Formel ($L^1$-64)

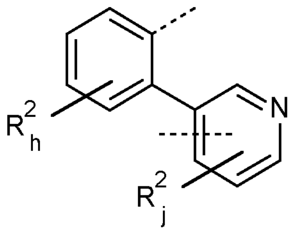
Formel ($L^1$-65)

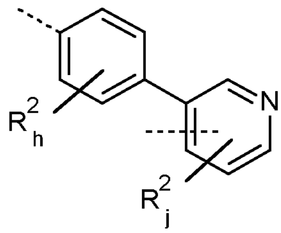
Formel ($L^1$-66)

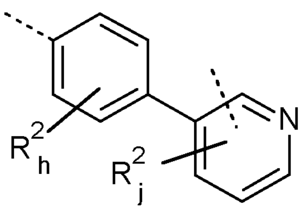
Formel ($L^1$-67)

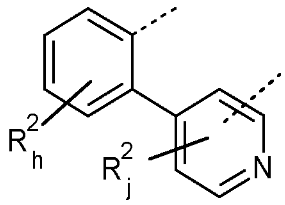
Formel ($L^1$-68)

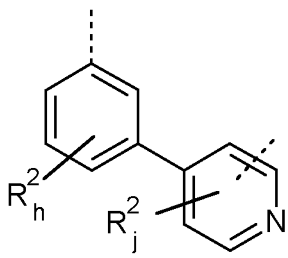
Formel ($L^1$-69)

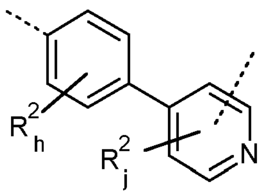
Formel ($L^1$-70)

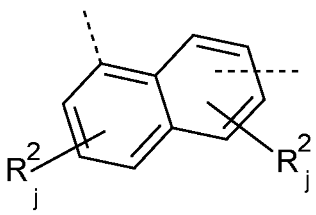
Formel ($L^1$-71)

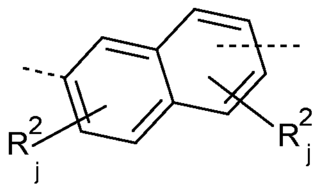
Formel ($L^1$-72)

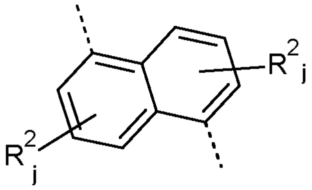
Formel ($L^1$-73)

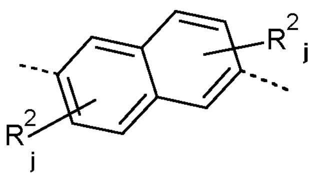
Formel ($L^1$-74)

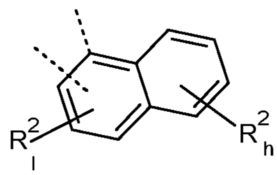
Formel ($L^1$-75)

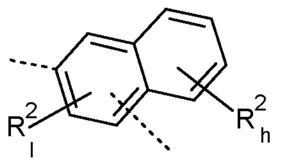

Formel ($L^1$-76)

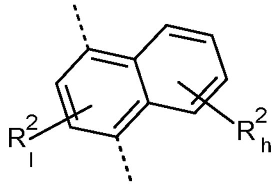

Formel ($L^1$-77)

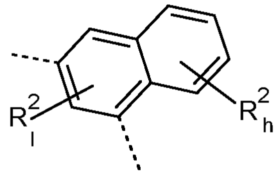

Formel ($L^1$-78)

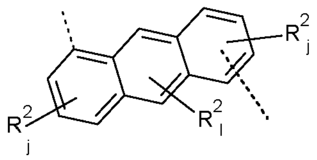

Formel ($L^1$-79)

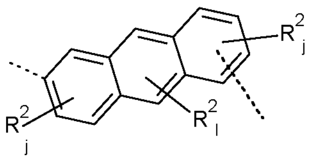

Formel ($L^1$-80)

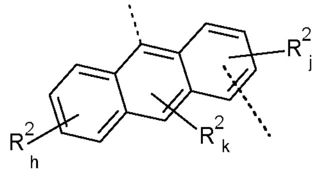

Formel ($L^1$-81)

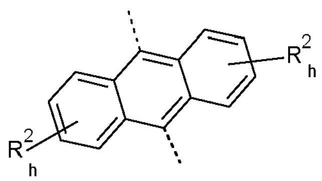

Formel ($L^1$-82)

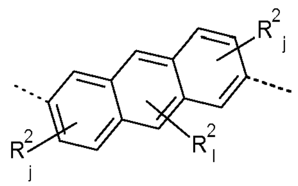

Formel ($L^1$-83)

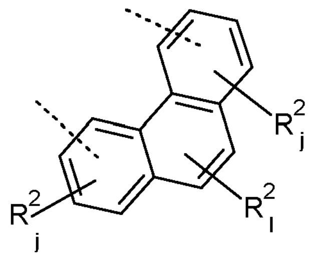

Formel ($L^1$-84)

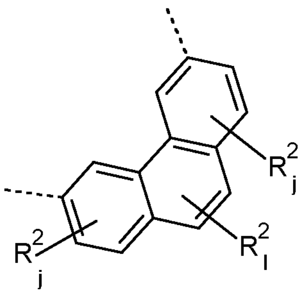

Formel ($L^1$-85)

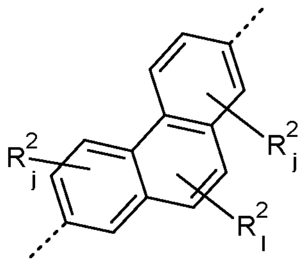

Formel ($L^1$-86)

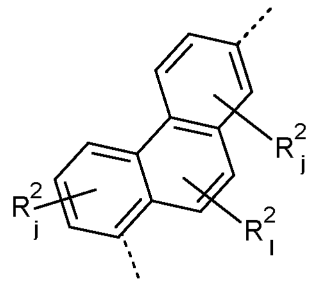

Formel ($L^1$-87)

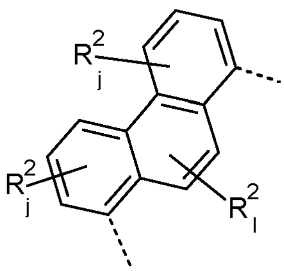

Formel ($L^1$-88)

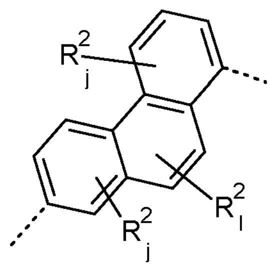

Formel ($L^1$-89)

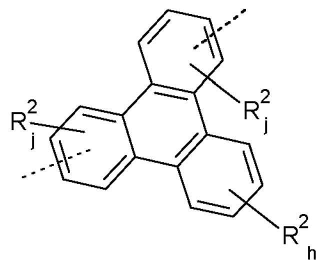

Formel ($L^1$-90)

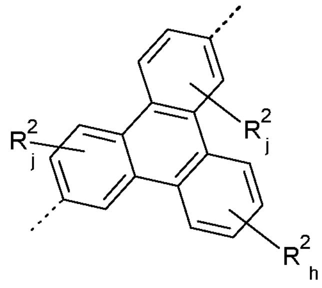

Formel ($L^1$-91)

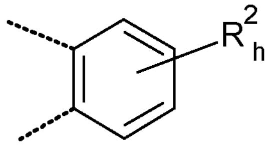

Formel ($L^1$-92)

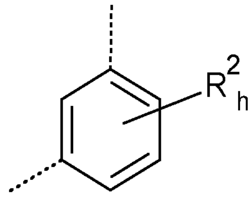

Formel ($L^1$-93)

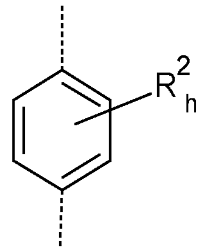

Formel ($L^1$-94)

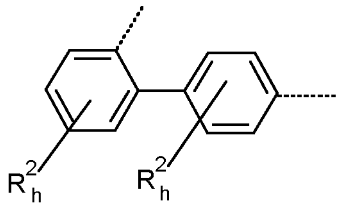

Formel ($L^1$-95)

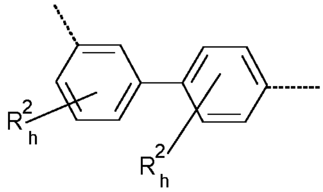

Formel ($L^1$-96)

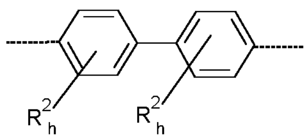

Formel ($L^1$-97)

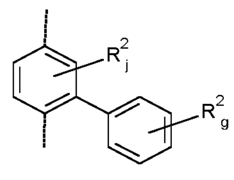

Formel ($L^1$-98)

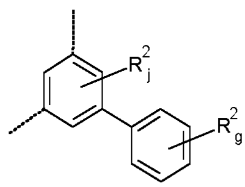

Formel ($L^1$-99)

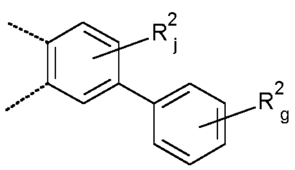

Formel ($L^1$-100)

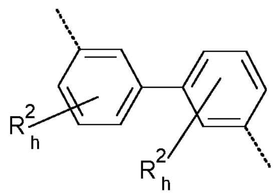

Formel ($L^1$-101)

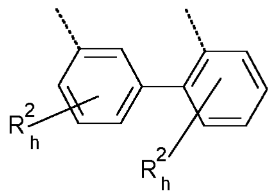

Formel ($L^1$-102)

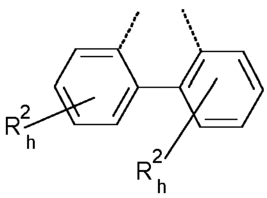

Formel ($L^1$-103)

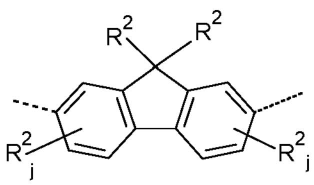

Formel ($L^1$-104)

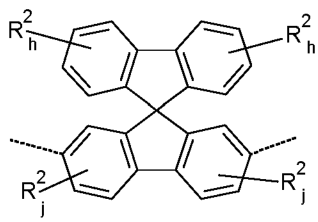

Formel ($L^1$-105)

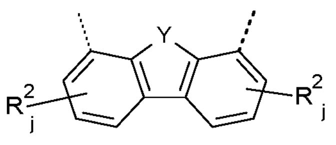

Formel ($L^1$-106)

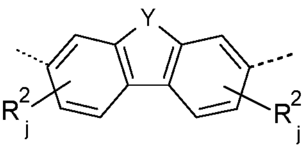

Formel ($L^1$-107)

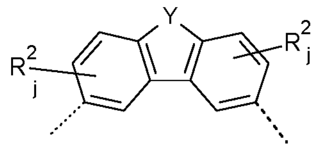

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder $NR^2$, vorzugsweise O oder S ist; und das Symbol $R^2$ die zuvor, insbesondere für Formel (A) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Ferner kann vorgesehen sein, dass eine der Gruppen $L^1$ und/oder $L^2$ in Strukturen der Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V), höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können.

Weiterhin kann vorgesehen sein, dass die Gruppen $L^1$ und/oder $L^2$ insgesamt in Strukturen der Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V), höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können

Bevorzugte erfindungsgemäße Verbindungen umfassen eine Gruppe $L^1$, die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^2$, beispielsweise bei einer Struktur gemäß Formel (A) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$, beispielsweise bei einer Struktur gemäß Formel (A) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Besonders bevorzugt sind erfindungsgemäße Verbindungen, welche die folgenden Eigenschaften aufweisen:

| $Ar^a$ und $Ar^b$ | $L^1$ | Position $L^1$ an Phenylring mit $Y^1$ | Position $L^1$ an Phenylring mit $Y^2$ | Index n für $R^1$ |
|---|---|---|---|---|
| $R^1$-1 bis $R^1$-87 | Bindung oder $L^1$-1 | (Ic), (IIc), (IIIc), (Id), (IId), (IIId) | (Ig), (IIg), (IIIg), (Ih), (IIh), (IIIh) | 0, 1, 2 |
| $R^1$-1 | $L^1$-94 | (Ic), (IIc), (IIIc) | (Ig), (IIg), (IIIg) | 0, 1 |
| $R^1$-1 | $L^1$-94 | (Id), (IId), (IIId) | (Ig), (IIg), (IIIg) | 0, 1 |
| $R^1$-1 | Bindung | (Ic), (IIc), (IIIc) | (Ig), (IIg), (IIIg) | 0, 1 |
| $R^1$-1 | Bindung | (Id), (IId), (IIId) | (Ig), (IIg), (IIIg) | 0, 1 |

Besonders bevorzugt sind Verbindungen, die eine Struktur der Formel (W-1) aufweisen, wobei $Y^3$ eine Gruppe der Formel $C(R^2)_2$ ist und die Verbindungen die folgenden Eigenschaften aufweisen:

| $Ar^a$ und $Ar^b$ | $L^1$ | Position $L^1$ an Phenylring mit $Y^1$ | Position $L^1$ an Phenylring mit $Y^2$ | Index n für $R^1$ |
|---|---|---|---|---|
| $R^1$-1 bis $R^1$-87 | Bindung oder $L^1$-1 | (Ic), (IIc), (IIIc), (Id), (IId), (IIId) | (Ig), (IIg), (IIIg), (Ih), (IIh), (IIIh) | 0, 1, 2 |
| $R^1$-1 | $L^1$-94 | (Ic), (IIc), (IIIc) | (Ig), (IIg), (IIIg) | 0, 1 |
| $R^1$-1 | $L^1$-94 | (Id), (IId), (IIId) | (Ig), (IIg), (IIIg) | 0, 1 |
| $R^1$-1 | Bindung | (Ic), (IIc), (IIIc) | (Ig), (IIg), (IIIg) | 0, 1 |
| $R^1$-1 | Bindung | (Id), (IId), (IIId) | (Ig), (IIg), (IIIg) | 0, 1 |

Ferner sind Verbindungen besonders bevorzugt, bei denen $Y^2$ eine Gruppe der Formel N(Ar) ist und die Verbindungen die folgenden Eigenschaften aufweisen:

| $Ar^a$ und $Ar^b$ | $L^1$ | Position $L^1$ an Phenylring mit $Y^1$ | Position $L^1$ an Phenylring mit $Y^2$ | Index n für $R^1$ |
|---|---|---|---|---|
| $R^1$-1 bis $R^1$-87 | Bindung oder $L^1$-1 | (Ic), (IIc), (IIIc), (Id), (IId), (IIId) | (Ig), (IIg), (IIIg), (Ih), (IIh), (IIIh) | 0, 1, 2 |
| $R^1$-1 | $L^1$-94 | (Ic), (IIc), (IIIc) | (Ig), (IIg), (IIIg) | 0, 1 |
| $R^1$-1 | $L^1$-94 | (Id), (IId), (IIId) | (Ig), (IIg), (IIIg) | 0, 1 |
| $R^1$-1 | Bindung | (Ic), (IIc), (IIIc) | (Ig), (IIg), (IIIg) | 0, 1 |
| $R^1$-1 | Bindung | (Id), (IId), (IIId) | (Ig), (IIg), (IIIg) | 0, 1 |

Der Index g in Formel $R^1$-1 in zuvor genannten Tabellen ist vorzugsweise 0, 1, 2 oder 3, besonders bevorzugt 0 oder 1, speziell bevorzugt 0; der Index h in Formel $L^1$-1 beziehungsweise $L^1$-94 in zuvor genannten Tabellen ist vorzugsweise 0, 1, 2 oder 3, besonders bevorzugt 0 oder 1, speziell bevorzugt 0.

In den zuvor dargelegten Tabellen bedeutet die Zuordnung $Ar^a$ und $Ar^b$ ist $R^1$-1 bis $R^1$-87, dass sowohl die Gruppe $Ar^a$ als auch die Gruppe $Ar^b$ ausgewählt ist aus Resten der zuvor dargelegten Formeln $R^1$-1 bis $R^1$-87, vorzugsweise $R^1$-1. Die Zuordnung $L^1$ ist eine Bindung oder $L^1$-1 bedeutet, dass die Gruppe $L^1$ in den zuvor dargelegten Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V) jeweils eine Bindung oder einen Rest der zuvor dargelegten Formel $L^1$-1, vorzugsweise $L^1$-94 darstellt. Die Zuordnung, dass die Position $L^1$ an Phenylring mit $Y^1$ vorzugsweise der Formel (Ic), (IIc), (IIIc), (Id), (IId), (IIId) entspricht, bedeutet, dass die Gruppe $L^1$ vorzugsweise in para- oder meta-Stellung zur Gruppe $Y^1$ steht, wie dies in Formel (Ic), (IIc), (IIIc), (Id), (IId), (IIId) dargestellt ist. Die Zuordnung, dass die Position $L^1$ an Phenylring mit $Y^2$ vorzugsweise der Formel (Ig), (IIg), (IIIg), (Ih), (IIh), (IIIh) entspricht, bedeutet, dass die Gruppe $L^1$ vorzugsweise in para- oder meta-Stellung zur Gruppe $Y^2$ steht, wie dies in Formel (Ig), (IIg), (IIIg), (Ih), (IIh), (IIIh) dargestellt ist. Die Zuordnung, dass der Index n für $R^1$ 0, 1, 2 ist, bedeutet, dass in den zuvor dargelegten Formeln (A), (I), (II), (III), (Ia), (IIa), (IIIa), (Ib), (IIb), (IIIb), (Ic), (IIc), (IIIc), (Id), (IId), (IIId), (Ie), (IIe), (IIIe), (If), (IIf), (IIIf), (Ig), (IIg), (IIIg), (Ih), (IIh), (IIIh), (IV) und/oder (V) der Index n jeweils 0, 1 oder 2, vorzugsweise 0 oder 1 und speziell bevorzugt 0 ist.

Beispiele für geeignete erfindungsgemäße Verbindungen sind die nachstehend gezeigten Strukturen gemäß den folgenden Formeln 1 bis 217:

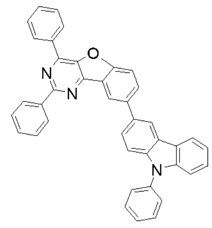

Formel 1

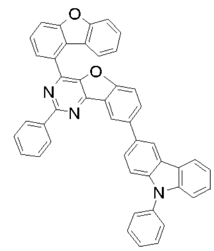

Formel 2

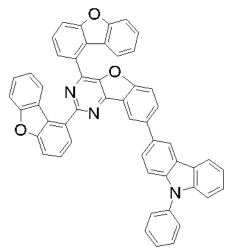

Formel 3

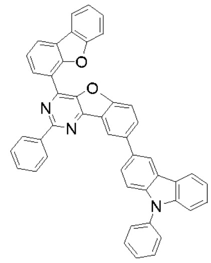

Formel 4

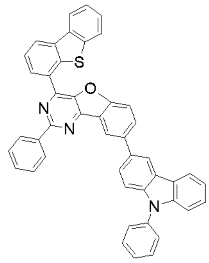

Formel 5

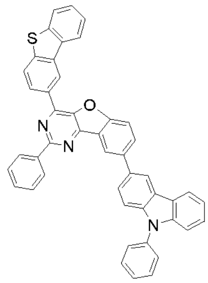

Formel 6

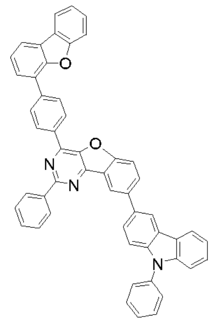

Formel 7

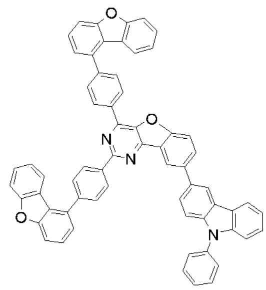

Formel 8

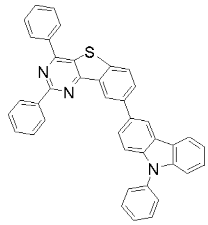

Formel 9

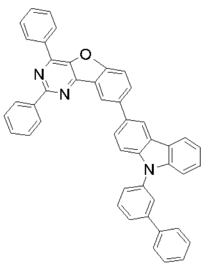

Formel 10

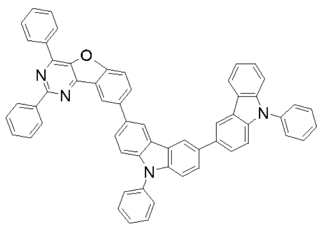

Formel 11

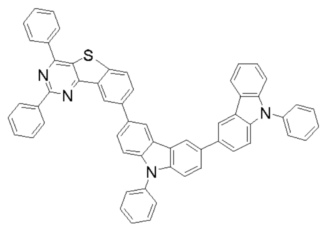

Formel 12

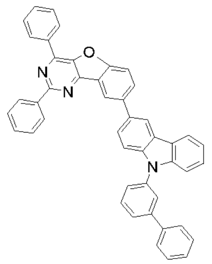

Formel 13

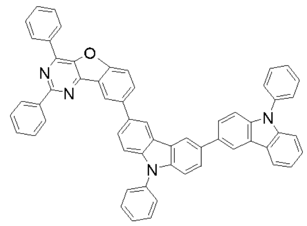

Formel 14

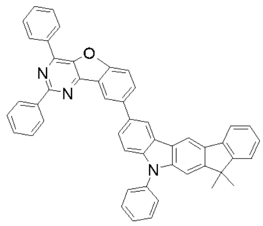

Formel 15

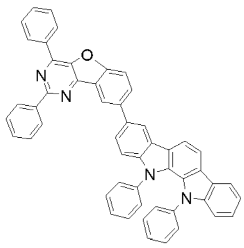

Formel 16

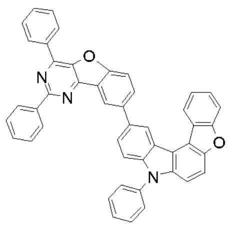

Formel 17

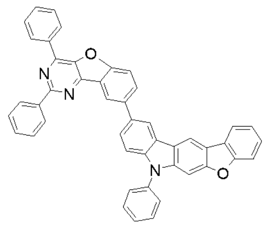

Formel 18

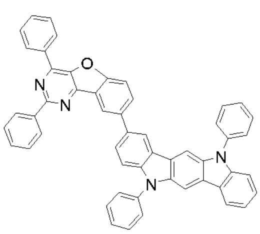

Formel 19

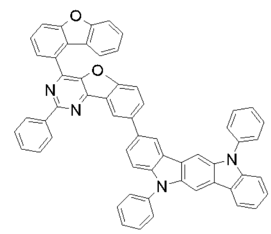

Formel 20

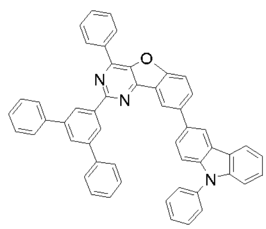

Formel 21

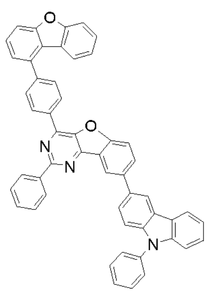

Formel 22

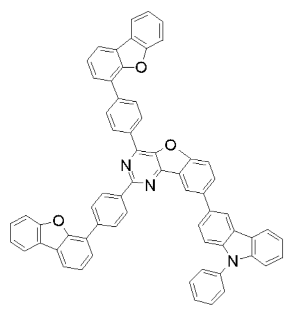

Formel 23

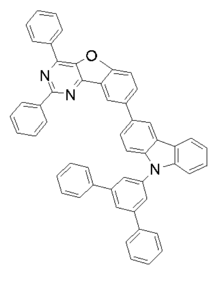

Formel 24

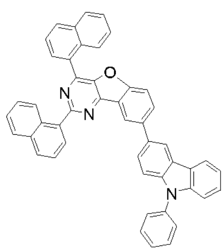

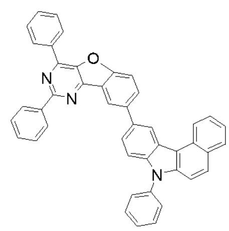

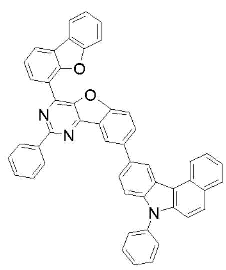

Formel 25 Formel 26 Formel 27

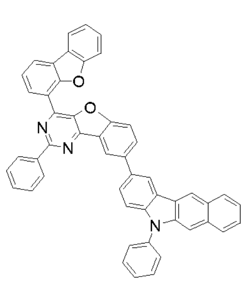
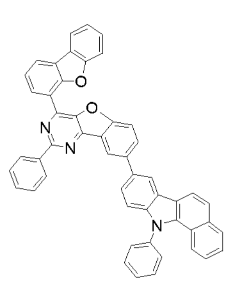
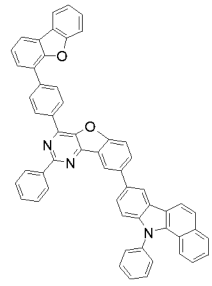

Formel 28 Formel 29 Formel 30

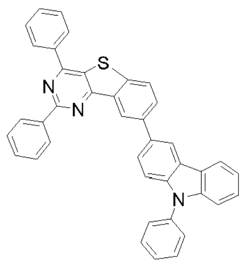
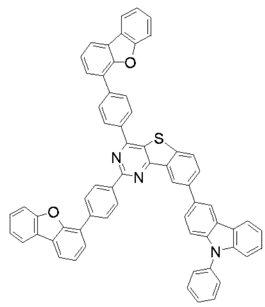
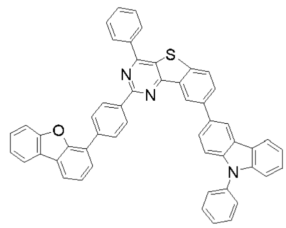

Formel 31 Formel 32 Formel 33

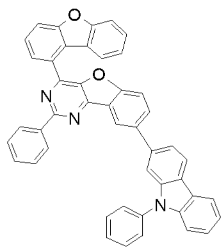
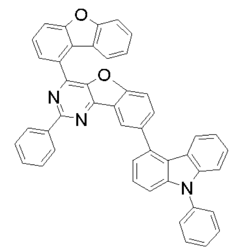
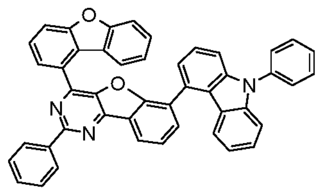

Formel 34 Formel 35 Formel 36

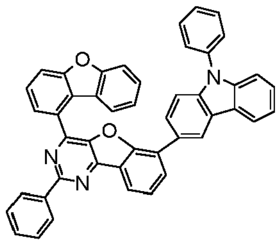
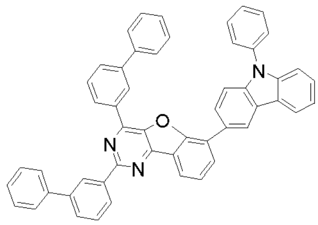
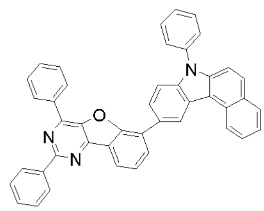

Formel 37 Formel 38 Formel 39

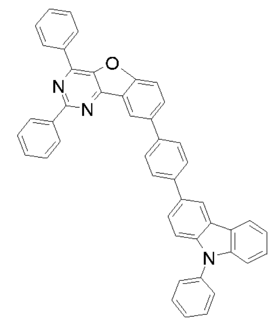

Formel 40

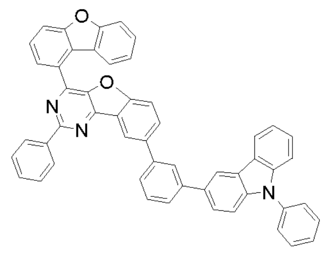

Formel 41

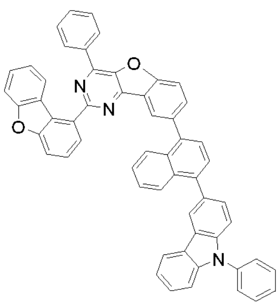

Formel 42

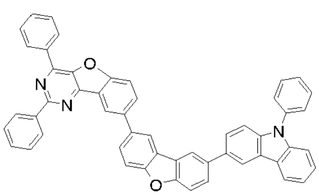

Formel 43

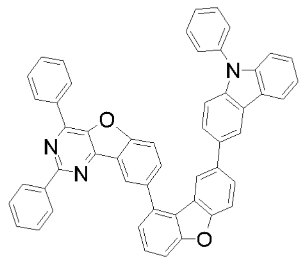

Formel 44

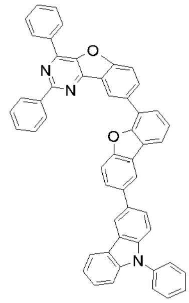

Formel 45

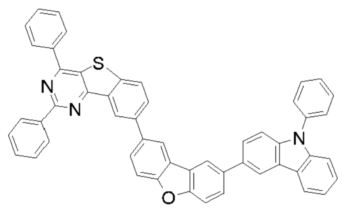

Formel 46

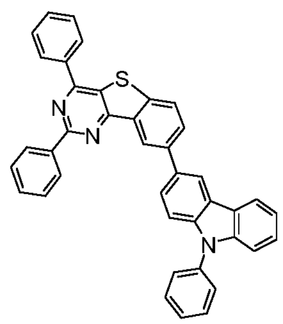

Formel 47

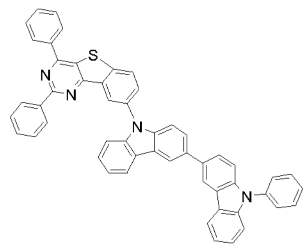

Formel 48

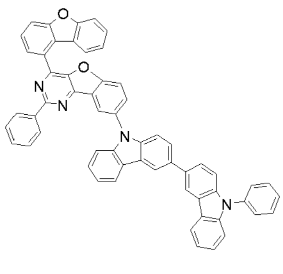

Formel 49

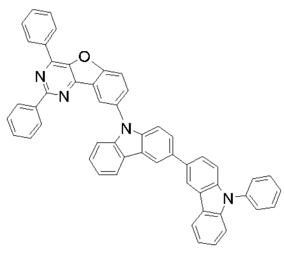

Formel 50

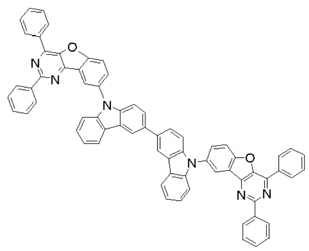

Formel 51

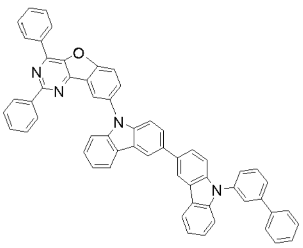
Formel 52

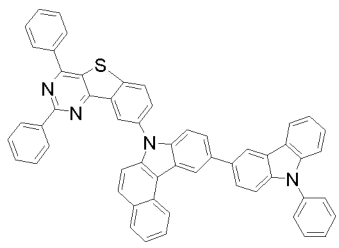
Formel 53

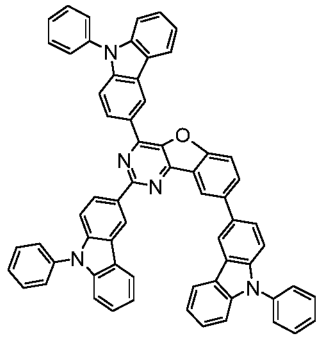
Formel 54

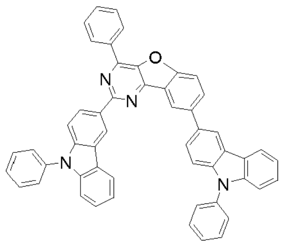
Formel 55

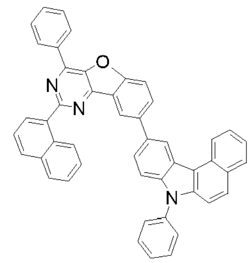
Formel 56

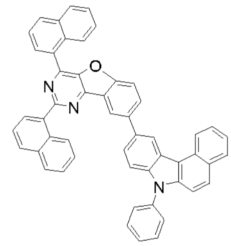
Formel 57

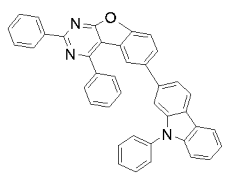
Formel 58

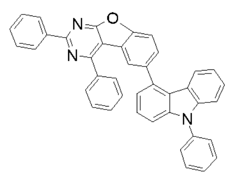
Formel 59

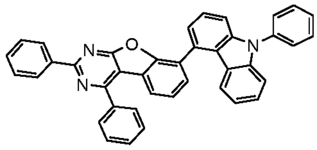
Formel 60

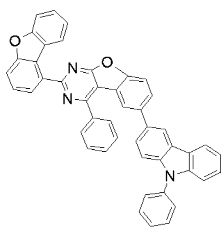
Formel 61

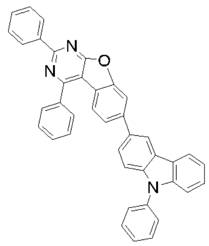
Formel 62

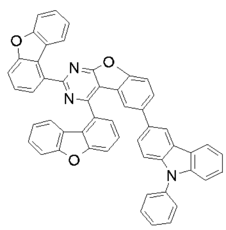
Formel 63

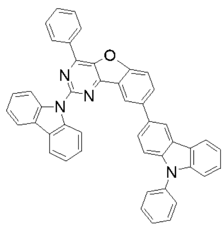
Formel 64

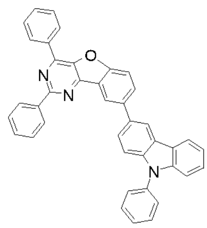
Formel 65

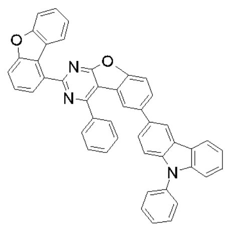
Formel 66

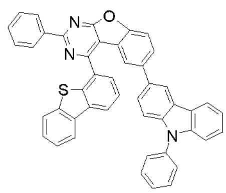
Formel 67

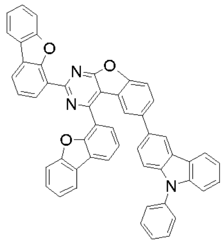
Formel 68

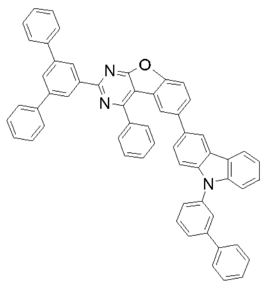
Formel 69

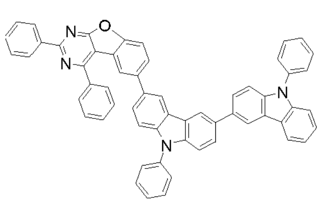
Formel 70

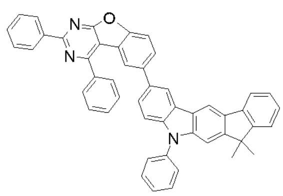
Formel 71

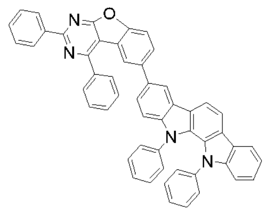
Formel 72

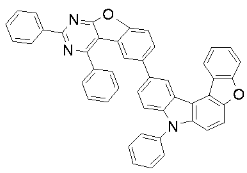
Formel 73

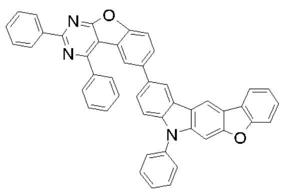
Formel 74

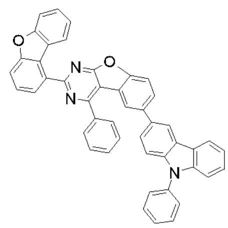
Formel 75

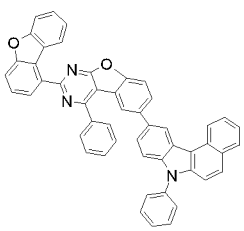
Formel 76

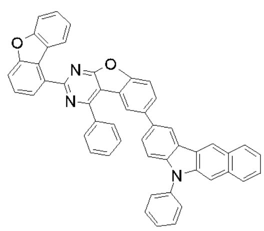
Formel 77

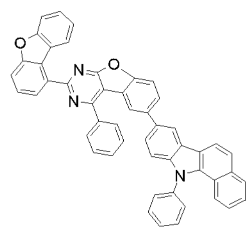
Formel 78

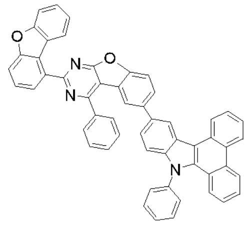
Formel 79

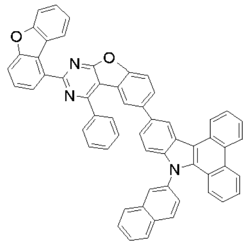
Formel 80

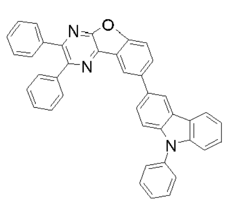
Formel 81

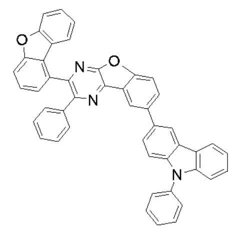

Formel 82

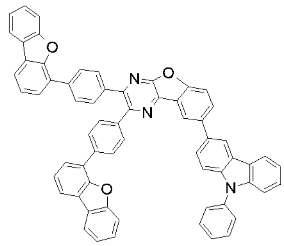

Formel 83

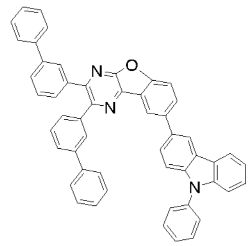

Formel 84

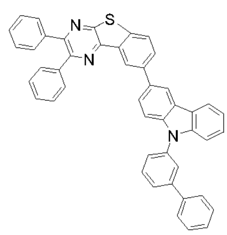

Formel 85

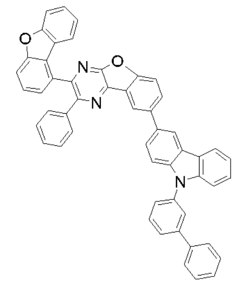

Formel 86

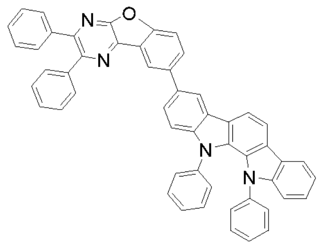

Formel 87

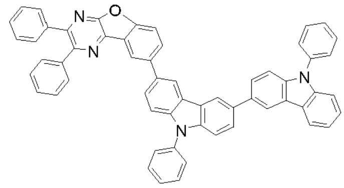

Formel 88

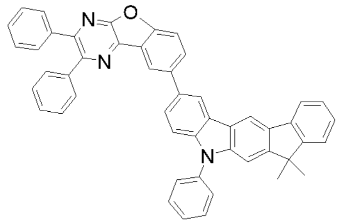

Formel 89

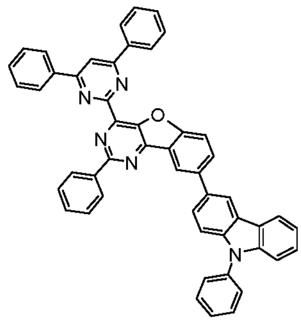

Formel 90

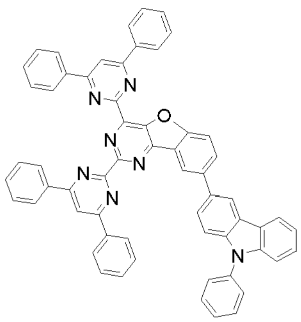

Formel 91

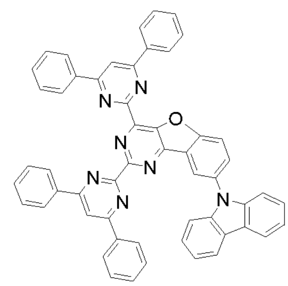

Formel 92

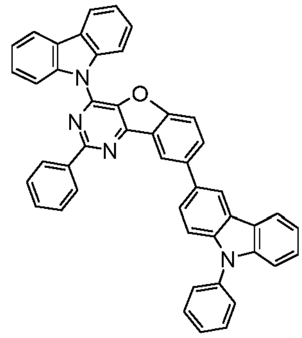

Formel 93

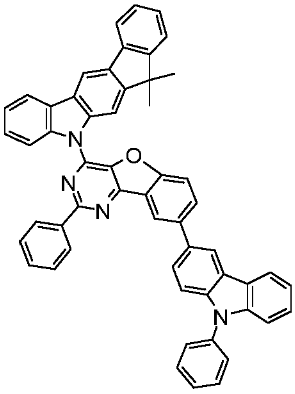
Formel 94

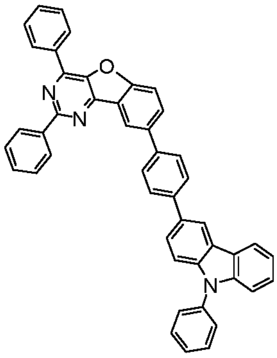
Formel 95

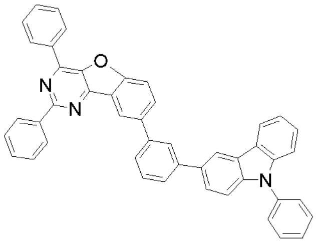
Formel 96

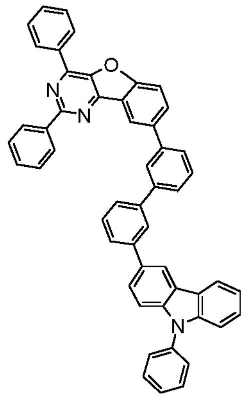
Formel 97

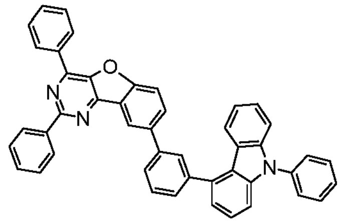
Formel 98

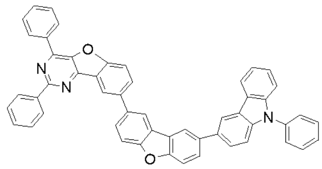
Formel 99

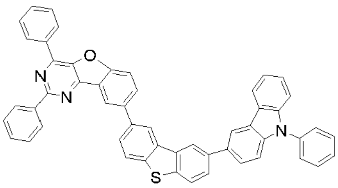
Formel 100

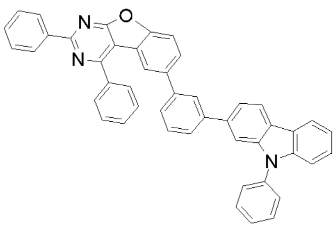
Formel 101

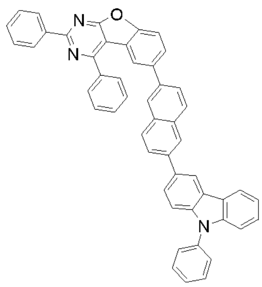
Formel 102

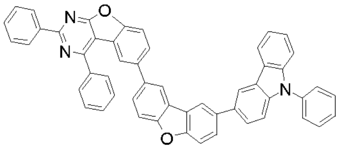
Formel 103

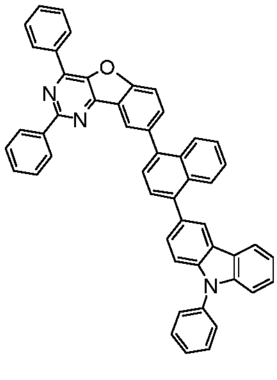
Formel 104

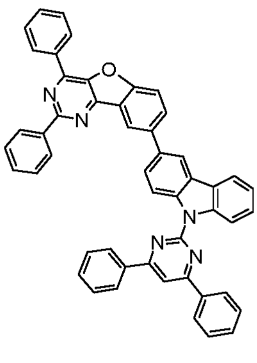
Formel 105

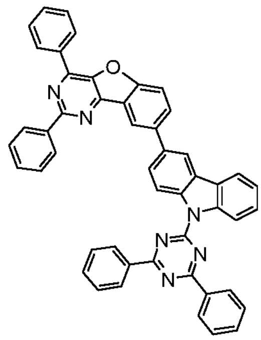

Formel 106

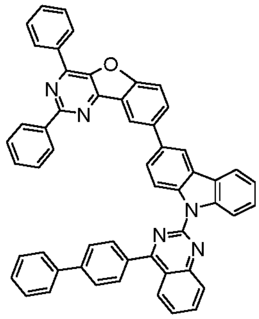

Formel 107

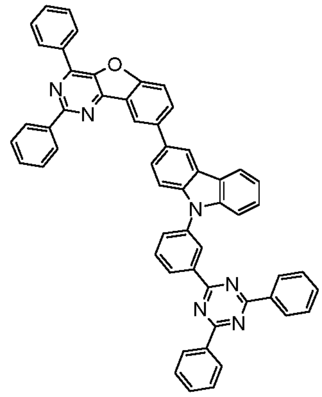

Formel 108

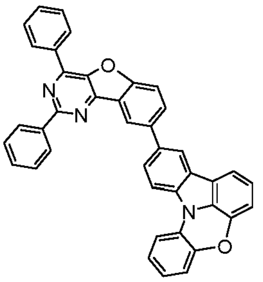

Formel 109

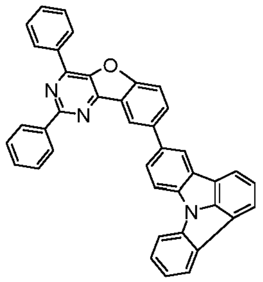

Formel 110

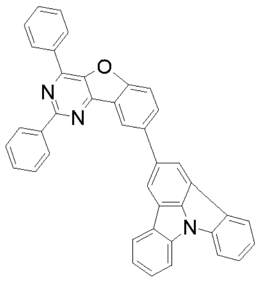

Formel 111

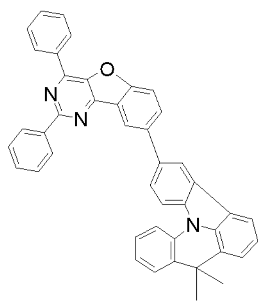

Formel 112

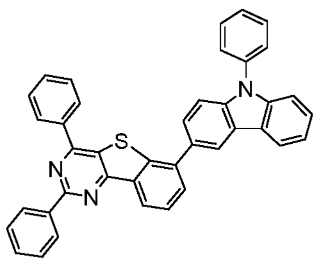

Formel 113

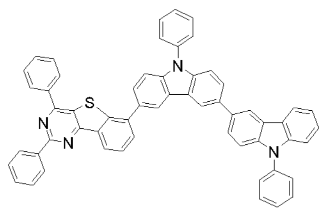

Formel 114

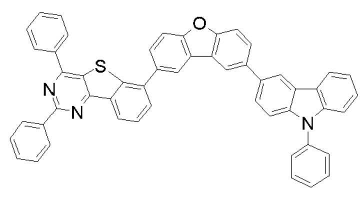

Formel 115

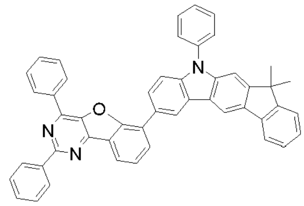

Formel 116

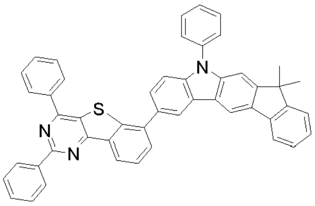

Formel 117

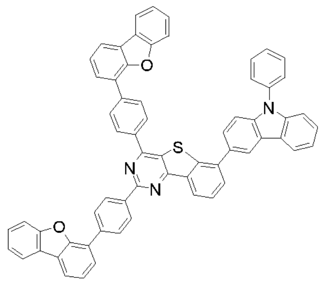
Formel 118

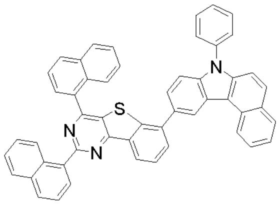
Formel 119

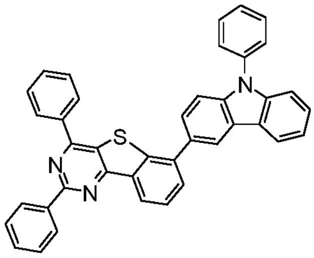
Formel 120

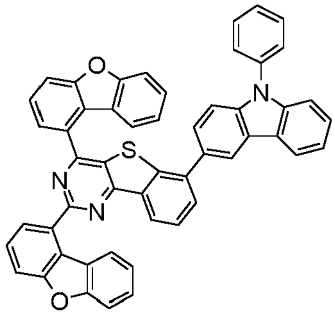
Formel 121

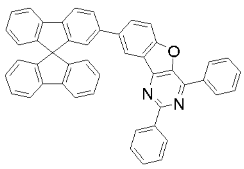
Formel 122

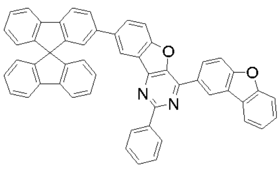
Formel 123

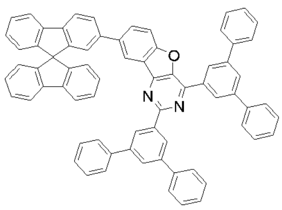
Formel 124

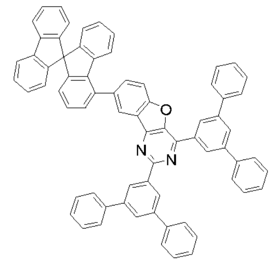
Formel 125

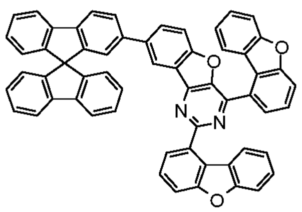
Formel 126

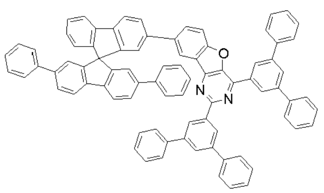
Formel 127

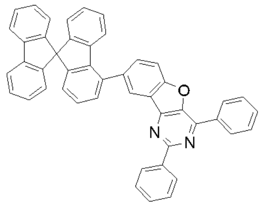
Formel 128

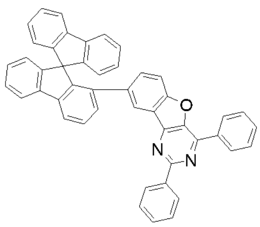
Formel 129

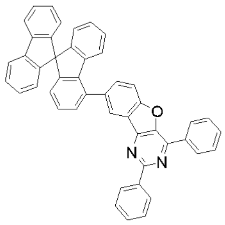
Formel 130

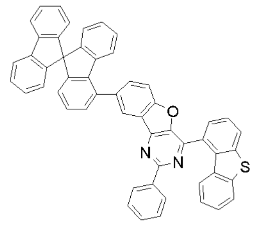
Formel 131

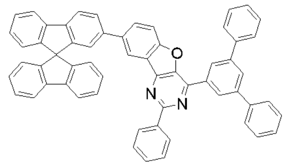
Formel 132

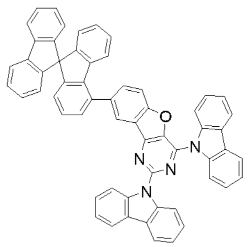
Formel 133

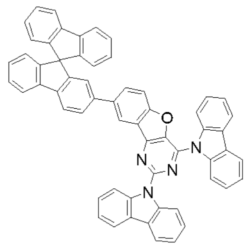
Formel 134

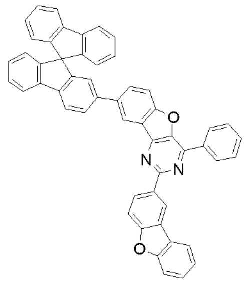
Formel 135

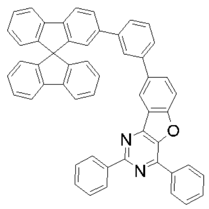
Formel 136

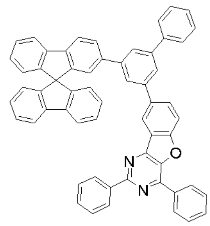
Formel 137

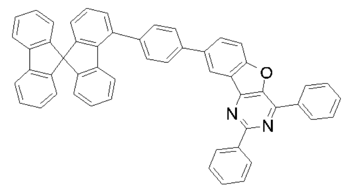
Formel 138

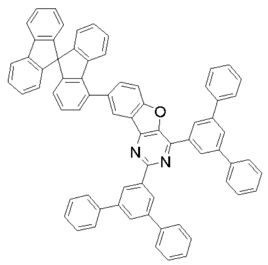
Formel 139

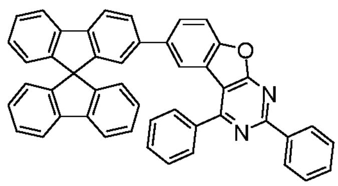
Formel 140

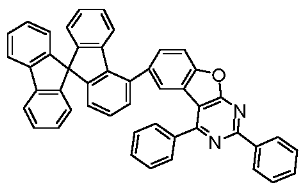
Formel 141

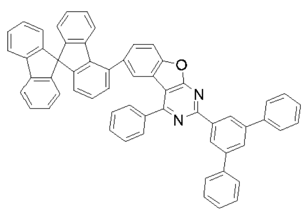
Formel 142

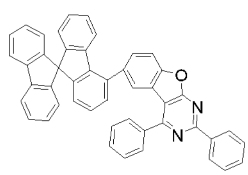
Formel 143

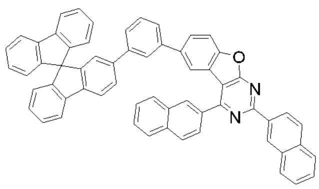
Formel 144

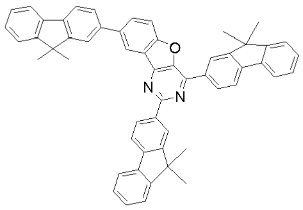

Formel 145

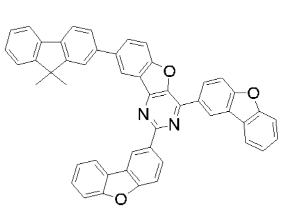

Formel 146

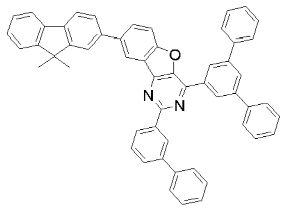

Formel 147

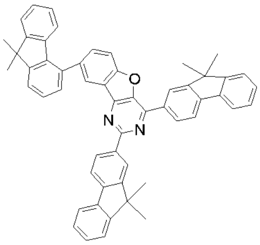

Formel 148

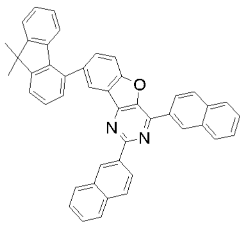

Formel 149

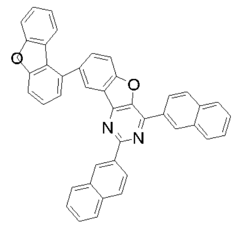

Formel 150

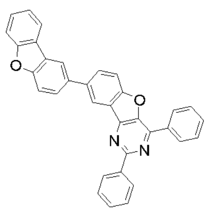

Formel 151

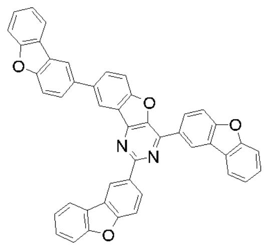

Formel 152

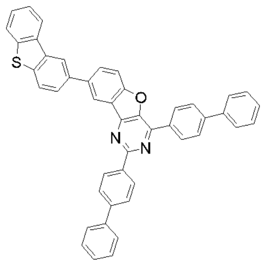

Formel 153

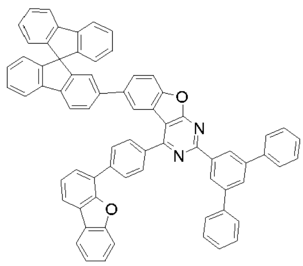

Formel 154

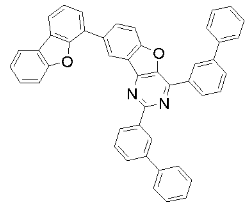

Formel 155

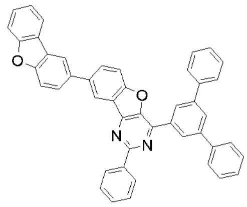

Formel 156

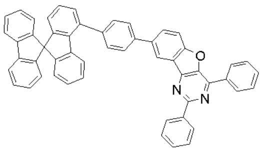

Formel 157

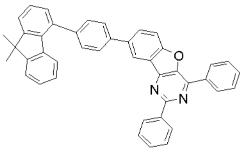

Formel 158

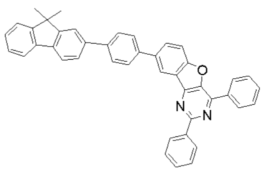

Formel 159

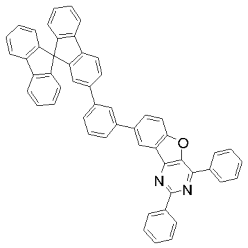
Formel 160

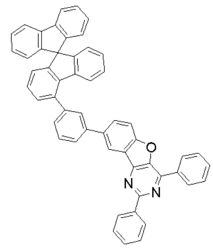
Formel 161

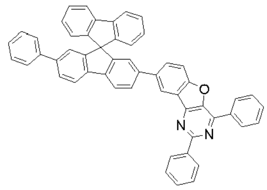
Formel 162

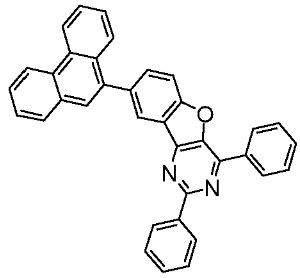
Formel 163

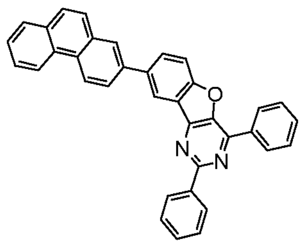
Formel 164

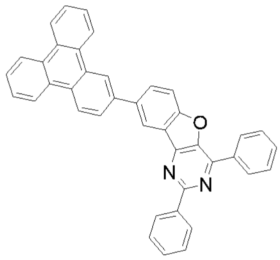
Formel 165

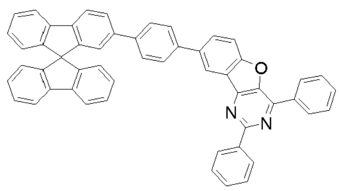
Formel 166

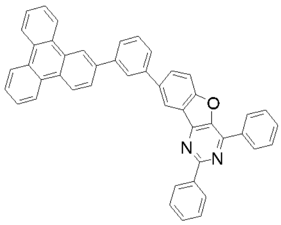
Formel 167

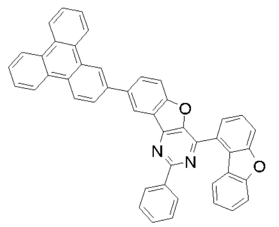
Formel 168

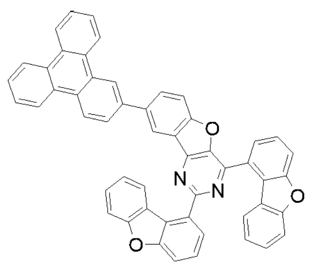
Formel 169

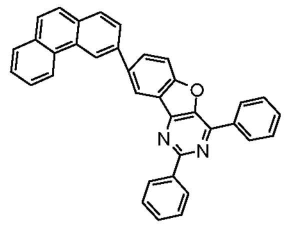
Formel 170

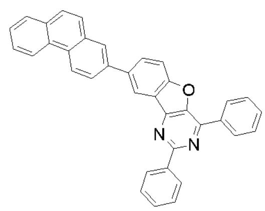
Formel 171

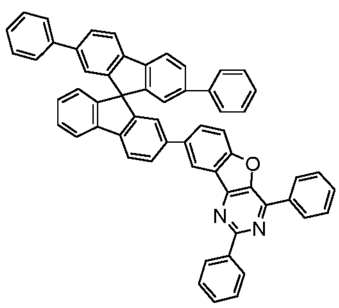
Formel 172

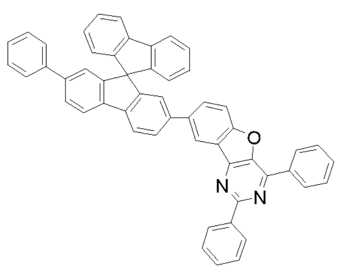
Formel 173

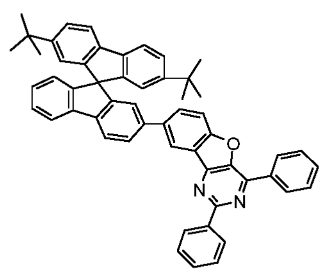
Formel 174

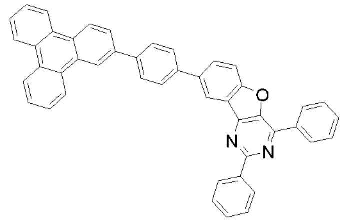
Formel 175

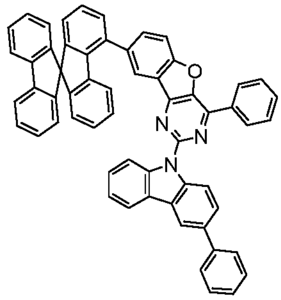
Formel 176

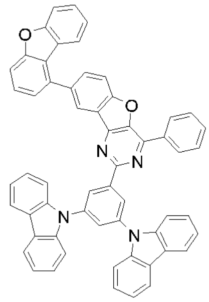
Formel 177

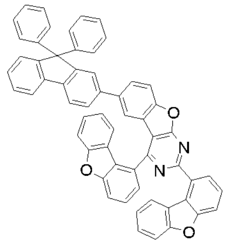
Formel 178

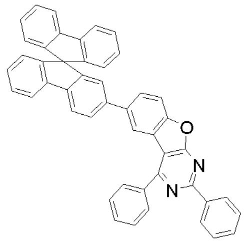
Formel 179

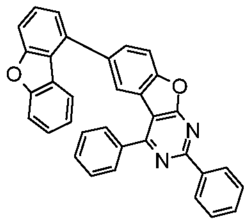
Formel 180

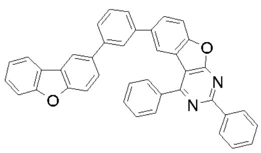
Formel 181

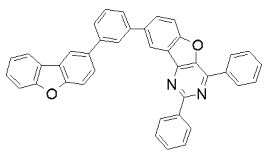
Formel 182

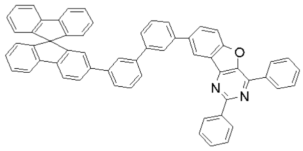
Formel 183

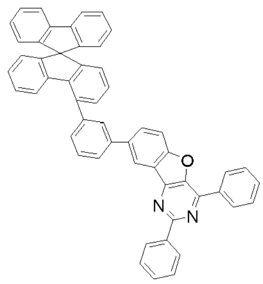
Formel 184

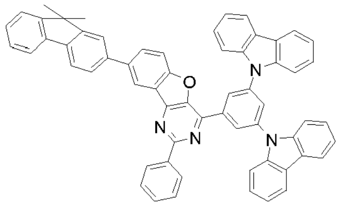
Formel 185

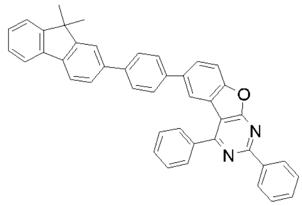
Formel 186

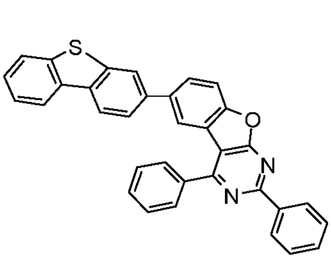
Formel 187

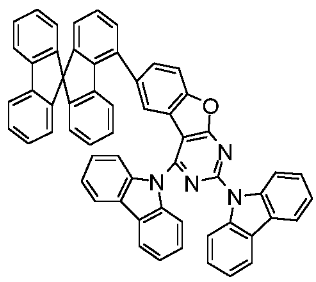
Formel 188

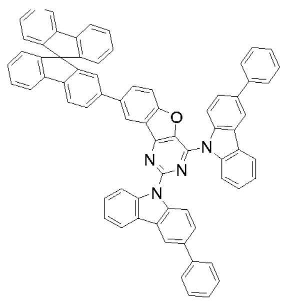
Formel 189

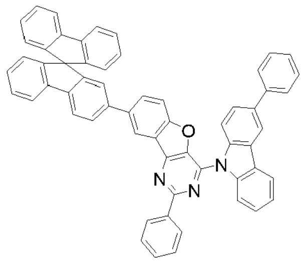

Formel 190

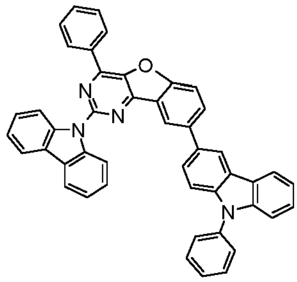

Formel 191

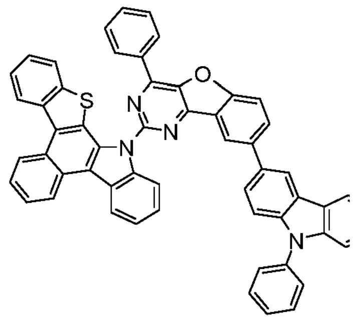

Formel 192

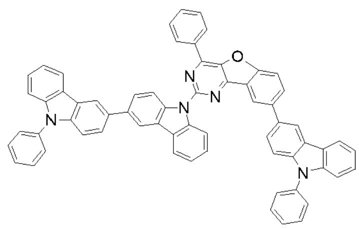

Formel 193

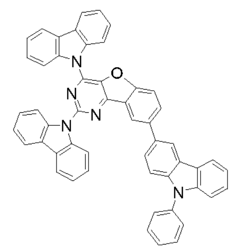

Formel 194

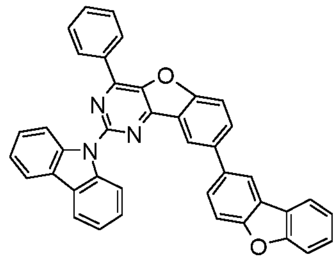

Formel 195

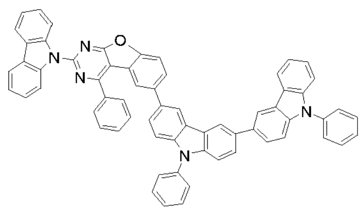

Formel 196

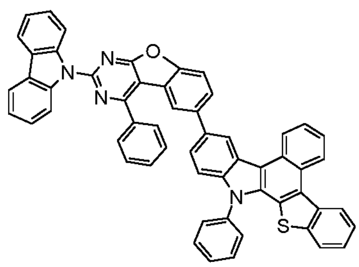

Formel 197

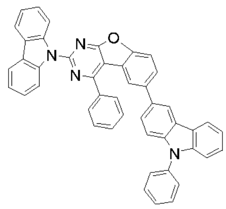

Formel 198

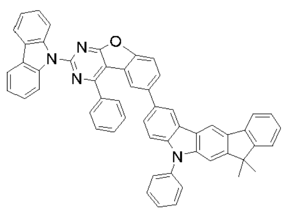

Formel 199

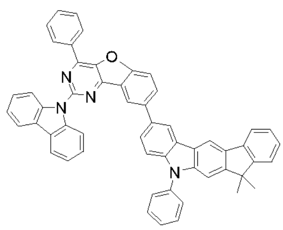

Formel 200

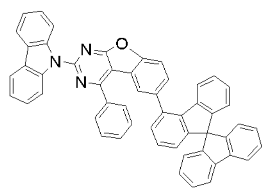

Formel 201

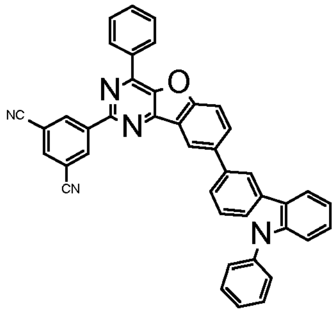

Formel 202

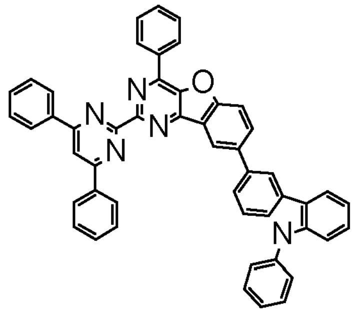

Formel 203

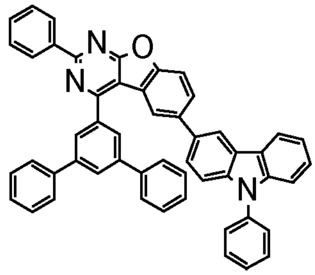

Formel 204

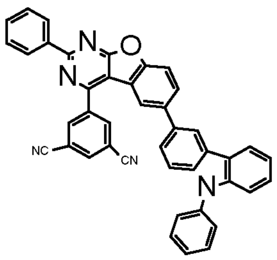

Formel 205

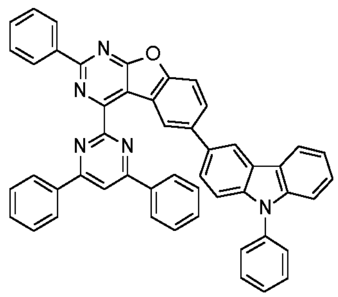

Formel 206

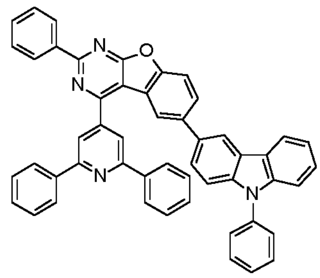

Formel 207

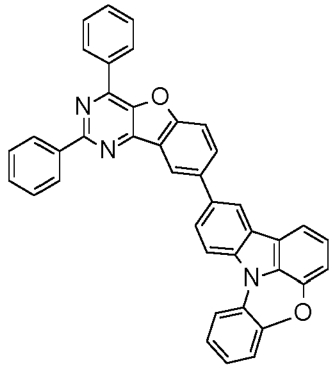

Formel 208

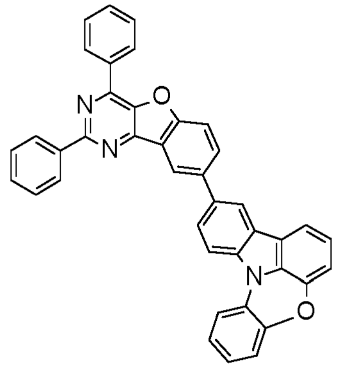

Formel 209

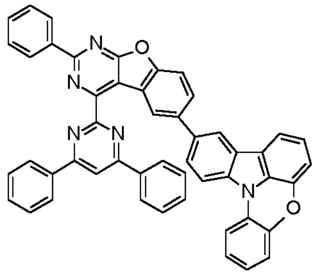

Formel 210

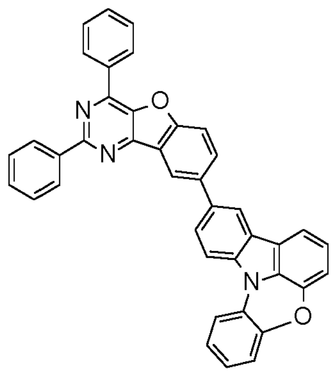

Formel 211

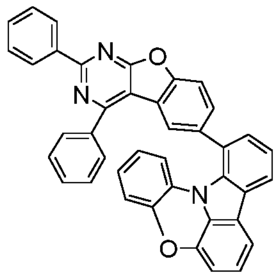

Formel 212

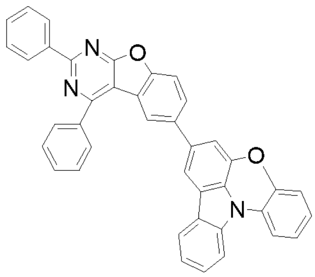

Formel 213

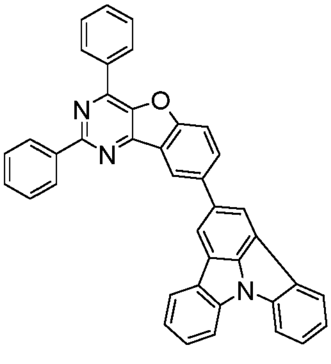

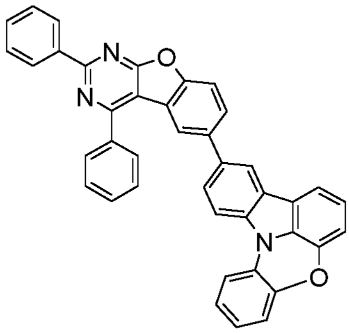

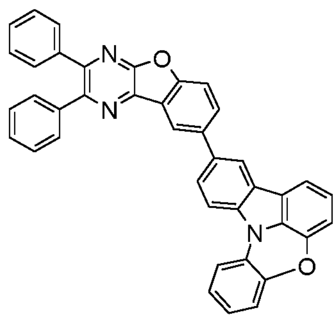

Formel 214 Formel 215 Formel 216

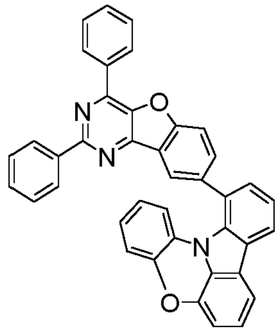

Formel 217

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen der Formel (A), bei dem in einer Kupplungsreaktion eine Verbindung, umfassend mindestens eine Diazadibenzofuran- oder Diazadibenzothiophen-Gruppe, mit einer Gruppe, umfassend mindestens einen Carbazol-, Fluoren-, Phenanthren-, Benzofuran- und/oder Benzothiophen-Rest, verbunden wird.

Geeignete Verbindungen mit einer Diazadibenzofuran- oder Diazadibenzothiophen-Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

## Synthese Schema 1

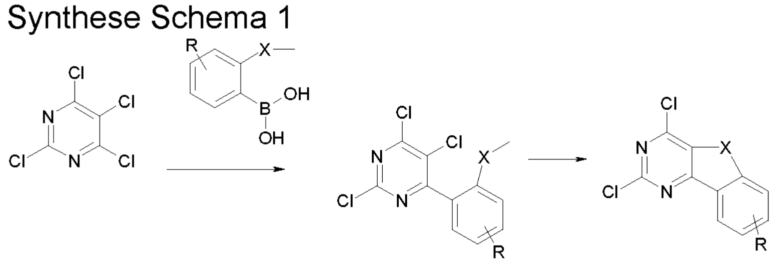

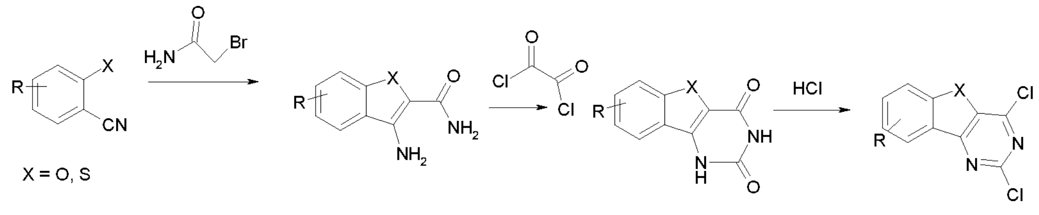


## Synthese Schema 2

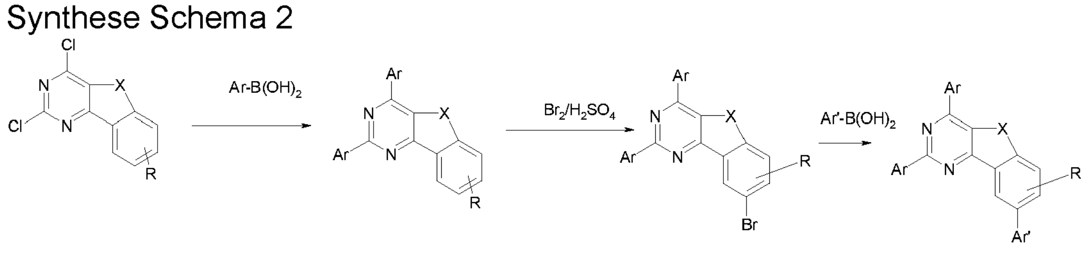


## Synthese Schema 3

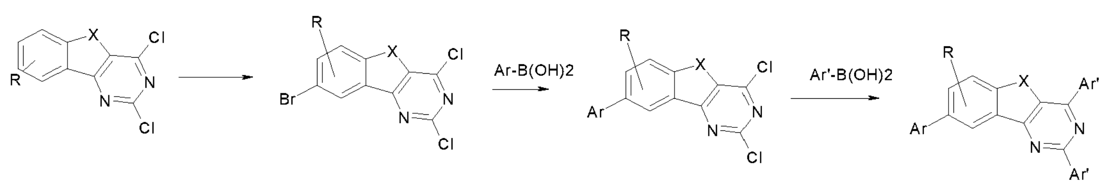


## Synthese Schema 4

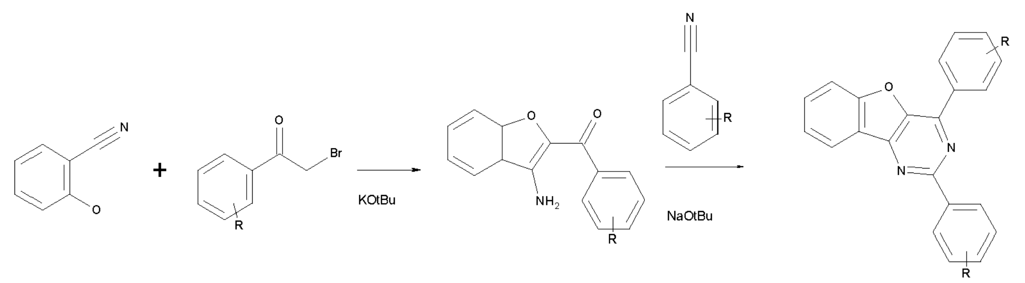


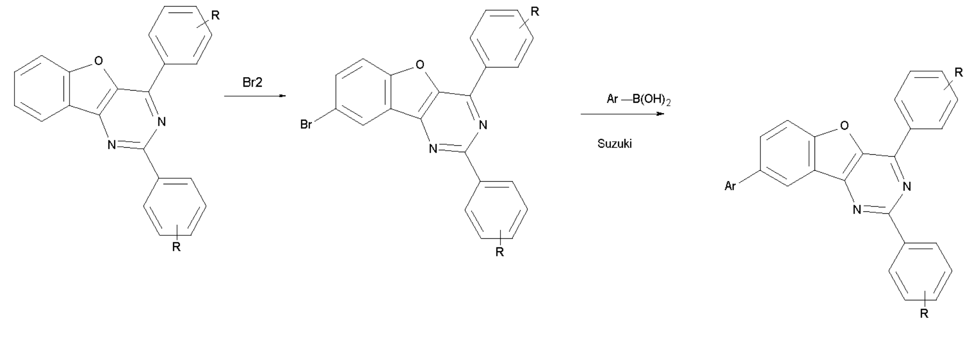

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen der Formel (A) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen der Formel (A) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (A) oder erfindungsgemäße Verbindungen, wobei ein oder mehrere Bindungen der erfindungsgemäßen Verbindungen oder der Strukturen der Formel (A) zum Polymer, Oligomer oder Dendrimer vorhanden sind. Je nach Verknüpfung der Strukturen der Formel (A) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (A) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen der allgemeinen Formel (A) bzw. die zuvor und nachfolgend ausgeführten bevorzugten Ausführungsformen bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol,

Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung der Formel (A) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine Verbindungder Formel (A) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung der Formel (A) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960 und den noch nicht offen gelegten Anmeldungen EP 13004411.8, EP 14000345.0, EP 14000417.7 und EP 14002623.8 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

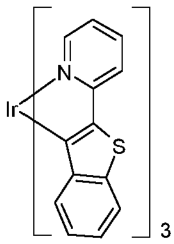
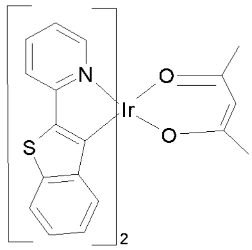
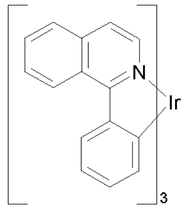

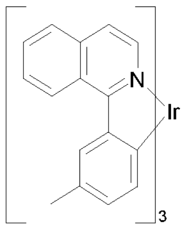
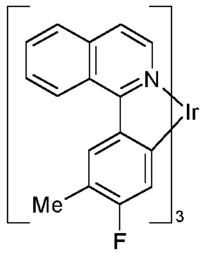
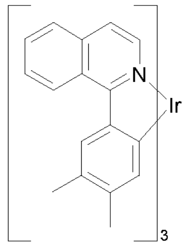

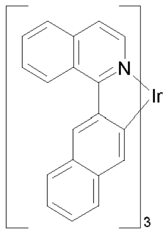
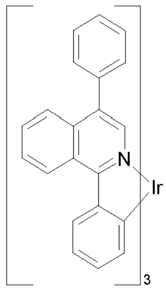
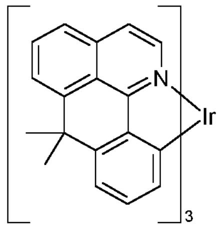

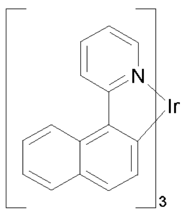
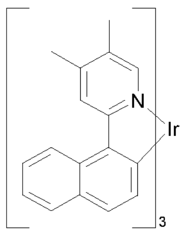
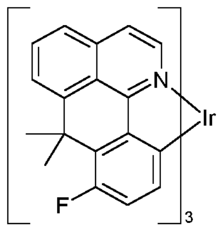

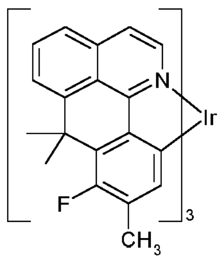
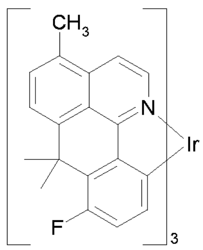
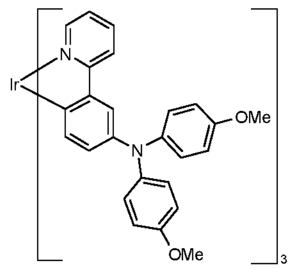

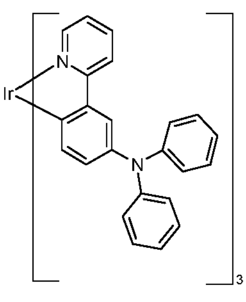
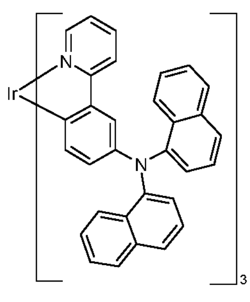
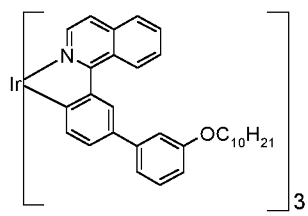

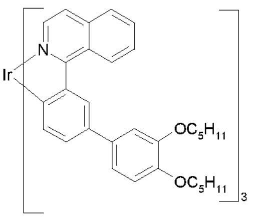
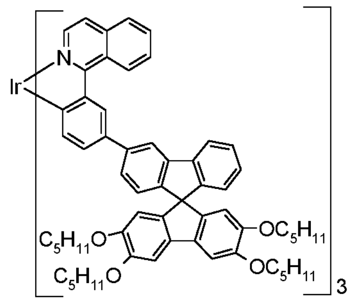
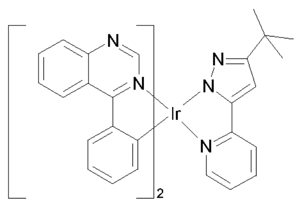
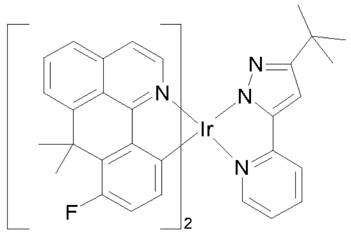
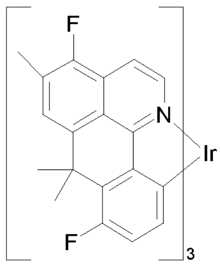
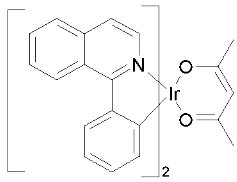
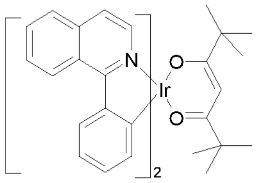
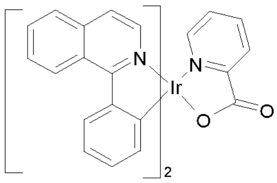
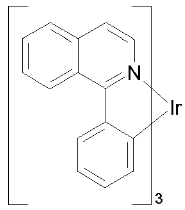
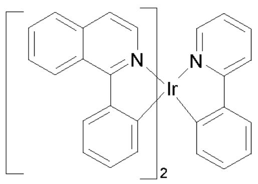
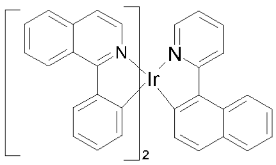
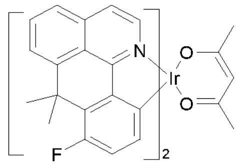
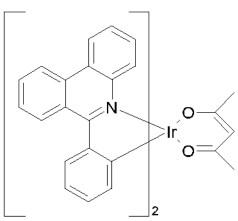
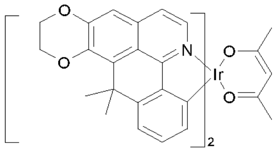
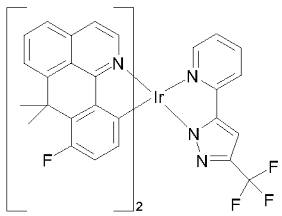
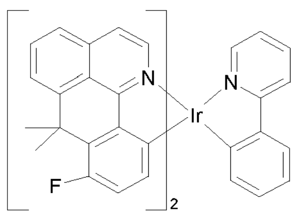
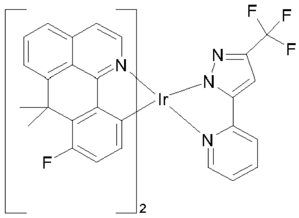
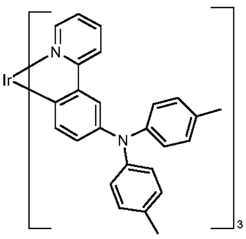
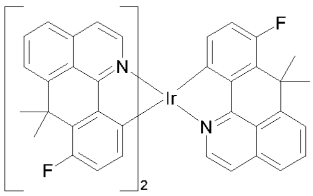
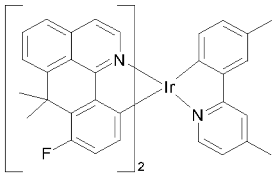
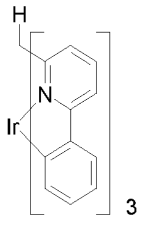

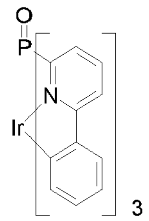

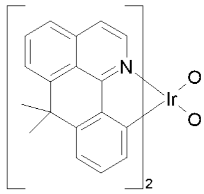

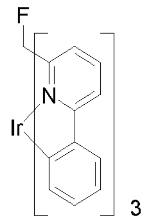

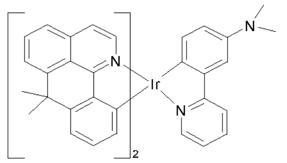

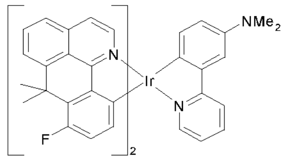

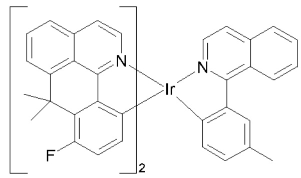

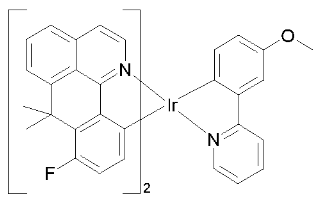

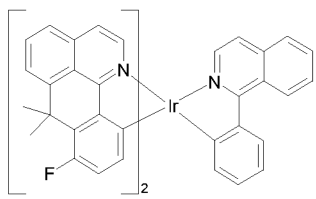

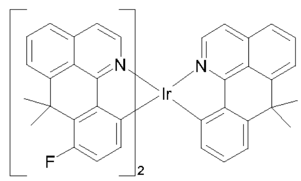

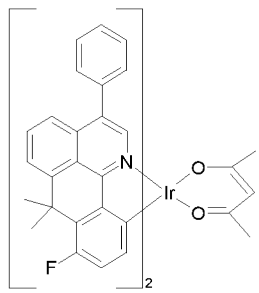

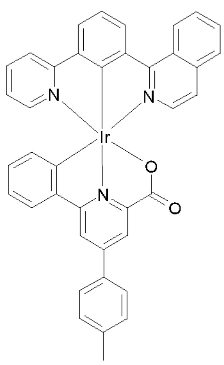

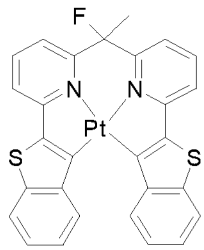

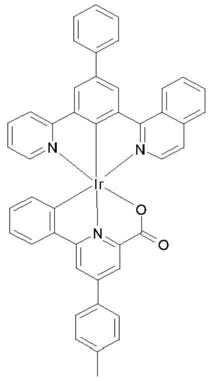

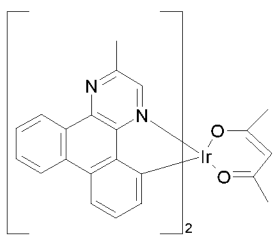

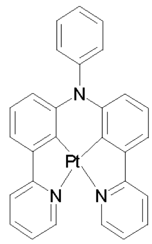

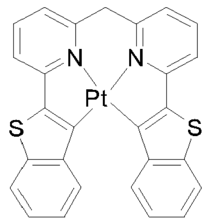

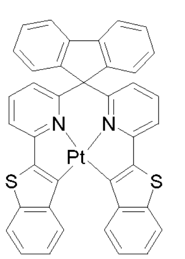

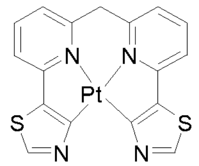

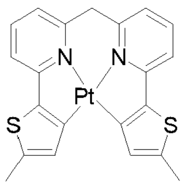
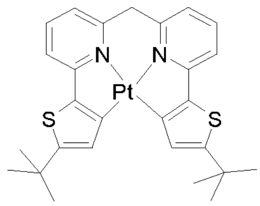
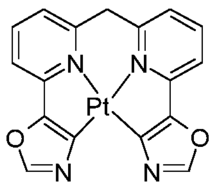
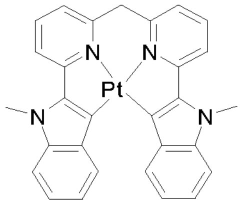
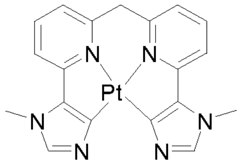
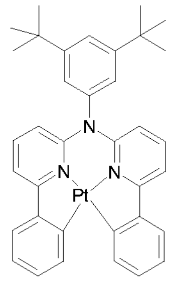
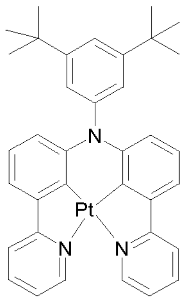
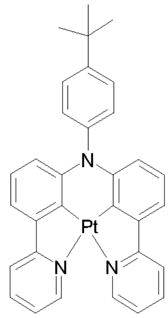
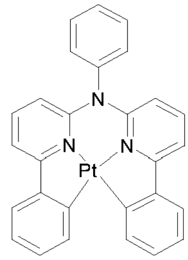
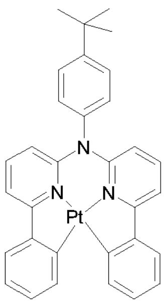
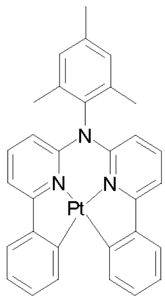
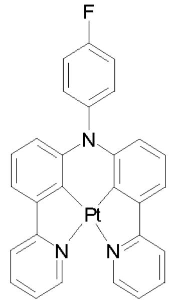
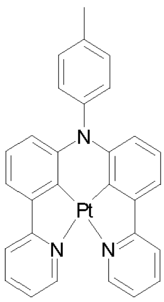
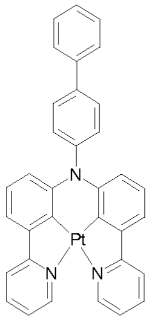
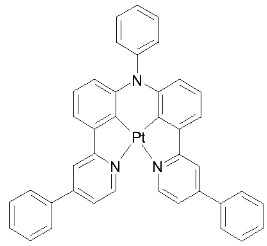

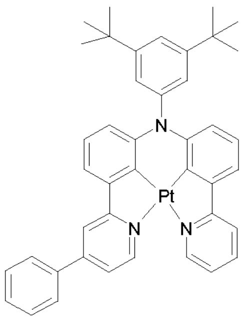
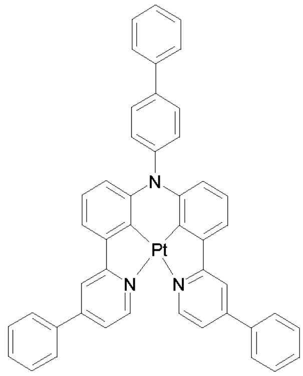
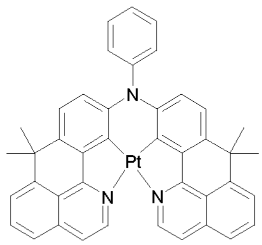
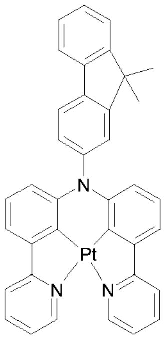
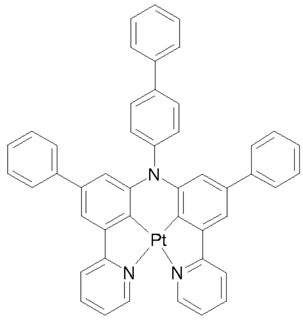
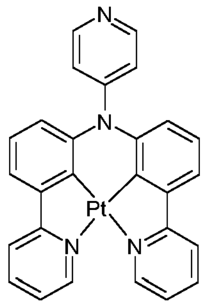
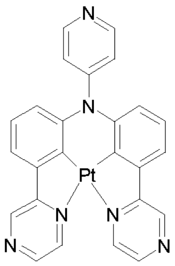
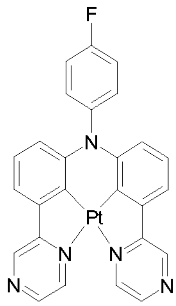
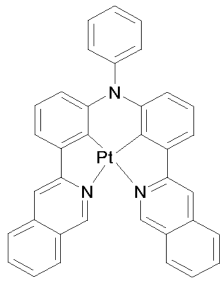
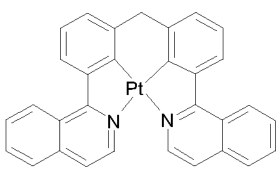
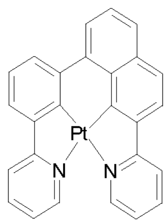
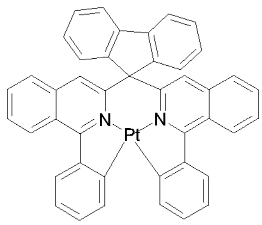
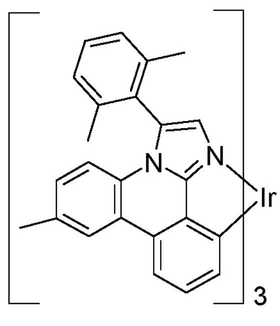
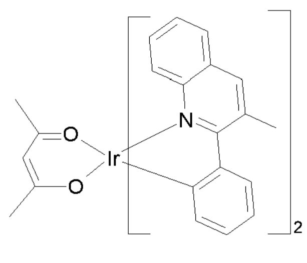
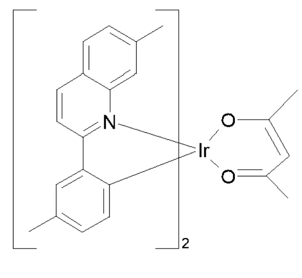

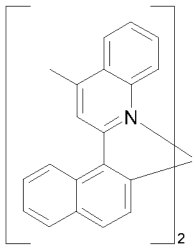
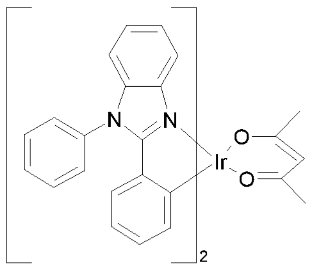
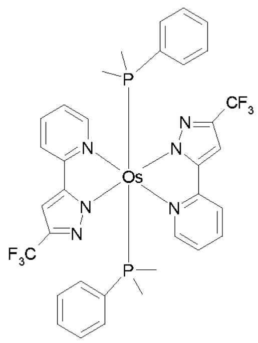

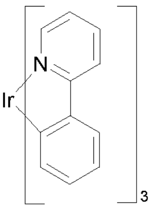
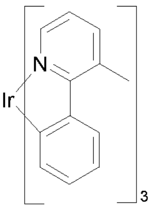
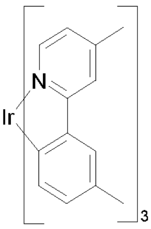

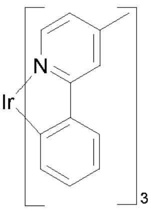
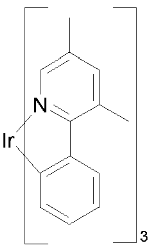
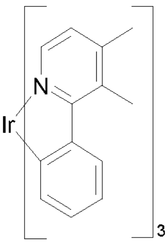

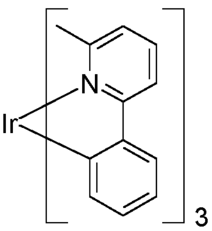
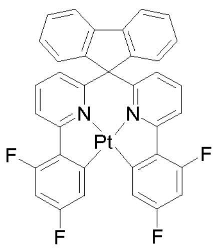
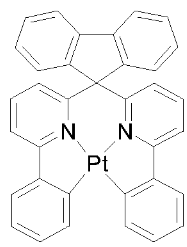

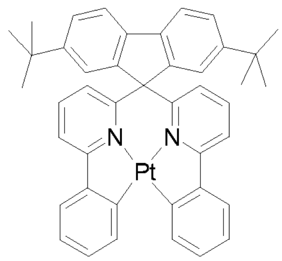
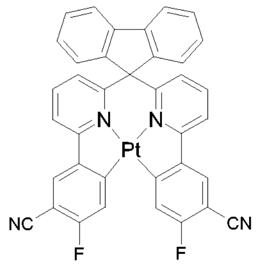
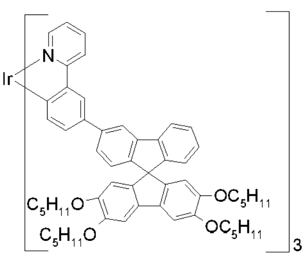

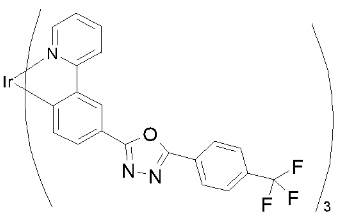
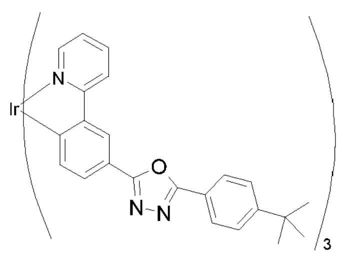
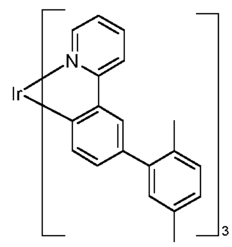

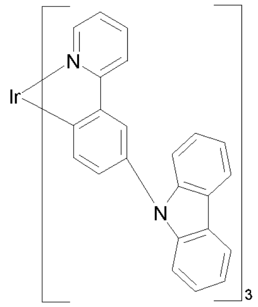
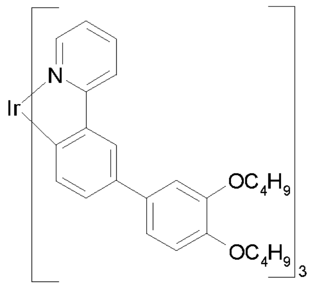
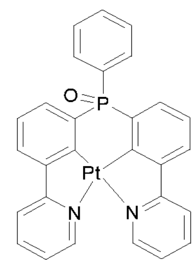
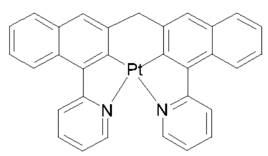
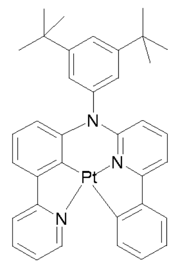
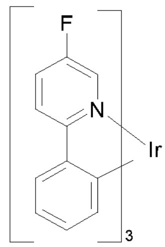
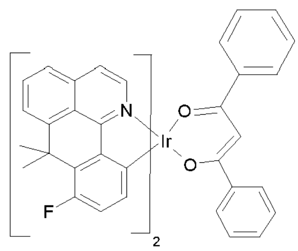
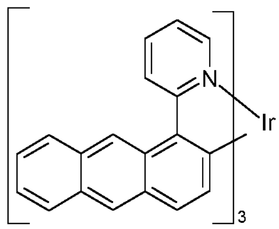
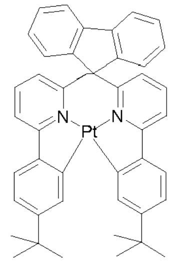
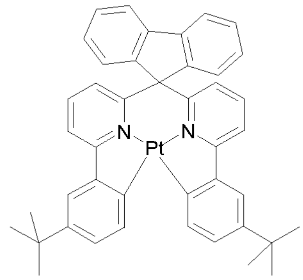
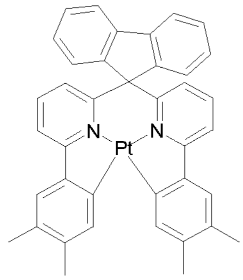
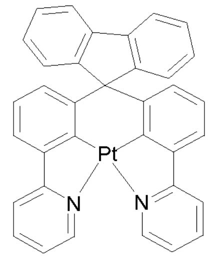
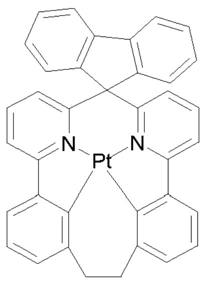
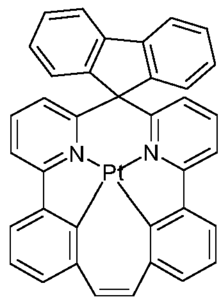
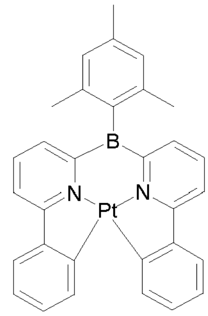

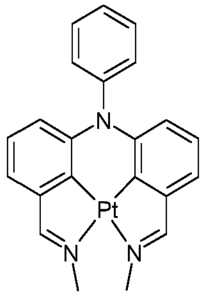
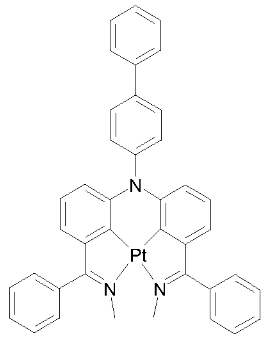
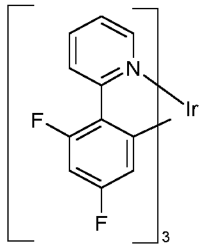

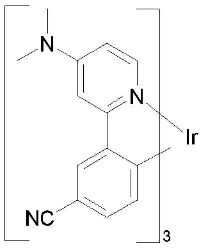
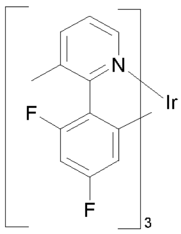
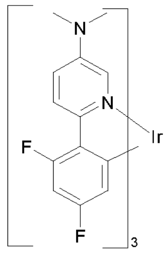

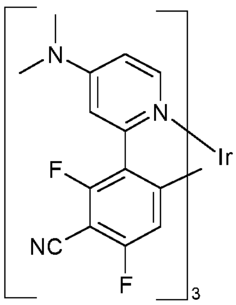
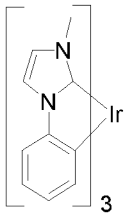
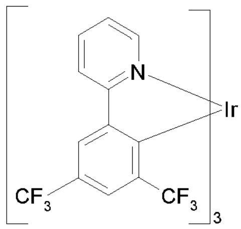

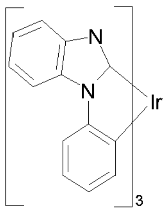
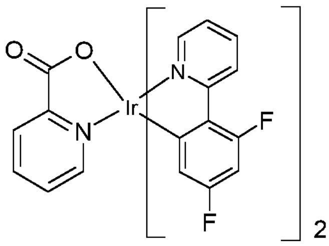
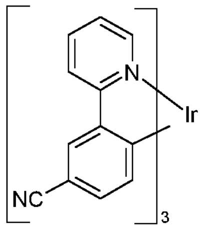

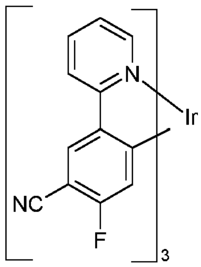
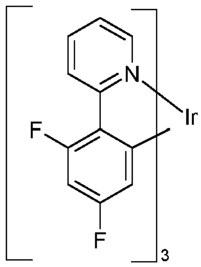
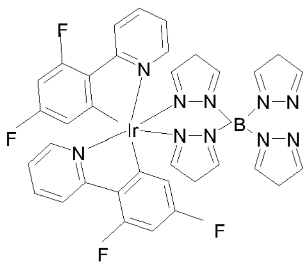

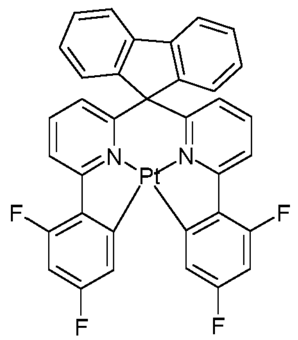 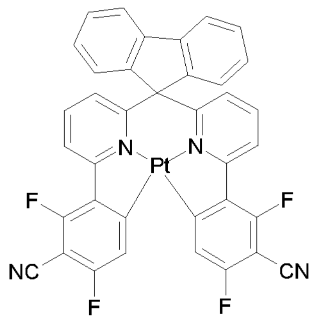 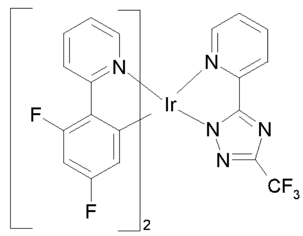

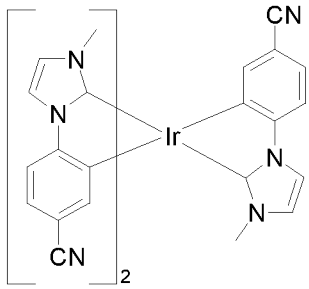 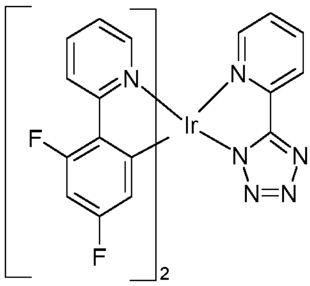 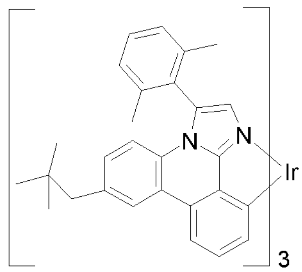

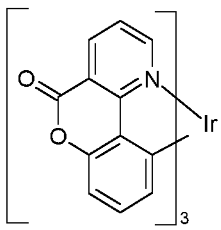 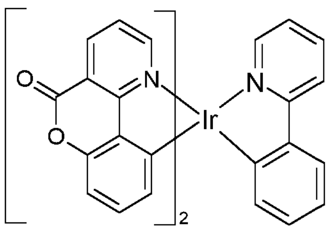 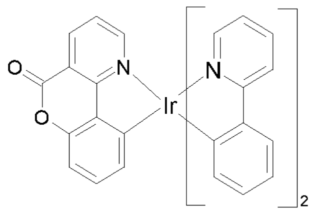

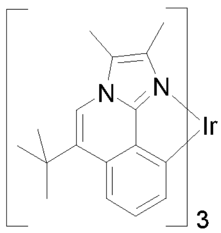 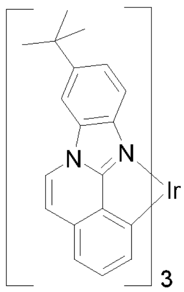 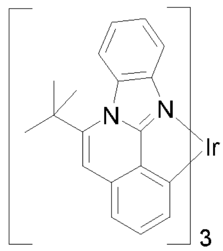

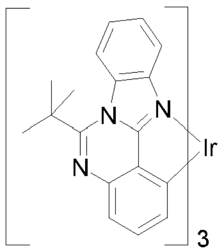 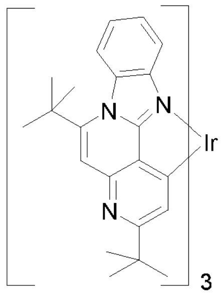 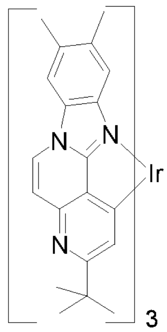

Die oben beschriebenen Verbindung der Formel (A) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung der Formel (A), enthält. Dabei sind bevorzugte elektronische Vorrichtungen ausgewählt aus der Gruppe bestehend aus organischen Elektrolumineszenzvorrichtungen

(OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung der Formel (A). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung der Formel (A) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung. Geeignete Matrixmaterialien, welche in Kombination mit den Verbindungen gemäß Formel (A) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011 /137951 oder WO 2013/064206, oder 4-Spirocarbazol-Derivate, z. B. gemäß WO 2014/094963 oder der noch nicht offen gelegten Anmeldung EP 14002104.9. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

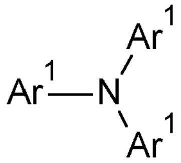

Formel (TA-1)

wobei $Ar^1$ gleich oder verschieden bei jedem Auftreten ein aromatisches oder heteroaromatisches Ringsystem mit 5 bis 60 aromatischen Ringatomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (A) genannte Bedeutung aufweist. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3-oder 4-Fluorenyl, 1-, 2-, 3-oder 4-Spirobifluorenyl, Pyridyl, Pyrimidinyl, 1-, 2-, 3-oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^1$ gleich oder verschieden bei jedem Auftreten ausgewählt aus den oben genannten Gruppen ($R^1$-1) bis ($R^1$-87), vorzugsweise ($R^1$-1) bis ($R^1$-54), insbesondere bevorzugt ($R^1$-1) bis ($R^1$-51), speziell bevorzugt ($R^1$-1) bis ($R^1$-37), wobei Reste gemäß ($R^1$-1) ganz besonders bevorzugt sind. Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

In einer bevorzugten Ausführungsform der Verbindungen der Formel (TA-1) ist mindestens eine Gruppe $Ar^1$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^1$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^1$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^1$ ausgewählt aus einer Fluoren- oder Spirobifluorengruppe, insbesondere einer 4-Fluoren- bzw. 4-Spirobifluorengruppe, und eine Gruppe $Ar^1$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^1$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

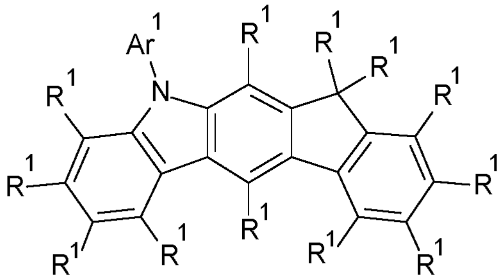

Formel (TA-2)

wobei $Ar^1$ und $R^1$ die oben insbesondere für Formeln (A) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Eine bevorzugte Ausführungsform der Verbindungen der Formel (TA-2) sind die Verbindungen der folgenden Formel (TA-2a),

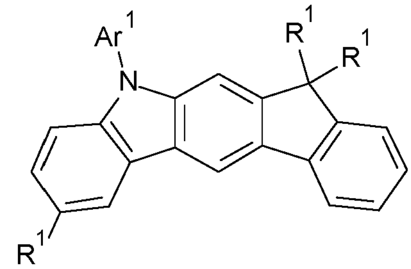

Formel (TA-2a)

wobei $Ar^1$ und $R^1$ die oben, insbesondere für Formeln (A) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

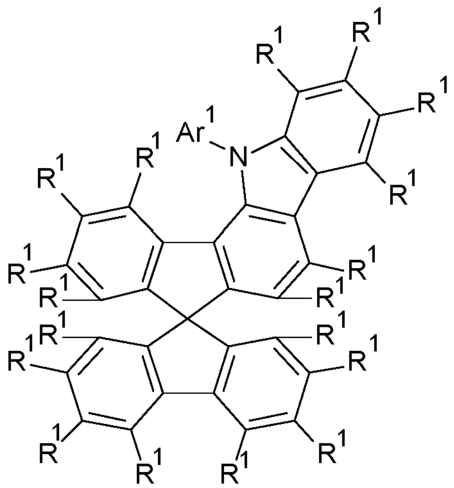

Formel (TA-3)

wobei $Ar^1$ und $R^1$ die oben, insbesondere für Formel (A) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Eine bevorzugte Ausführungsform der Verbindungen der Formel (TA-3) sind die Verbindungen der folgenden Formel (TA-3a),

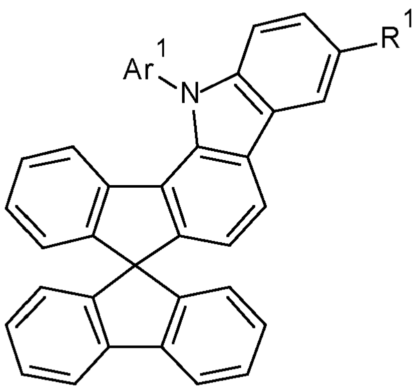

Formel (TA-3a)

wobei $Ar^1$ und $R^1$ die oben, insbesondere für Formel (A) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

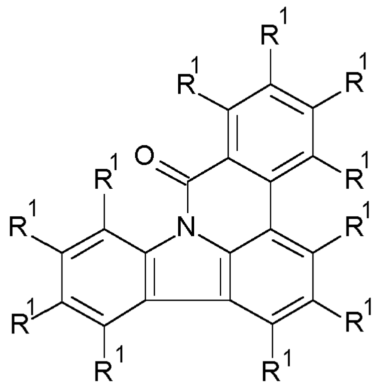

Formel (LAC-1)

wobei $R^1$ die oben, insbesondere für Formel (A) aufgeführte Bedeutung aufweist.

Eine bevorzugte Ausführungsform der Verbindungen der Formel (LAC-1) sind die Verbindungen der folgenden Formel (LAC-1 a),

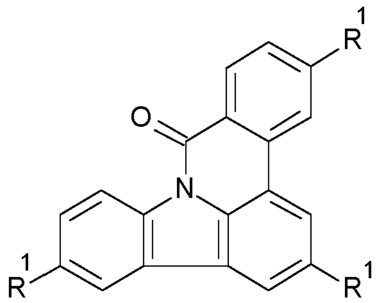

Formel (LAC-1a)

wobei $R^1$ die oben, insbesondere für Formel (A) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (A) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nichtaromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung der Formel (A) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung der Formel (A) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung der Formel (A) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung der Formel (A) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen der Formel (A) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen, Oligomere, Polymere oder Dendrimere mit Strukturen gemäß Formel (A) bzw. die zuvor und nachfolgend ausgeführten bevorzugten Ausführungsformen, insbesondere als elektronenleitende Materialien und/oder Matrixmaterialien, weisen eine sehr gute Lebensdauer auf.

2. Elektronischen Vorrichtungen, insbesondere organische Elektrolumineszenzvorrichtungen enthaltend Verbindungen, Oligomere, Polymere oder Dendrimere mit Strukturen gemäß Formel (A) bzw. die zuvor und nachfolgend ausgeführten bevorzugten Ausführungsformen als elektronenleitende Materialien, Elektroneninjektionsmaterialien und/oder Matrixmaterialien weisen eine hervorragende Effizienz auf. Insbesondere ist die Effizienz deutlich höher gegenüber analogen Verbindungen, die keine Struktureinheit gemäß Formel (A) enthalten.

3. Die erfindungsgemäßen Verbindungen, Oligomere, Polymere oder Dendrimere mit Strukturen gemäß Formel (A) bzw. die zuvor und nachfolgend ausgeführten bevorzugten Ausführungsformen zeigen eine sehr hohe Stabilität und führen zu Verbindungen mit einer sehr hohen Lebensdauer.

4. Mit Verbindungen, Oligomere, Polymere oder Dendrimere mit Strukturen gemäß Formel (A) bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomere, Polymere oder Dendrimere mit Strukturen gemäß Formel (A) bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektron-Leiterstrukturen.

6. Verbindungen, Oligomere, Polymere oder Dendrimere mit Strukturen gemäß Formel (A) bzw. die zuvor und nachfolgend ausgeführten bevozugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit Strukturen gemäß Formel (A) bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit Strukturen gemäß Formel (A) bzw. die zuvor und nachfolgend ausgeführten bevorzugten Ausführungsformen bilden aus Lösungen sehr gute Filme.

9. Die Verbindungen der Formel (A) und Oligomere, Polymere oder Dendrimere umfassend Strukturen gemäß Formel (A) bzw. die zuvor und nachfolgend ausgeführten bevorzugten Ausführungsformen weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Hostmaterial, Matrixmaterial, Elektronentransportmaterial, Elektroneninjektionsmaterial und/oder Lochblockiermaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Dies gilt insbesondere für erfindungsgemäße Verbindungen, die keine Carbazolstruktur aufweisen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (A) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern

dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

**Beispiele**

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

**Synthesebeispiele**

**a) 2,4-Diphenyl-benzo[4,5]furo[3,2-d]pyrimidin**

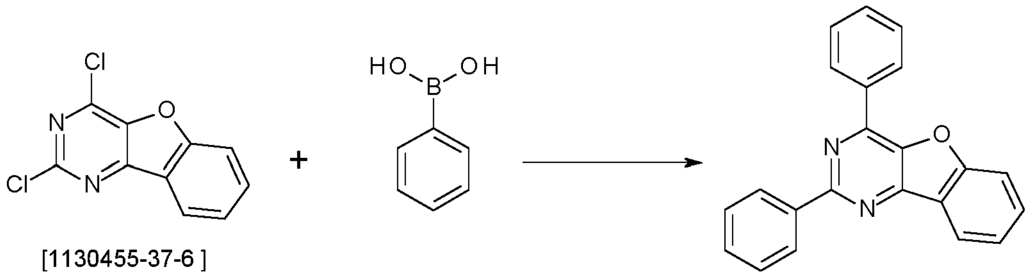

[1130455-37-6 ]

13 g (110.0 mmol) phenyl-boronsäure, 13 g (55 mmol) 2,4-Dichlorobenzo[4,5]furo[3,2-d]pyrimidin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert und anschließend zur Trockene eingeengt. Der Rückstand wird aus Toluol und aus Dichlormethan /Heotan umkristallisiert. Ausbeute: 15 g (47 mmol), 87 % der Theorie.

Analog dazu werden die folgenden Verbindungen hergestellt:

| | Edukt 1 | Edukt 2 | Produkt | Ausbeute |
|---|---|---|---|---|
| 2a | 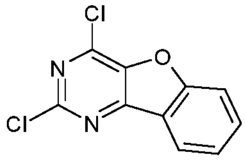 [1130455-37-6 ] | 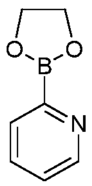 [317810-27-8] | 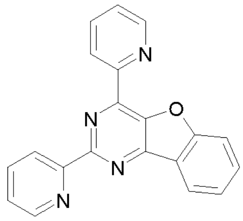 | 89% |
| 3a | 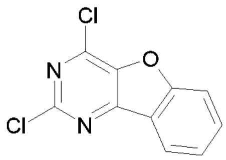 [1130455-37-6 ] | 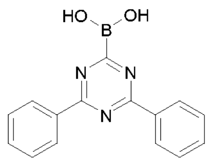 [1251825-65-6 ] | 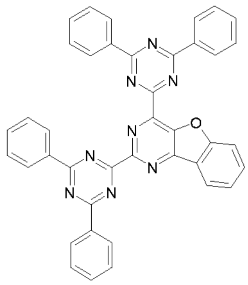 | 70% |
| 4a | 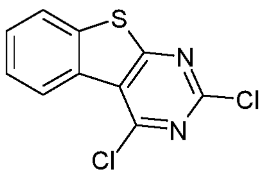 [76872-40-7] | 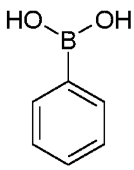 | 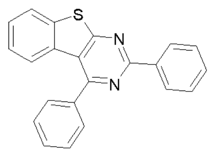 | 77% |
| 5a | 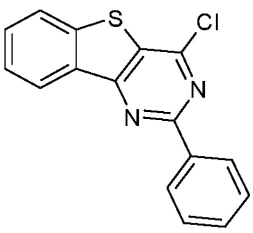 [900463-54-9 ] | 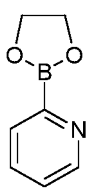 [317810-27-8] | 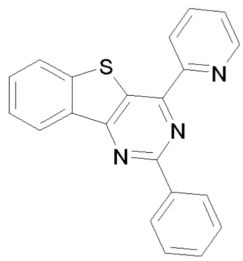 | 76% |
| 6a | 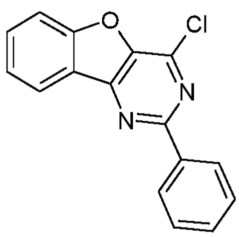 [134221-88-8 ] | 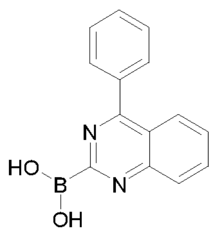 [1629973-75-6 ] | 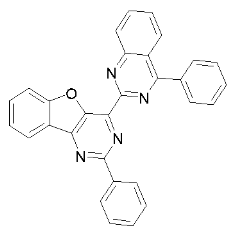 | 77% |

(fortgesetzt)

| | Edukt 1 | Edukt 2 | Produkt | Ausbeute |
|---|---|---|---|---|
| 7a | 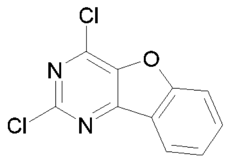 [1130455-37-6 ] | 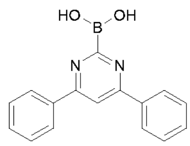 [1350818-50-6 ] | 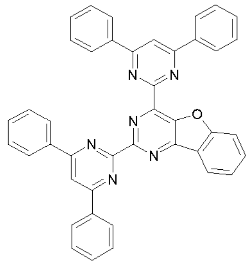 | 74% |
| 8a | 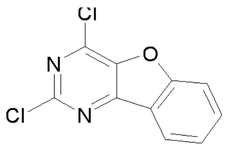 [1130455-37-6 ] | 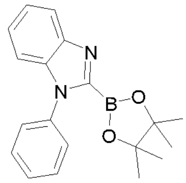 [1316275-47-4] | 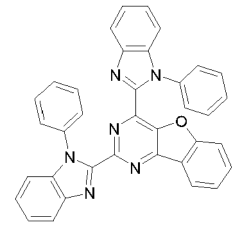 | 76% |
| 9a | 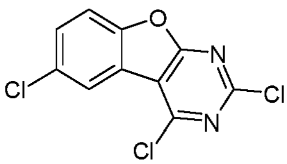 [1130455-37-6 ] | 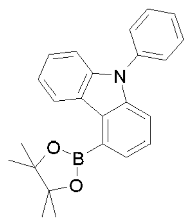 [1547492-13-6] | 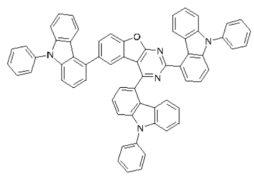 | 69% |
| 10a | 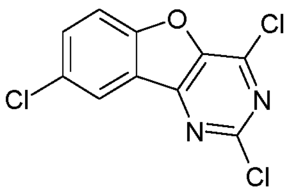 [160199-00-8] | 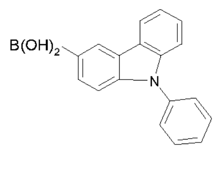 854952-58-2 | 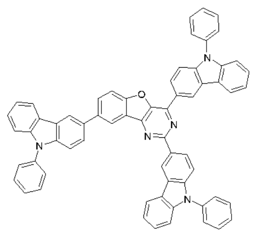 | 75% |
| 11a | 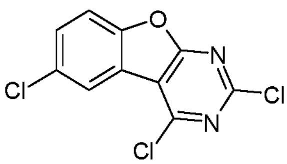 [1130455-37-6 ] | 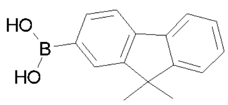 [333432-28-3] | 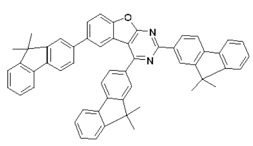 | 73% |
| 12a | 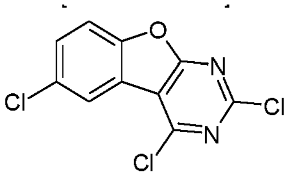 [1130455-37-6 ] | 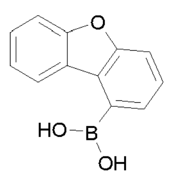 [162607-19-4 | 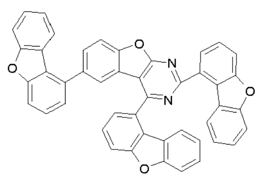 | 75% |

(fortgesetzt)

| | Edukt 1 | Edukt 2 | Produkt | Ausbeute |
|---|---|---|---|---|
| 13a | 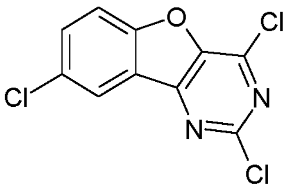 [160199-00-8] | 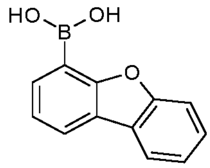 [100124-06-9 ] | 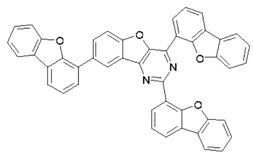 | 76% |
| 14a | 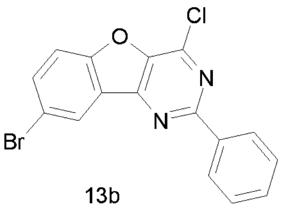 13b | 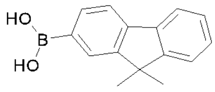 [333432-28-3] | 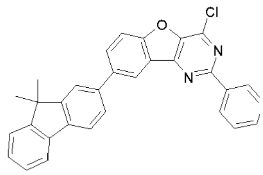 | 68% |
| 15a | 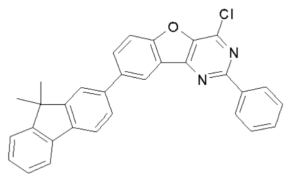 | 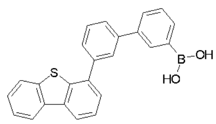 [1346010-12-5 ] | 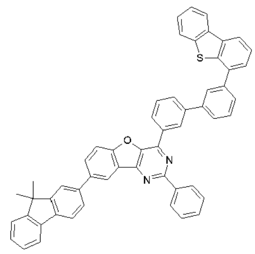 | 71% |
| 16a | 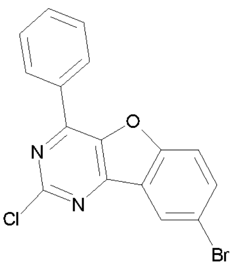 | 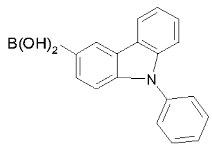 854952-58-2 | 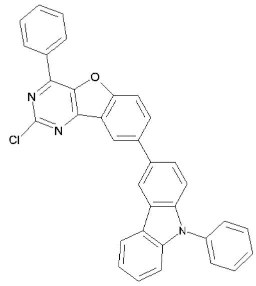 | 73% |

Das Produkt **9a** und **13a** wird via Säulenchromatographie an Kieselgel mit Toluol/Heptan (1:2) gereinigt und abschließend im Hochvakuum (p = 5 x $10^{-7}$ mbar) sublimiert (Reinheit 99,9%).

**b) 8-Bromo-2,4-diphenyl-benzo[4,5]furo[3,2-d]pyrimidin**

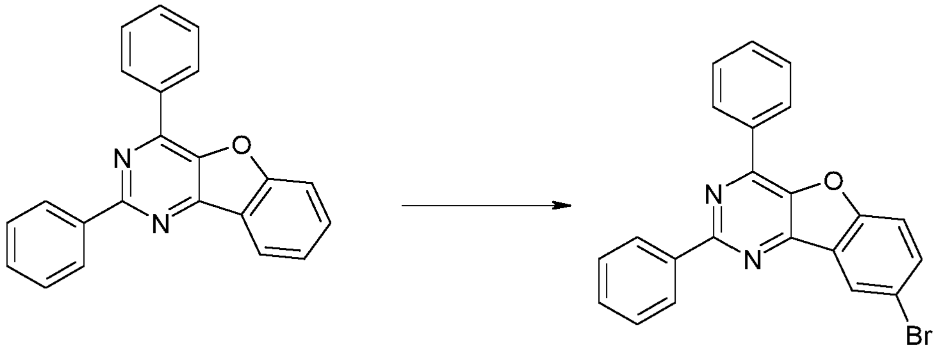

61 g (190.0 mmol) 2,4-Diphenyl-benzo[4,5]furo[3,2-d]pyrimidin weden 2000 mL Essigsäure(100%) und 2000 mL Schwefelsäure (95-98%) suspendiert. Zu dieser Suspension werden portionsweise 34 g (190 mmol) NBS zugegeben und 2 Stunden in Dunkelheit gerührt. Danach mit Wasser/Eis versetzt und Feststoff abgetrennt und mit Ethanol nachgewaschen. Der Rückstand wir im Toluol umkristallisiert. Die Ausbeute beträgt 65 g (163 mmol), entsprechend 86 % der Theorie.

Analog dazu werden die folgenden Verbindungen hergestellt:

| | Edukt 1 | Produkt | Ausbeute |
|---|---|---|---|
| 2b | 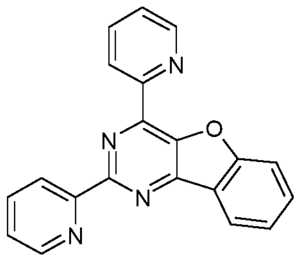 | 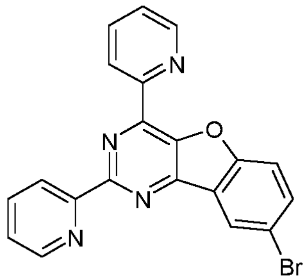 | 85% |
| 3b | 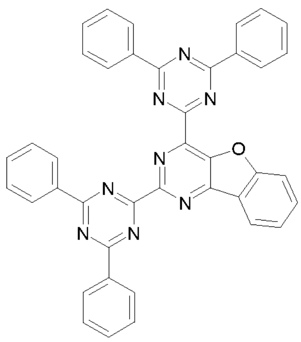 | 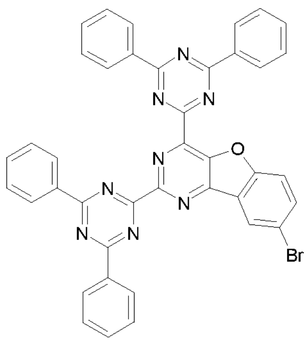 | 84% |
| 4b | 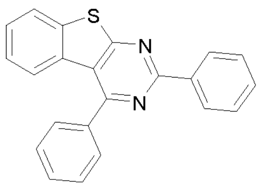 | 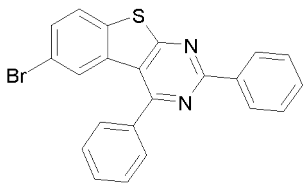 | 85% |

(fortgesetzt)

| | Edukt 1 | Produkt | Ausbeute |
|---|---|---|---|
| 5b | 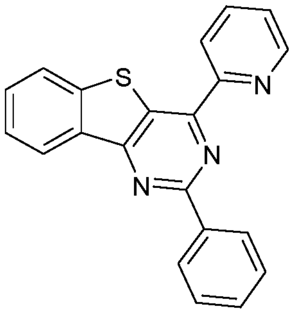 | 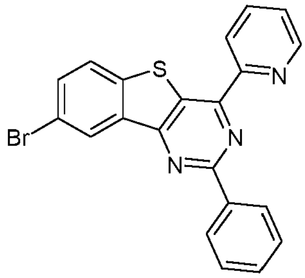 | 79% |
| 6b | 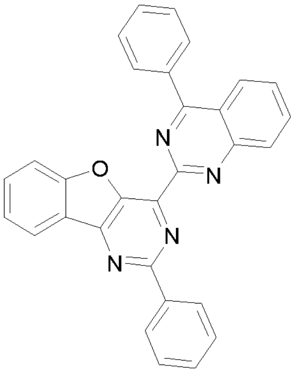 | 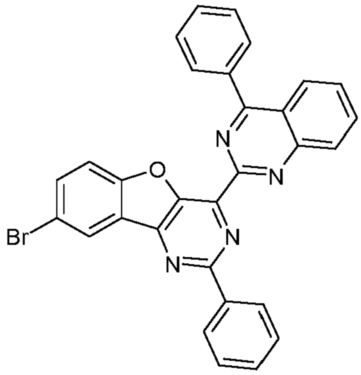 | 70% |
| 7b | 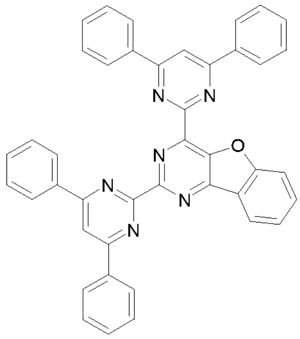 | 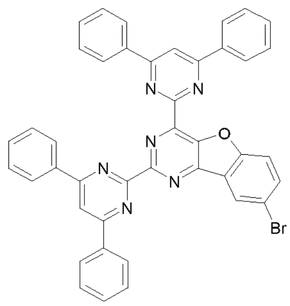 | 73% |
| 8b | 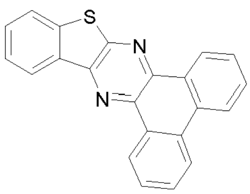 [101537-71-7 ] | 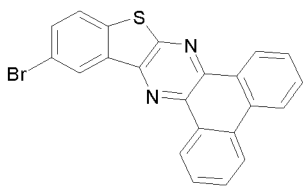 | 80% |
| 9b | 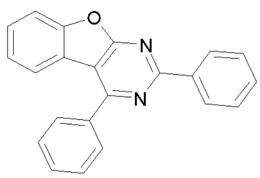 [129190-79-0 ] | 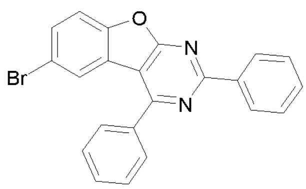 | 85% |

(fortgesetzt)

| | Edukt 1 | Produkt | Ausbeute |
|---|---|---|---|
| 10b | 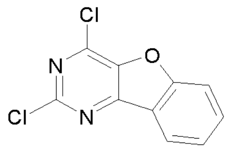 [1130455-37-6 ] | 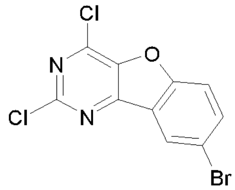 | 69% |
| 11b | 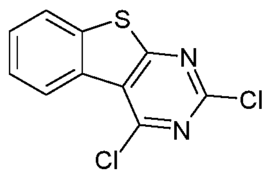 [76872-40-7] | 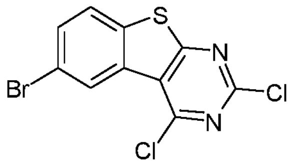 | 71% |
| 12b | 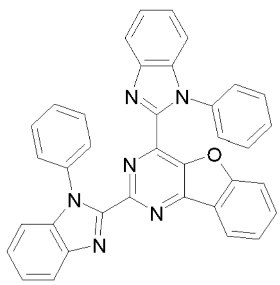 | 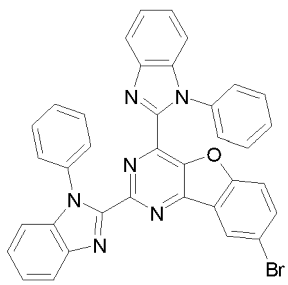 | 74% |
| 13b | 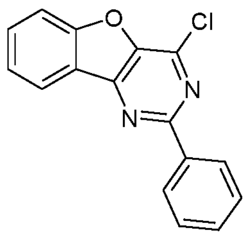 [134221-88-8 ] | 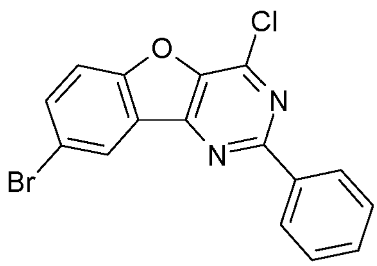 65% | 67% |
| 14b | 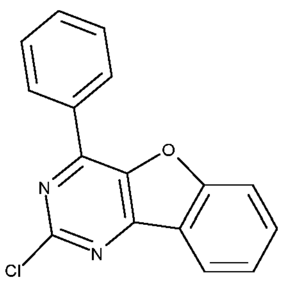 [1801233-17-9] | 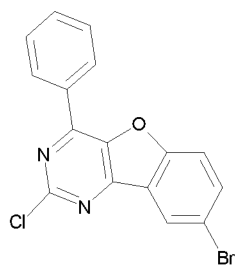 | 56% |

**c) 2,4-Diphenyl-8-(9-phenyl-9H-carbazol-3-yl)-benzo[4,5]furo[3,2-d]pyrimidin**

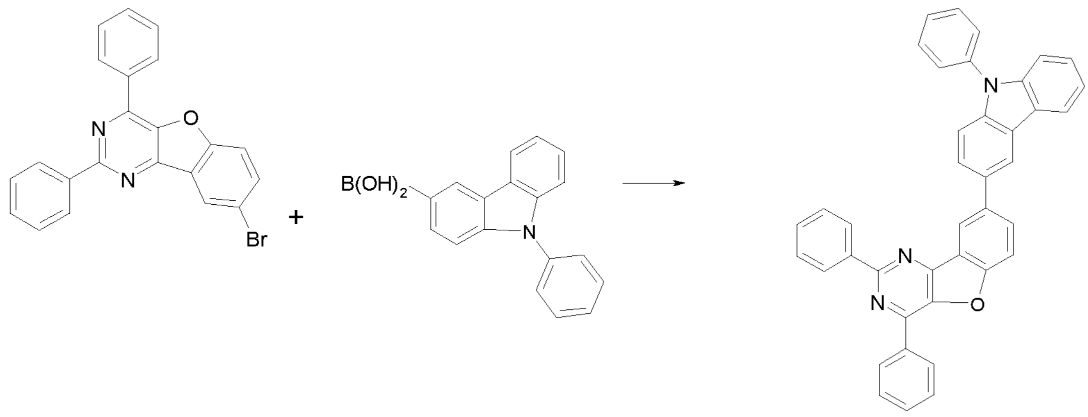

62 g (156 mmol) **8-Bromo-2,4-diphenyl-benzo[4,5]furo[3,2-d]pyrimidin,** 50 g (172 mmol) N-Phenyl-carbazol-3-boronsäure und 36 g (340 mmol) Natriumcarbonat werden in 1000 mL Ethylenglycoldiaminether und 280 mL Wasser suspendiert. Zu dieser Suspension werden 1,8 g (1,5 mmol) Tetrakies(triphenylphosphin)-palladium(0) zugegeben, und die Reaktionsmischung wird 16 h unter Rückfluss erhitzt. Nach Erkalten wird die organische Phase abgetrennt, über Kieselgel filtriert und anschließend zur Trockene eingeengt. Das Produkt wird via Säulenchromatographie an Kieselgel mit Toluol/Heptan (1:2) gereinigt und abschließend im Hochvakuum (p = 5 x $10^{-7}$ mbar) sublimiert (Reinheit 99,9%). Die Ausbeute beträgt 60 g (106 mmol), entsprechend 69 % der Theorie. Analog dazu werden die folgenden Verbindungen hergestellt:

| | Edukt 1 | Edukt 2 | Produkt | Ausbeute |
|---|---|---|---|---|
| 1c | 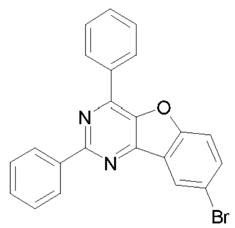 | 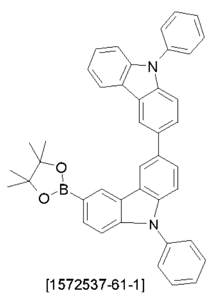 [1572537-61-1] | 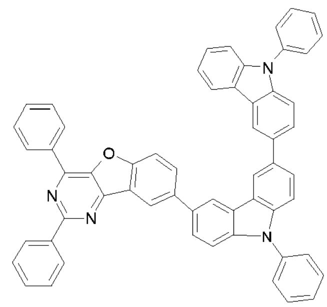 | 65% |
| 2c | 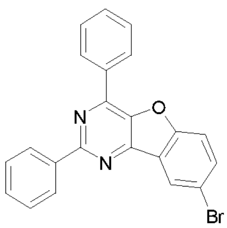 | 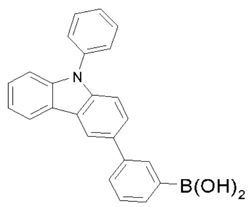 | 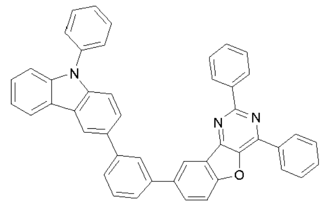 | 68% |
| 3c | 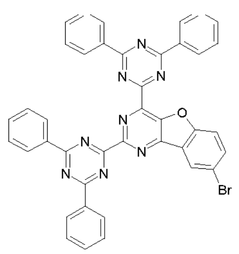 | 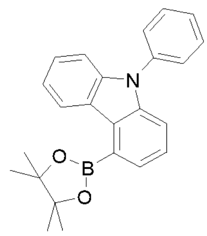 [1547492-13-6] | 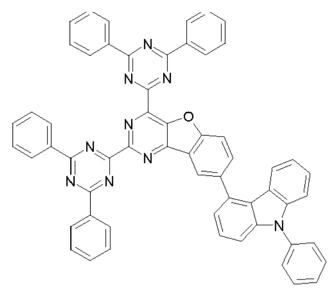 | 65% |

(fortgesetzt)

| | Edukt 1 | Edukt 2 | Produkt | Ausbeute |
|---|---|---|---|---|
| 4c | 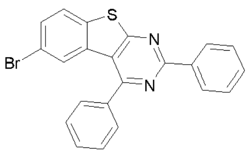 | 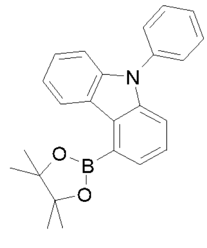 [1547492-13-6] | 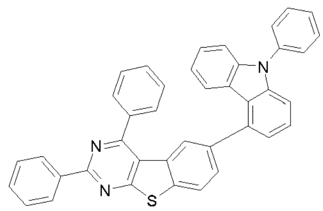 | 65% |
| 5c | 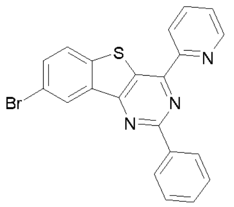 | 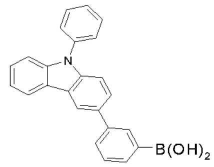 854952-60-6 | 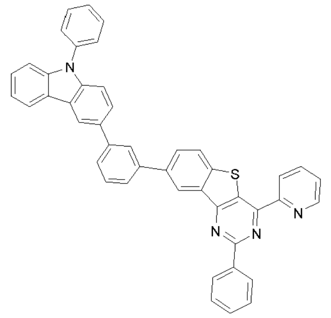 | 71% |
| 6c | 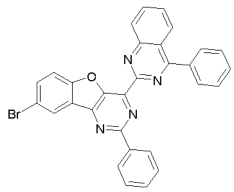 | 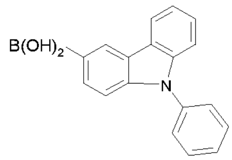 854952-58-2 | 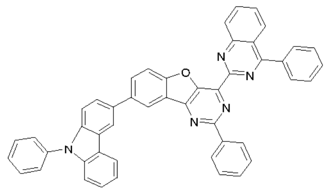 | 76% |
| 7c | 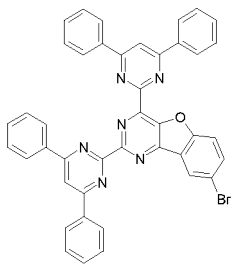 | 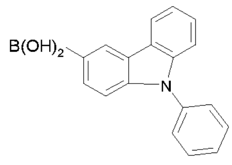 854952-58-2 | 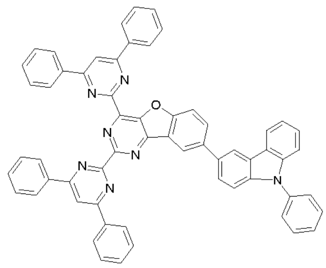 | 74% |
| 8c | 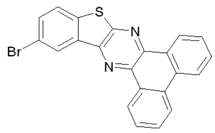 | 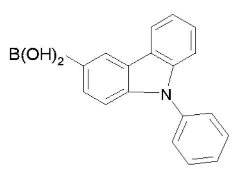 854952-58-2 | 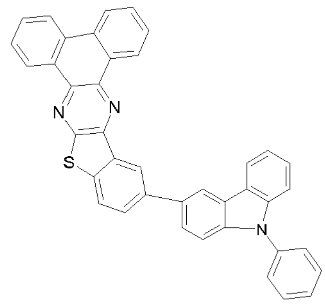 | 71% |

(fortgesetzt)

| | Edukt 1 | Edukt 2 | Produkt | Ausbeute |
|---|---|---|---|---|
| 9c | 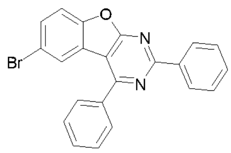 | 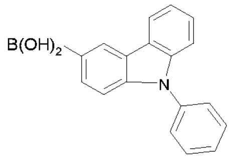 854952-58-2 | 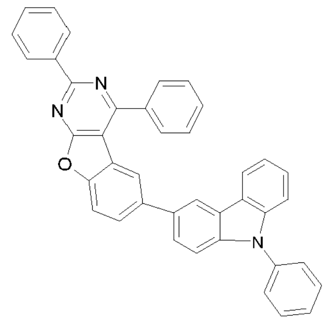 | 69% |
| 10c | 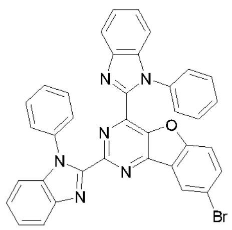 | 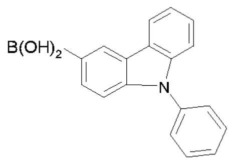 854952-58-2 | 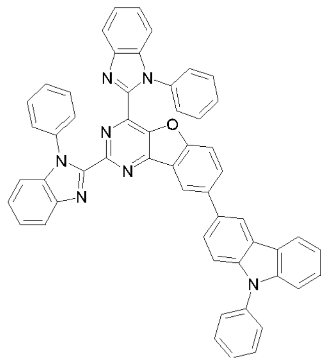 | 70% |
| 11c | 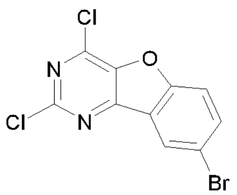 | 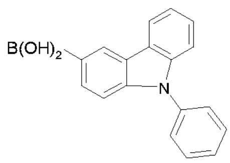 854952-58-2 | 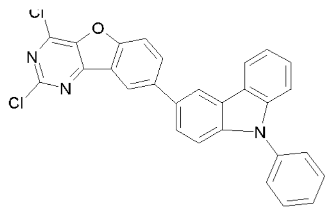 | 77% |
| 12c | 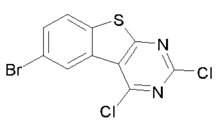 | 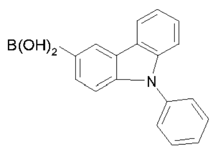 854952-58-2 | 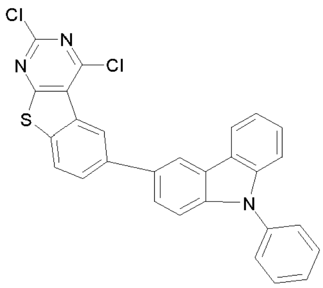 | 60% |
| 13c | 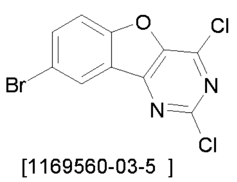 [1169560-03-5 ] | 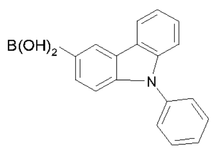 854952-58-2 | 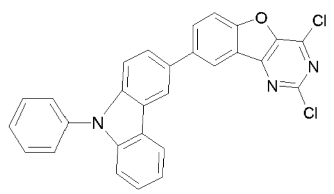 | 56% |

(fortgesetzt)

| | Edukt 1 | Edukt 2 | Produkt | Ausbeute |
|---|---|---|---|---|
| 14c | 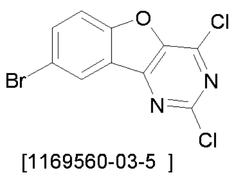 [1169560-03-5 ] | 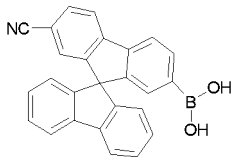 [1838683-74-1 ] | 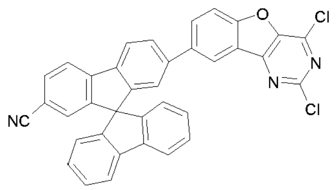 | 64% |
| 15c | 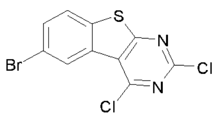 | 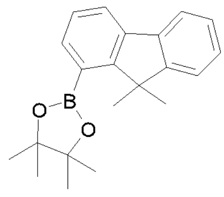 [1792218-53-1] | 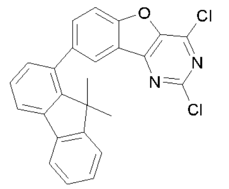 | 65% |
| 16c | 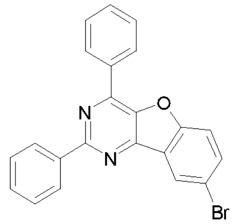 | 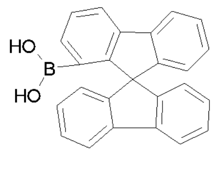 [1561044-65-2 ] | 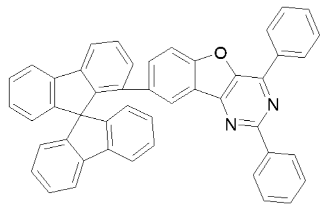 | 67% |
| 17c | 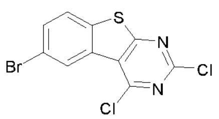 | 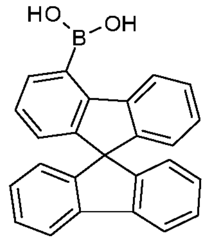 [1421789-05-0] | 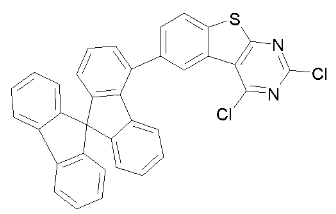 | 68% |
| 18c | 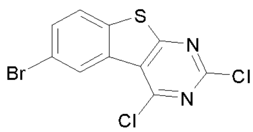 | 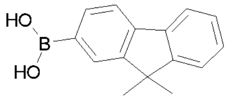 [333432-28-3] | 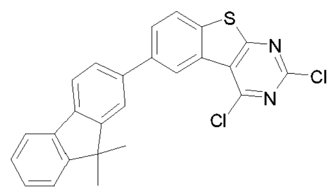 | 66% |
| 19c | 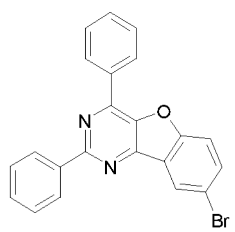 | 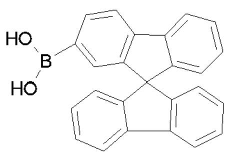 [ 236389-21-2 ] | 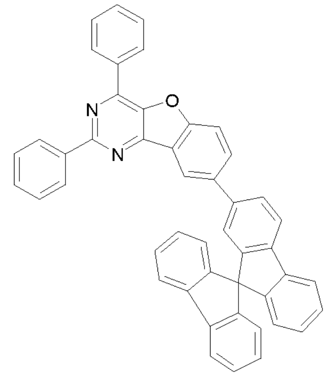 | 77% |

(fortgesetzt)

| | Edukt 1 | Edukt 2 | Produkt | Ausbeute |
|---|---|---|---|---|
| 20c | 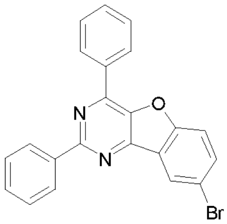 | 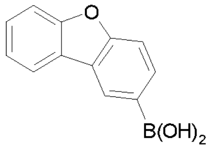 [402936-15-6] | 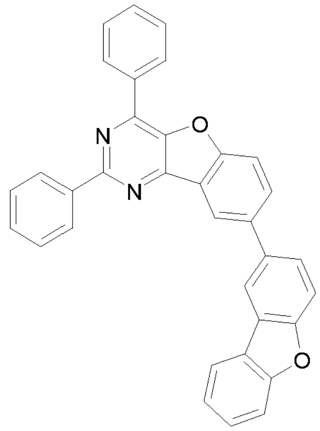 | 75% |
| 21c | 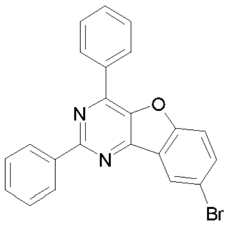 | 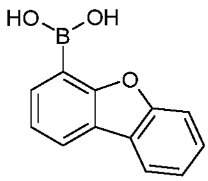 [100124-06-9 ] | 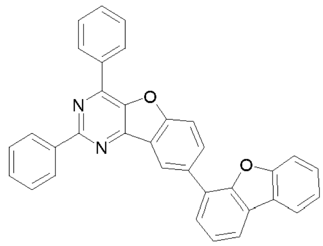 | 80% |
| 22c | 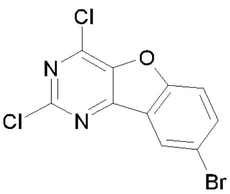 | 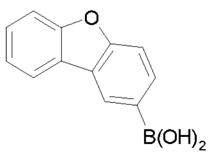 [402936-15-6] | 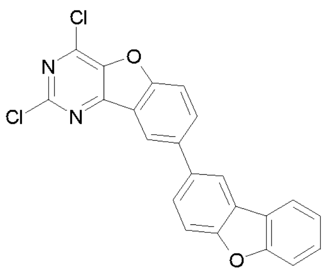 | 64% |
| 23c | 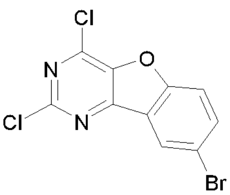 | 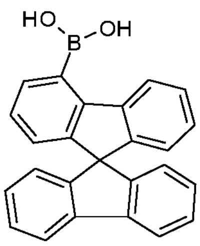 [1421789-05-0] | 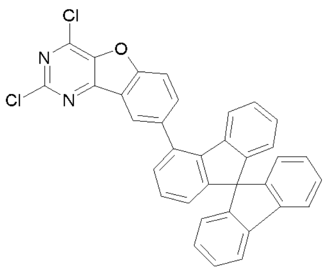 | 61% |

(fortgesetzt)

| | Edukt 1 | Edukt 2 | Produkt | Ausbeute |
|---|---|---|---|---|
| 24c | 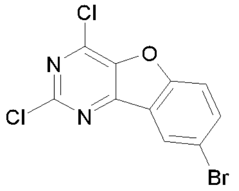 | 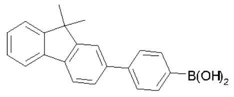 [736928-21-5 ] | 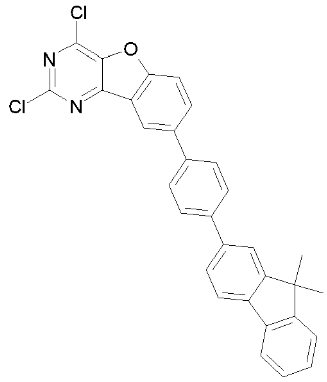 | 62% |

Analog dazu werden aus **11c- 17c** sowie **12c-18c** und **22c-24c** die folgenden Verbindungen hergestellt:

| | Edukt 1 | Edukt 2 | Produkt | Aus beu te |
|---|---|---|---|---|
| 25c | 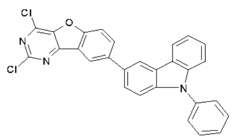 | 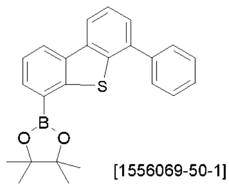 [1556069-50-1] | 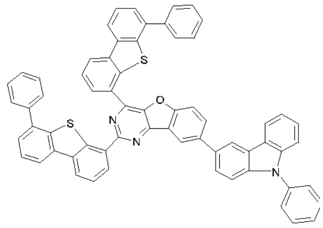 | 72% |
| 26c | 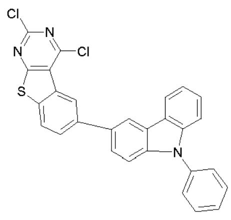 | 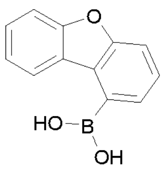 [162607-19-4 | 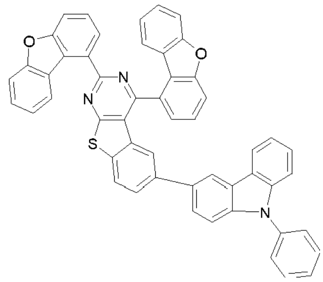 | 74% |
| 27c | 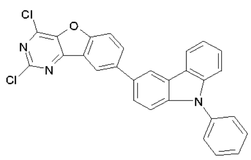 | 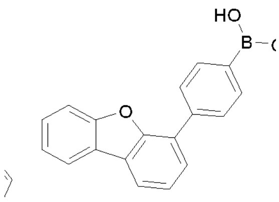 [796071-96-0 ] | 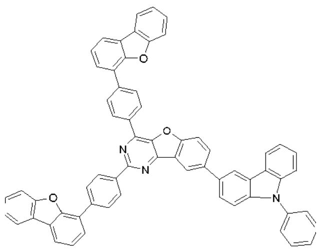 | 68% |
| 28c | 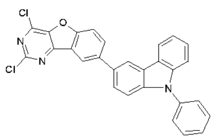 | 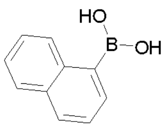 [13922-41-3 ] | 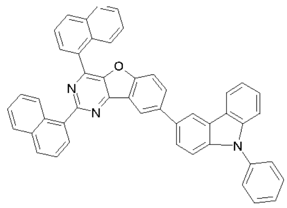 | 66% |

(fortgesetzt)

| | Edukt 1 | Edukt 2 | Produkt | Aus beu te |
|---|---|---|---|---|
| 29c | 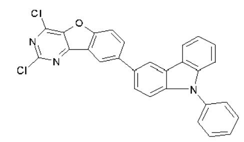 | 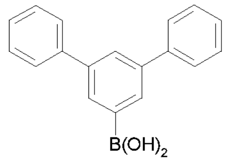 [128388-54-5 ] | 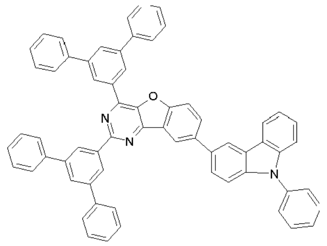 | 64% |
| 30c | 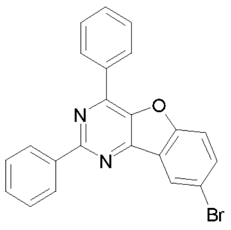 | 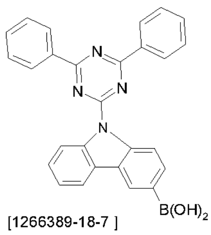 [1266389-18-7 ] | 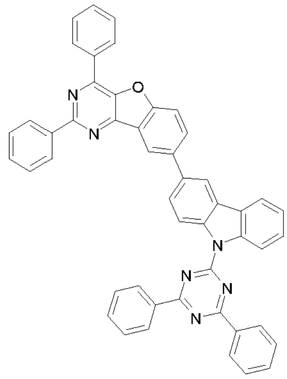 | 72% |
| 31c | 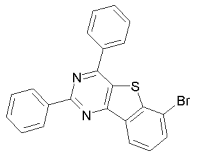 [1801233-18-0] | 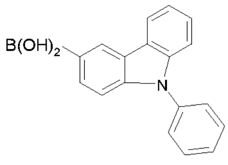 854952-58-2 | 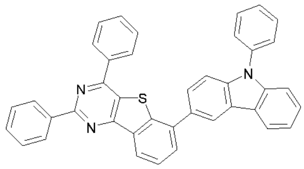 | 73% |
| 32c | 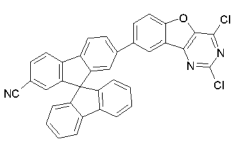 | 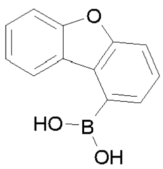 [162607-19-4 | 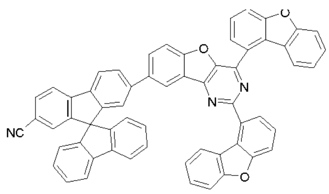 | 71% |
| 33c | 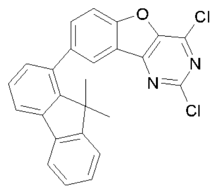 | 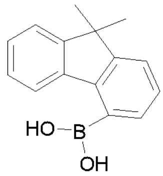 [1246022-50-3] | 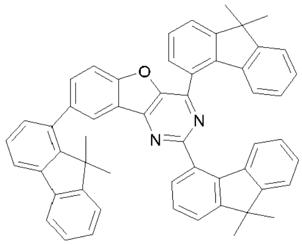 | 75% |
| 34c | 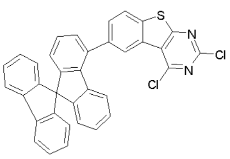 | 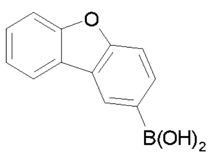 [402936-15-6] | 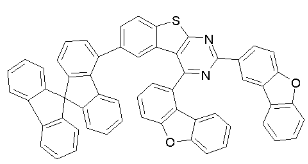 | 75% |

(fortgesetzt)

| | Edukt 1 | Edukt 2 | Produkt | Aus beu te |
|---|---|---|---|---|
| 35c | 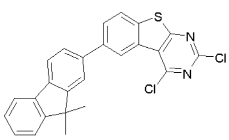 | 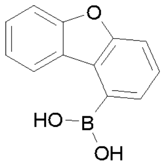 [162607-19-4 | 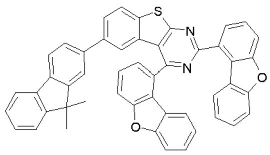 | 76% |
| 36c | 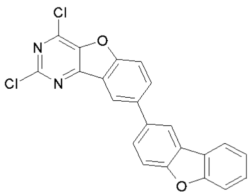 | 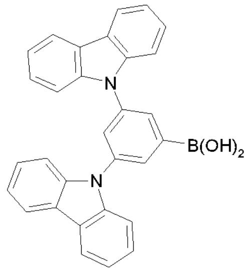 [854952-51-5 ] | 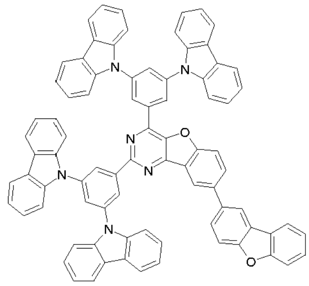 | 63% |
| 37c | 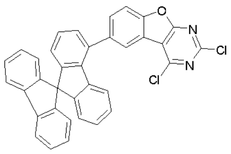 | 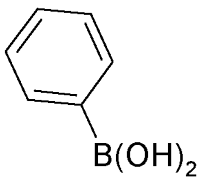 | 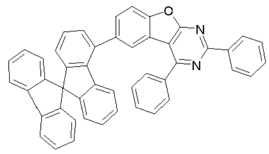 | 75% |
| 38c | 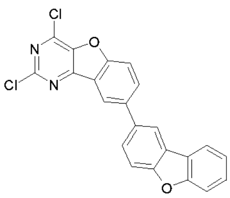 | 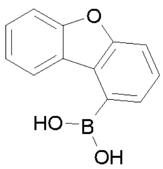 [162607-19-4 | 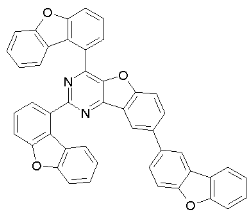 | 73% |
| 39c | 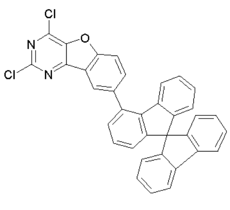 | 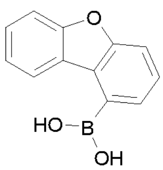 [162607-19-4 | 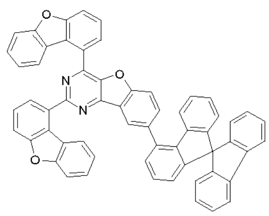 | 76% |
| 40c | 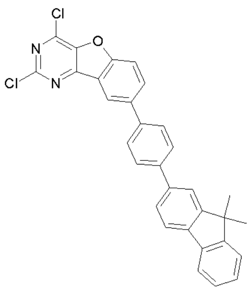 | 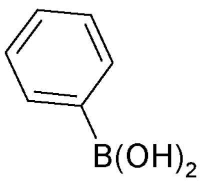 | 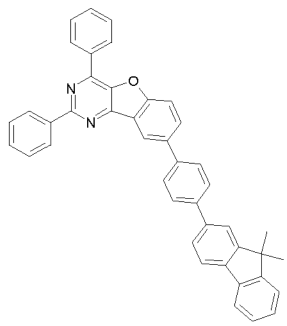 | 78% |

**d) 2,4-Bis-carbazol-9-yl-8-[2-eth-(Z)-ylidene-3,3-dimethyl-1-prop-2-en-(Z)-ylidene-indan-4-yl]-benzo[4,5]furo[3,2-d]pyrimidin**

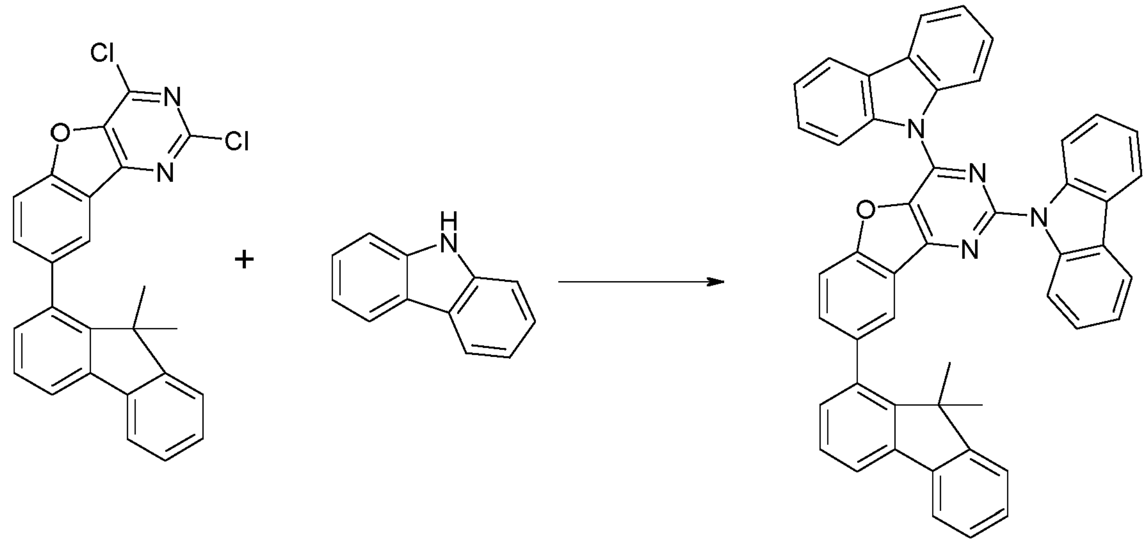

Eine entgaste Lösung von 53 g (147 mmol) 2,4-Dichloro-8-(9,9-dimethyl-9H-fluoren-1-yl)-benzo[4,5]furo[3,2-d]pyrimidin und 24 g(147 mmol) 9H-carbazol in 600 mL Toluol wird 1 h mit $N_2$ gesättigt. Danach wird die Lösung zuerst mit 2.09 mL (8.6 mmol) P(*t*Bu)$_3$, dann mit 1.38 g (6.1 mmol) Palladium(II)acetat versetzt und anschließend werden 17.7 g (185 mmol) NaOtBu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird mit 3 x 50 mL Toluol gewaschen, über $MgSO_4$ getrocknet und das Lösungsmittel im Vakuum entfernt. Danach wird das Rohprodukt über Kieselgel mit Heptan/Essigsäureester (20/1) chromatographisch gereinigt. Der Rückstand wird aus Toluol umkristallisiert und abschließend im Hochvakuum (p = 5 x $10^{-6}$ mbar) sublimiert.

Die Ausbeute beträgt 67 g (95 mmol), entsprechend 78% der Theorie.

Analog können folgende Verbindungen erhalten werden:

| | Edukt 1 | Edukt 2 | Produkt | Ausbeute |
|---|---|---|---|---|
| 1d | 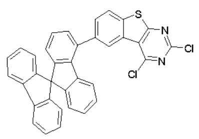 | 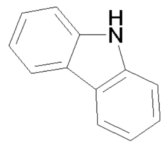 | 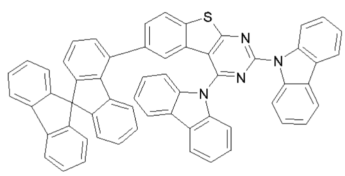 | 75% |
| 2d | 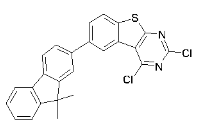 | 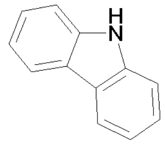 | 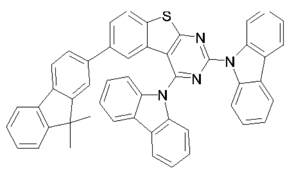 | 70% |
| 3d | 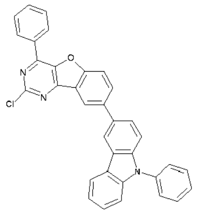 | 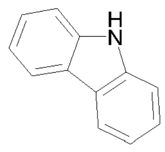 | 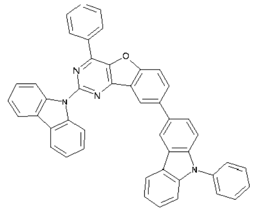 | 73% |

**e) 4-Phenyl-2,8-bis-(9-phenyl-9H-carbazol-3-yl)-benzo[4,5]thieno[3,2-d]pyrimidin (1e):**

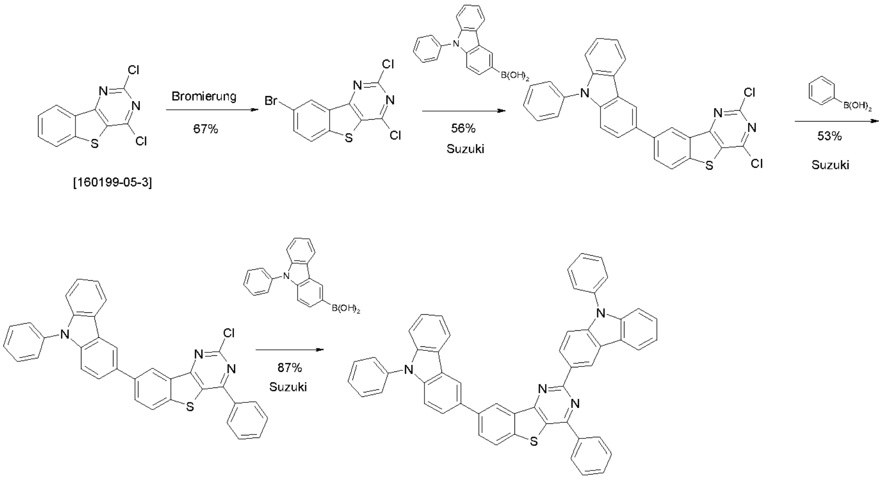


2,4-Dichloro-benzo[4,5]thieno[3,2-d]pyrimidin wird analog zu Vorschrift b bromiert, anschließend mit Phenylcarbazolboronsäure via Suzuki umgesetzt analog zu Vorschrift c umgesetzt und dann wiederum analog zu Vorschrift c erst mit Phenylboronsäure und zum Schluss mit Phenylcarbazolboronsäure umgesetzt.

f) **4-(6-Dibenzofuran-4-yl-pyridin-2-yl)-2,8-di-pyridin-2-ylbenzo[4,5]thieno[3,2-d]pyrimidin (1f)**

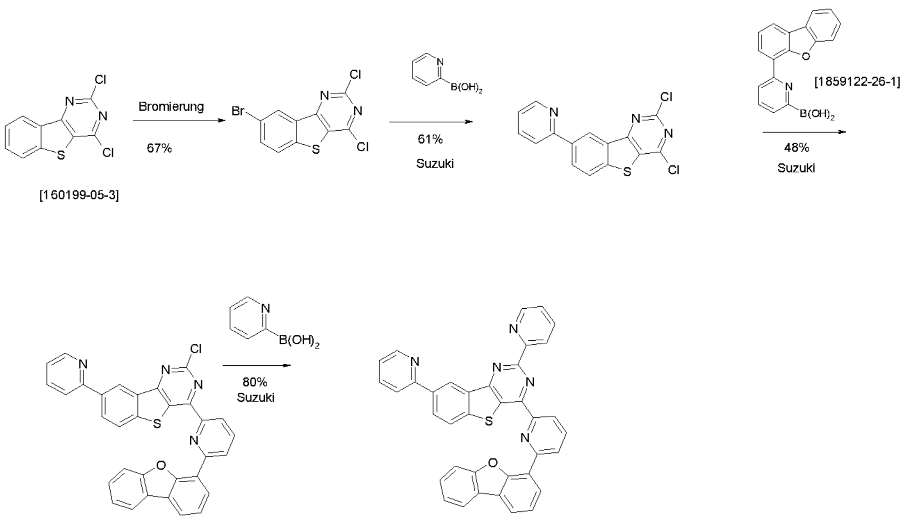


Die Herstellung erfolgt gemäß der unter e) zuvor dargelegten Vorschrift.

**g) 2,2'-Bis-carbazol-9-yl-4,4'-diphenyl-[8,8']bi[benzo[4,5]thieno[3,2-d]pyrimidinyl]**

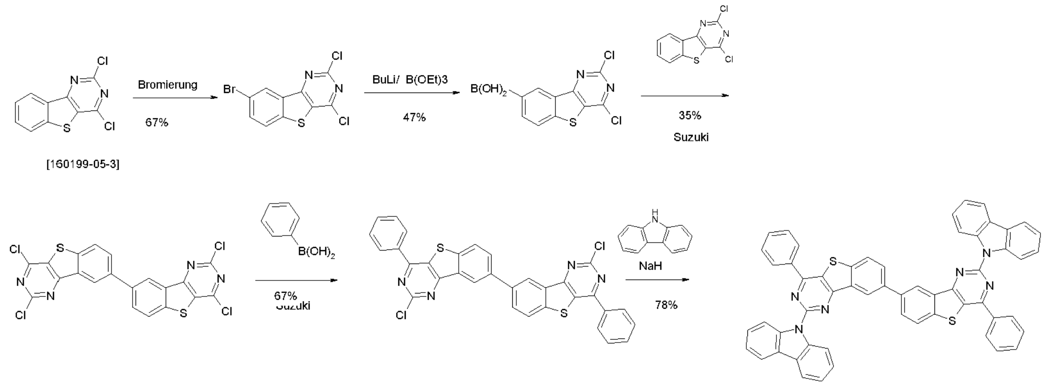


2,4-Dichloro-benzo[4,5]thieno[3,2-d]pyrimidin wird analog zu Vorschrift b bromiert, anschließend mit BuLi und Triethylborat zu entsprechende Böronsöure umgewandelt. Dann erfolgt die Kupplung analog zu Vorschrift **c** zu entsprechende Dimer und dann wiederum analog zu Vorschrift c erst mit Phenylboronsäure und zum Schluss durch Umsetzung mit NaH und Carbazol über nucleophile Substitution zum Zielmolekül umgesetzt.

**j) 2-Carbazol-9-yl-8-dibenzothiophen-2-yl-4-phenylbenzo[4,5]thieno[3,2-d]pyrimidin**

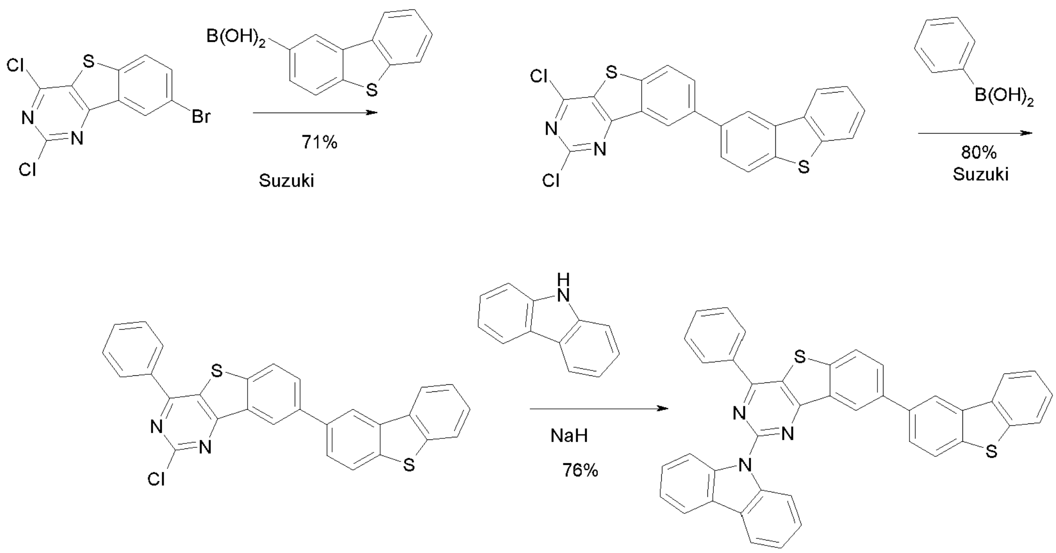


Die Herstellung erfolgt gemäß der unter g) zuvor dargelegten Vorschrift.

**Herstellung der OLEDs**

In den folgenden Beispielen V1 bis E13 (siehe Tabellen 1 und 2) werden die Daten verschiedener OLEDs vorgestellt.

**Vorbehandlung für die Beispiele V1-E13:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand,

Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. EQE1000 schließlich bezeichnet die externe Quanteneffizienz bei einer Betriebsleuchtdichte von 1000 cd/$m^2$. Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/$m^2$ und L1 = 70% in Tabelle 2 bedeutet, dass die in Spalte LD angegebene Lebensdauer der Zeit entspricht, nach der die Anfangsleuchtdichte von 4000 cd/$m^2$ auf 2800 cd/$m^2$ absinkt. Analog bedeutet L0;j0 = 20mA/$cm^2$, L1 = 80%, dass die Leuchtdichte bei Betrieb mit 20mA/$cm^2$ nach der Zeit LD auf 80% ihres Anfangswertes absinkt.

Die Daten der verschiedenen OLEDs sind in Tabelle 2 zusammengefasst. Die Beispiele V1- V4 sind Vergleichsbeispiele gemäß dem Stand der Technik, die Beispiele E1-E13 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Materialien in phosphoreszenten OLEDs**

Die erfindungsgemäßen Materialien ergeben bei Einsatz in der Emissionsschicht (EML) in OLEDs wesentliche Verbesserungen gegenüber dem Stand der Technik, vor allem bezüglich Lebensdauer. Durch Einsatz der erfindungsgemäßen Verbindungen 3d, 1e, 1f und 15a lässt sich eine Steigung der Lebensdauer um ca. 20-30% gegenüber dem Stand der Technik erzielen (Vergleich von Beispiel E1 mit V1; Vergleich von Beispiel E2 mit V2; Vergleich von Beispiel E3 mit V3; Vergleich von Beispiel E4mit V4).

Tabelle 1: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 125nm | SpMA3 10nm | SdT1:TER5 (95%:5%) 40nm | -- | ST2:LiQ (50%:50%) 35nm | -- |
| V2 | HATCN 5nm | SpMA1 125nm | SpMA3 10nm | SdT2:TER5 (95%:5%) 40nm | -- | ST2:LiQ (50%:50%) 35nm | -- |
| V3 | HATCN 5nm | SpMA1 125nm | SpMA3 10nm | SdT3:TER5 (95%:5%) 40nm | -- | ST2:LiQ (50%:50%) 35nm | -- |
| V4 | HATCN 5nm | SpMA1 125nm | SpMA3 10nm | SdT4:TER5 (95%:5%) 40nm | -- | ST2:LiQ (50%:50%) 35nm | -- |
| E1 | HATCN 5nm | SpMA1 125nm | SpMA3 10nm | 3d:TER5 (95%:5%) 40nm | -- | ST2:LiQ (50%:50%) 35nm | -- |
| E2 | HATCN 5nm | SpMA1 125nm | SpMA3 10nm | 1e:TER5 (95%:5%) 40nm | -- | ST2:LiQ (50%:50%) 35nm | -- |
| E3 | HATCN 5nm | SpMA1 125nm | SpMA3 10nm | 1f:TER5 (95%:5%) 40nm | -- | ST2:LiQ (50%:50%) 35nm | -- |
| E4 | HATCN 5nm | SpMA1 125nm | SpMA3 10nm | 15a:TER5 (95%:5%) 40nm | -- | ST2:LiQ (50%:50%) 35nm | -- |
| E5 | HATCN 5nm | SpMA1 125nm | SpMA3 10nm | 11 a:TER5 (95%:5%) 40nm | -- | ST2:LiQ (50%:50%) 35nm | -- |
| E6 | HATCN | SpMA1 | SpMA3 | 12a:TER5 | -- | ST2:LiQ | -- |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| | 5nm | 125nm | 10nm | (95%:5%) 40nm | | (50%:50%) 35nm | |
| E7 | HATCN 5nm | SpMA1 125nm | SpMA3 10nm | 9c:TER5 (95%:5%) 40nm | -- | ST2:LiQ (50%:50%) 35nm | -- |
| E8 | HATCN 5nm | SpMA1 125nm | SpMA3 10nm | 1c:TER5 (95%:5%) 40nm | -- | ST2:LiQ (50%:50%) 35nm | -- |
| E9 | HATCN 5nm | SpMA1 125nm | SpMA3 10nm | 39c:TER5 (95%:5%) 40nm | -- | ST2:LiQ (50%:50%) 35nm | -- |
| E10 | HATCN 5nm | SpMA1 125nm | SpMA3 10nm | 19c:TER5 (95%:5%) 40nm | -- | ST2:LiQ (50%:50%) 35nm | -- |
| E11 | HATCN 5nm | SpMA1 125nm | SpMA3 10nm | 27c:TER5 (95%:5%) 40nm | -- | ST2:LiQ (50%:50%) 35nm | -- |
| E12 | HATCN 5nm | SpMA1 125nm | SpMA3 10nm | 30c:TER5 (95%:5%) 40nm | -- | ST2:LiQ (50%:50%) 35nm | -- |
| E13 | HATCN 5nm | SpMA1 235nm | SpMA3 20nm | 9c:IC3:TEG2 (45%:45%:10%) 30nm | -- | ST2:LiQ (50%:50%) 40nm | -- |

Tabelle 2: Daten der OLEDs

| Bsp. | U1000 (V) | EQE 1000 | CIE x/y bei 1000 cd/$m^2$ | $L_0$; $j_0$ | L1 % | LD (h) |
|---|---|---|---|---|---|---|
| V1 | 3.5 | 22.2 % | 0.67/0.33 | 50 mA/$cm^2$ | 95 | 45 |
| V2 | 3.6 | 21.6% | 0.67/0.33 | 50 mA/$cm^2$ | 95 | 40 |
| V3 | 3.7 | 22.2% | 0.67/0.33 | 50 mA/$cm^2$ | 95 | 5 |
| V4 | 3.7 | 22.4% | 0.67/0.33 | 50 mA/$cm^2$ | 95 | 45 |
| E1 | 3.6 | 21.8% | 0.67/0.33 | 50 mA/$cm^2$ | 95 | 60 |
| E2 | 3.7 | 22.4% | 0.67/0.33 | 50 mA/$cm^2$ | 95 | 50 |
| E3 | 3.6 | 22.1% | 0.67/0.33 | 50 mA/$cm^2$ | 95 | 10 |
| E4 | 3.7 | 22.3% | 0.67/0.33 | 50 mA/$cm^2$ | 95 | 55 |
| E5 | 3.7 | 22.2% | 0.67/0.33 | 50 mA/$cm^2$ | 95 | 50 |
| E6 | 3.8 | 22.4% | 0.67/0.33 | 50 mA/$cm^2$ | 95 | 45 |
| E7 | 3.7 | 22.5% | 0.67/0.33 | 50 mA/$cm^2$ | 95 | 50 |
| E8 | 3.6 | 22.2% | 0.67/0.33 | 50 mA/$cm^2$ | 95 | 60 |
| E9 | 3.8 | 22.0% | 0.67/0.33 | 50 mA/$cm^2$ | 95 | 50 |
| E10 | 3.8 | 22.2% | 0.67/0.33 | 50 mA/$cm^2$ | 95 | 55 |
| E11 | 3.8 | 22.4% | 0.67/0.33 | 50 mA/$cm^2$ | 95 | 60 |
| E12 | 3.4 | 21.8% | 0.67/0.33 | 50 mA/$cm^2$ | 95 | 45 |
| E13 | 3.4 | 20.2% | 0.33/0.63 | 40mA/$cm^2$ | 80 | 120 |

Tabelle 3: Strukturformeln der Materialien für die OLEDs

| | |
|---|---|
| 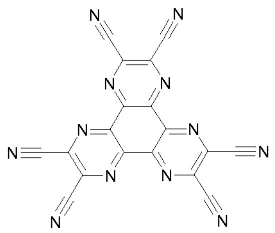 | 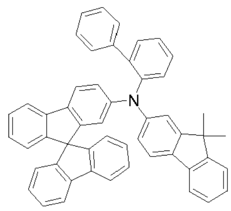 |
| HATCN | SpMA1 |
| 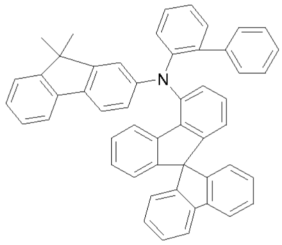 | 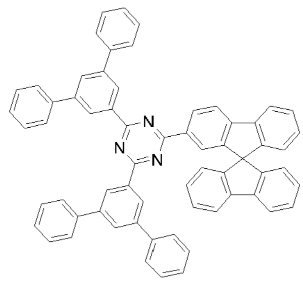 |
| SpMA3 | ST2 |
| 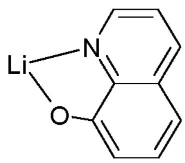 | 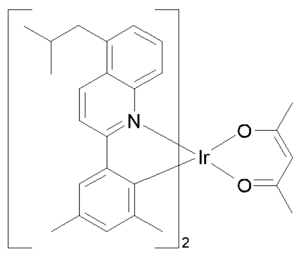 |
| LiQ | TER5 |
| 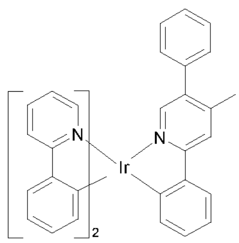 | 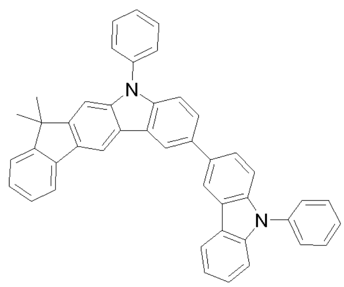 |
| TEG2 | IC3 |
| 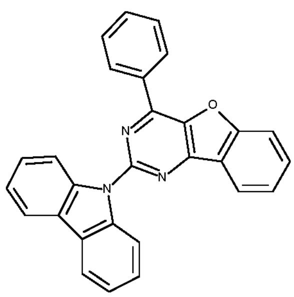 | 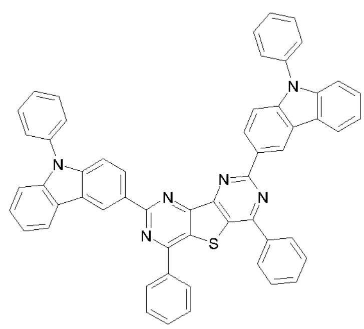 |
| SdT1 | SdT2 |

(fortgesetzt)

| | |
|---|---|
| 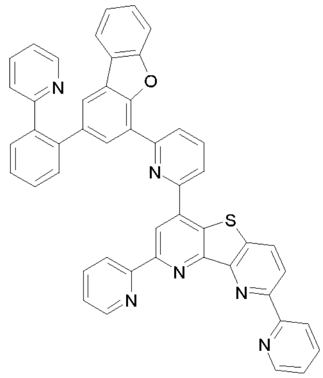 | 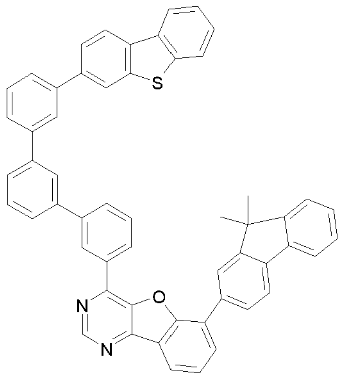 |
| SdT3 | SdT4 |
| 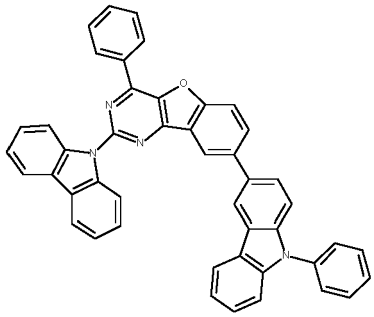 | 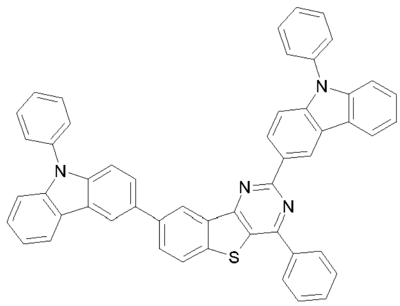 |
| 3d | 1e |
| 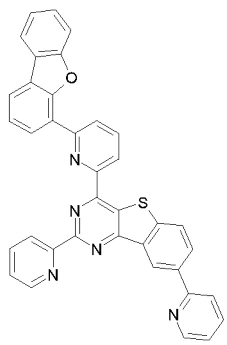 | 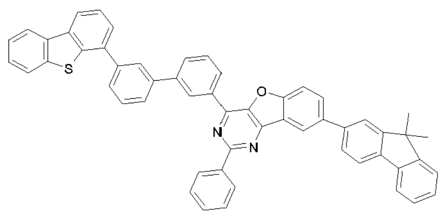 |
| 1f | 15a |
| 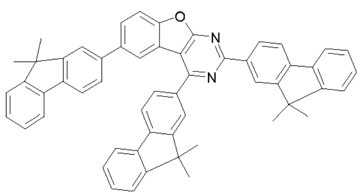 | 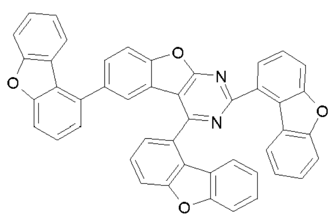 |
| 11a | 12a |

(fortgesetzt)

| | |
|---|---|
| 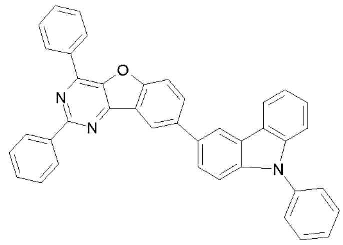 | 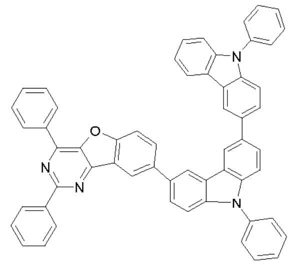 |
| 9c | 1c |
| 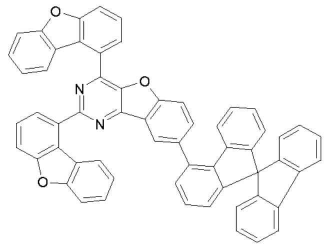 | 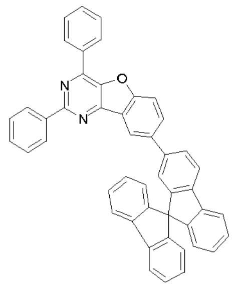 |
| 39c | 19c |
| 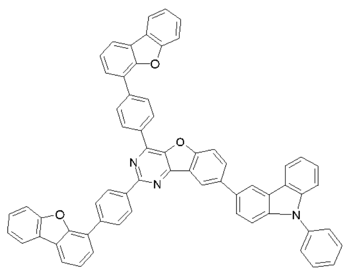 | 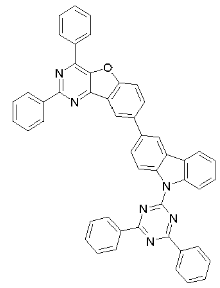 |
| 27c | 30c |

## Patentansprüche

**1.** Verbindung gemäß der Struktur der Formel (A)

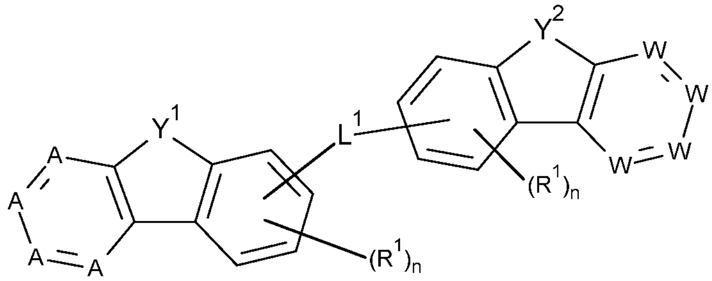

Formel (A)

wobei für die verwendeten Symbole gilt:

$Y^1$ ist O oder S;
$Y^2$ ist N(Ar), O oder $C(R^1)_2$;

W ist bei jedem Auftreten gleich oder verschieden N oder $CR^1$, vorzugsweise $CR^1$, mit der Maßgabe, dass nicht mehr als zwei der Gruppen W in einem Cyclus für N stehen;

$L^1$ ist eine Bindung oder ein aromatisches oder heteroaromatisches Ringsystem mit 5 bis 30 aromatischen Ringatomen, welches durch einen oder mehrere Reste $R^1$ substituiert sein kann;

A ist bei jedem Auftreten gleich oder verschieden N, $CAr^a$ oder $CAr^b$, wobei genau zwei A für N stehen, die durch mindestens eine Gruppe $CAr^a$ oder $CAr^b$ getrennt sind, mit der Maßgabe, dass A für $CAr^b$ steht, falls zu diesem A zwei N benachbart sind;

Ar ist bei jedem Auftreten gleich oder verschieden ein aromatisches oder heteroaromatisches Ringsystem mit 5 bis 30 aromatischen Ringatomen, welches durch einen oder mehrere Reste $R^1$ substituiert sein kann;

$Ar^a$ ist bei jedem Auftreten gleich oder verschieden ein aromatisches oder heteroaromatisches Ringsystem mit 5 bis 30 aromatischen Ringatomen, welches durch einen oder mehrere Reste $R^1$ substituiert sein kann;

$Ar^b$ ist bei jedem Auftreten gleich oder verschieden ein aromatisches oder heteroaromatisches Ringsystem mit 5 bis 30 aromatischen Ringatomen, welches durch einen oder mehrere Reste $R^1$ substituiert sein kann;

$R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)2$, $P(Ar^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C{=}CR^2-$, -C=C-, $Si(R^2)_2$, C=O, C=S, $C{=}NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere Substituenten $R^1$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nichtaromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C{=}NR^2$, $C{=}C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, CHO, $C(=O)R^3$, $CR^3{=}C(R^3)_2$, $C(=O)OR^3$, $C(=O)N(R^3)_2$, $Si(R^3)_3$, $P(R^3)_2$, $B(R^{13})_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C{=}CR^3-$, -C=C-, $Si(R^3)_2$, C=O, C=S, $C{=}NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

n ist 0, 1, 2 oder 3;

mit der Maßgabe, dass
falls die Gruppe $Y^2$ N(Ar) oder O darstellt, der Rest $Ar^a$ keine Carbazol-Gruppe umfasst, wobei dies Substituenten $R^1$, $R^2$ und $R^3$, die an den Rest $Ar^a$ gebunden sein können, einschließt.

**2.** Verbindung gemäß Anspruch 1, ausgewählt aus den Strukturen der Formeln (I), (II) oder (III)

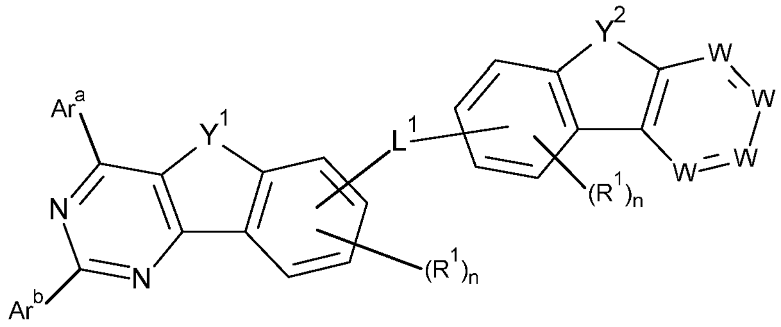

Formel (I)

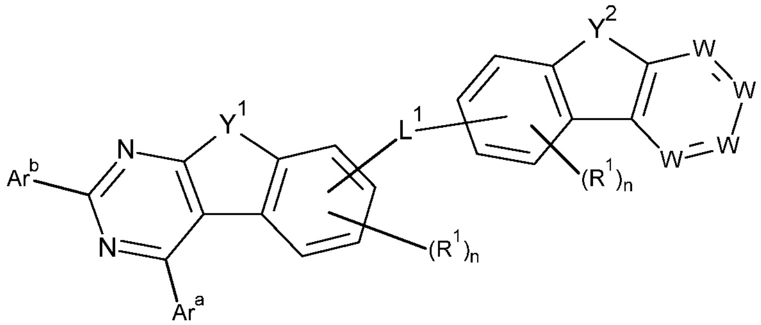

Formel (II)

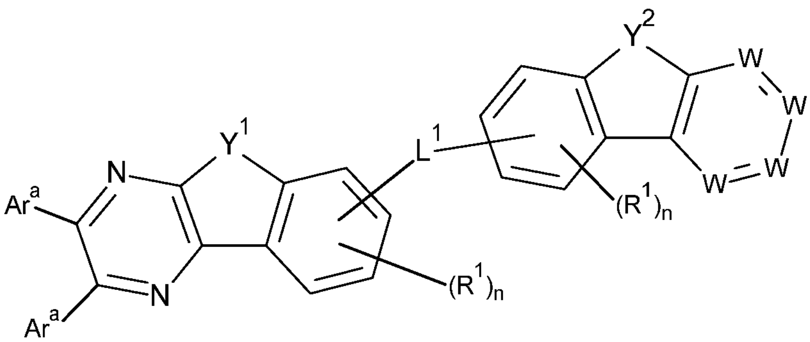

Formel (III)

worin die Symbole $Ar^a$, $Ar^b$, $Y^1$, $L^1$, $Y^2$, $R^1$, n und W die in Anspruch 1 dargelegte Bedeutung aufweisen, wobei Strukturen der Formel (I) und/oder (II) bevorzugt sind.

3. Verbindung gemäß Anspruch 1 oder 2, **dadurch gekennzeichnet, dass** zwei benachbarte Gruppen W jeweils für $CR^1$ stehen und zusammen eine Gruppe der Formel (W-1) bilden

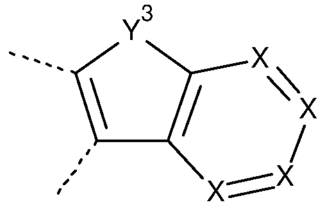

Formel (W-1)

worin

$Y^3$ N(Ar), O, S oder $C(R^2)_2$, vorzugsweise $C(R^2)_2$ ist,

X bei jedem Auftreten gleich oder verschieden N oder $CR^2$, vorzugsweise $CR^2$ ist, mit der Maßgabe, dass nicht mehr als zwei der Gruppen X in einem Cyclus für N stehen, wobei $R^2$ die in Anspruch 1 genannte Bedeutung aufweist, und

die gestrichelten Linien die Bindungen zu den benachbarten Atomen darstellen, wobei die Verbindungen umfassend Strukturen gemäß der Formel (I), (II) oder Formel (III) vorzugsweise höchstens eine Gruppe der Formel (W-1) pro Struktur aufweist.

4. Verbindungen gemäß mindestens einem der vorhergehenden Ansprüche, **dadurch gekennzeichnet, dass** die Verbindung mindestens eine der Strukturen der Formeln (Ia), (IIa) und/oder (IIIa) aufweist

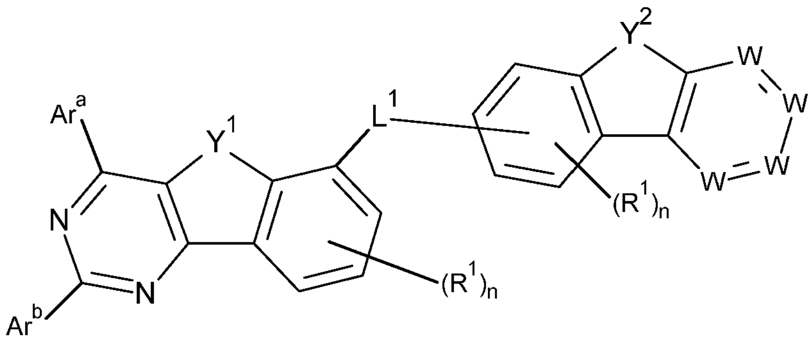

Formel (Ia)

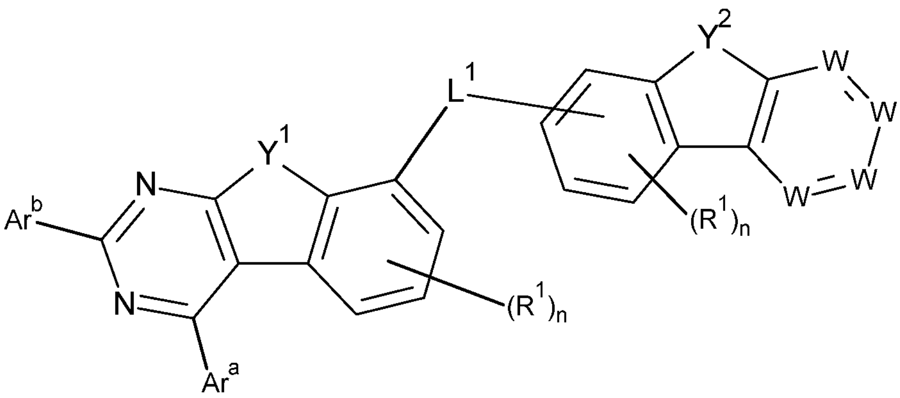

Formel (IIa)

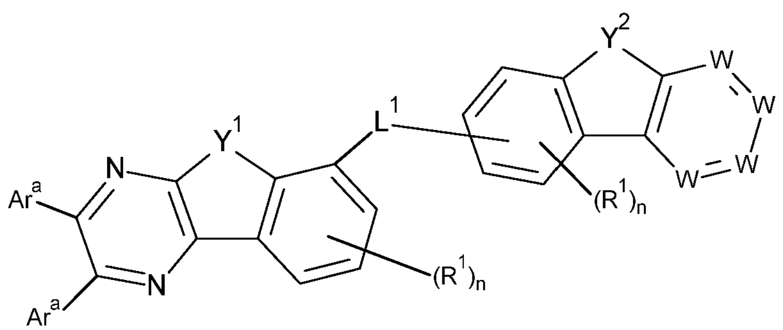

Formel (IIIa)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ia) und/oder (IIa) bevorzugt sind.

**5.** Verbindungen gemäß mindestens einem der vorhergehenden Ansprüche, **dadurch gekennzeichnet, dass** die Verbindung mindestens eine der Strukturen der Formeln (Ib), (IIb) und/oder (IIIb) aufweist

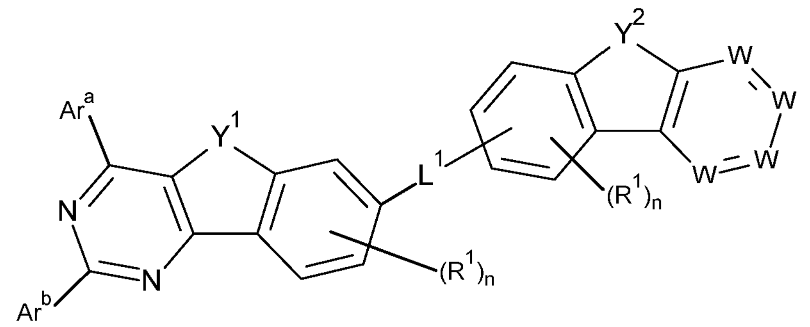

Formel (Ib)

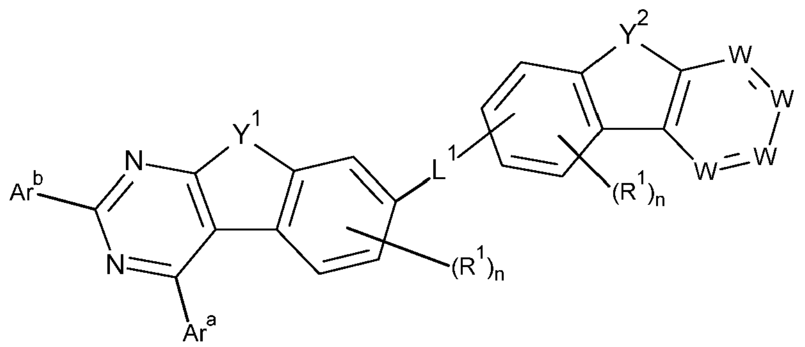

Formel (IIb)

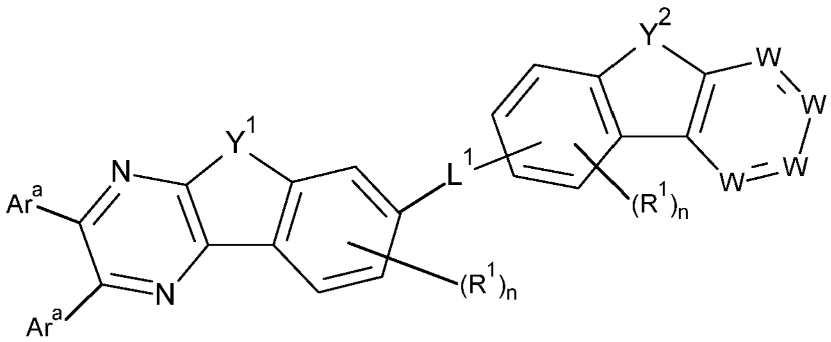

Formel (IIIb)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ib) und/oder (IIb) bevorzugt sind.

**6.** Verbindungen gemäß mindestens einem der vorhergehenden Ansprüche, **dadurch gekennzeichnet, dass** die Verbindung mindestens eine der Strukturen der Formeln (Ic), (IIc) und/oder (IIIc) aufweist

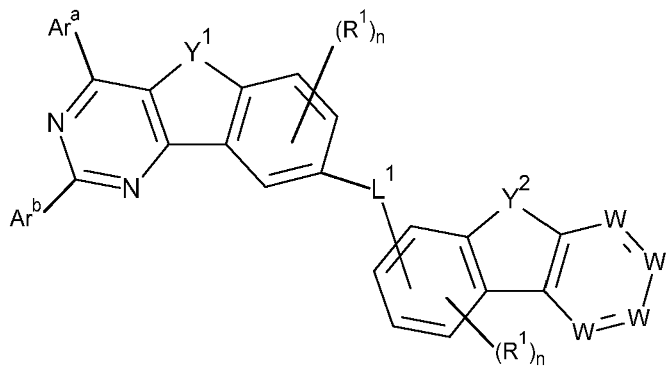

Formel (Ic)

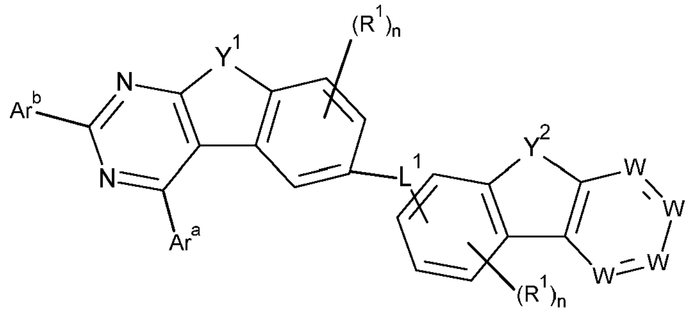

Formel (IIc)

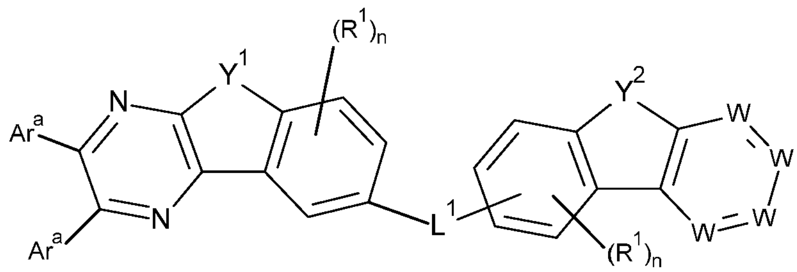

Formel (IIIc)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ic) und/oder (IIc) bevorzugt sind.

7. Verbindungen gemäß mindestens einem der vorhergehenden Ansprüche, **dadurch gekennzeichnet, dass** die Verbindung mindestens eine der Strukturen der Formeln (Id), (IId) und/oder (IIId) aufweist

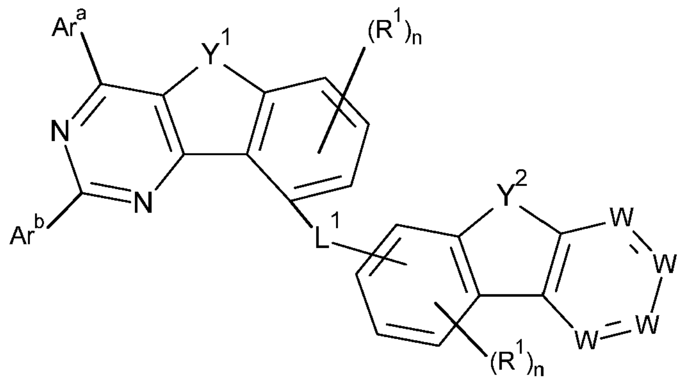

Formel (Id)

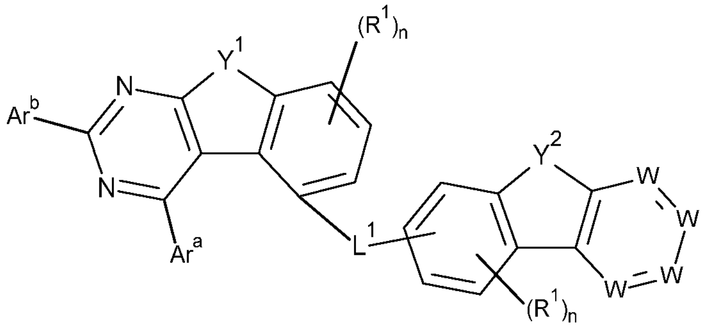

Formel (IId)

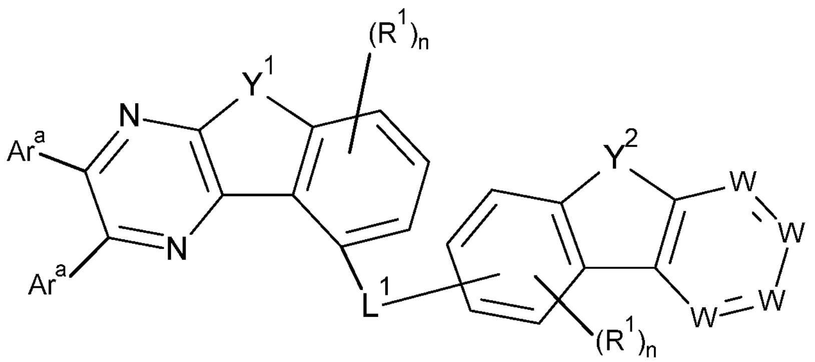

Formel (IIId)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Id) und/oder (IId) bevorzugt sind.

**8.** Verbindungen gemäß mindestens einem der vorhergehenden Ansprüche, **dadurch gekennzeichnet, dass** die Verbindung mindestens eine der Strukturen der Formeln (Ie), (IIe) und/oder (IIIe) aufweist

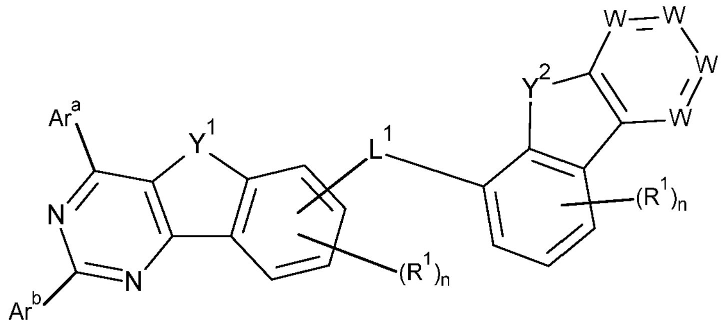

Formel (Ie)

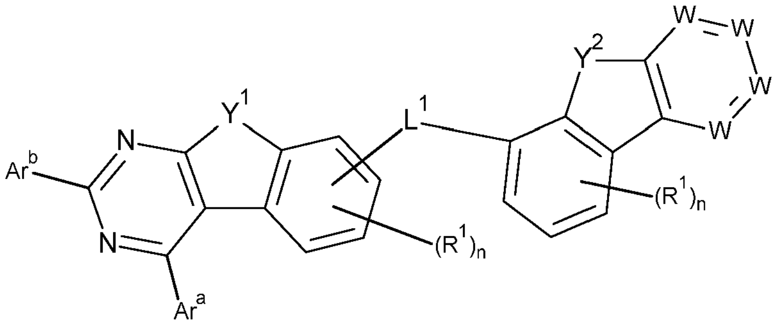

Formel (IIe)

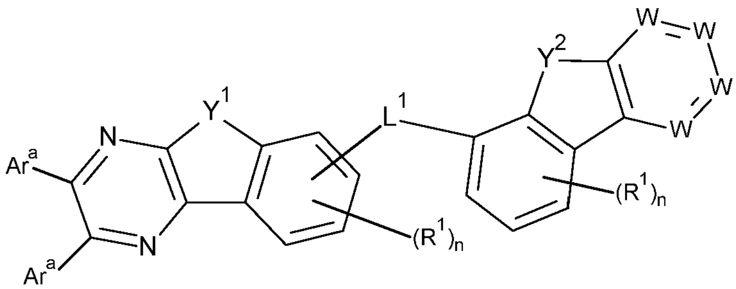

Formel (IIIe)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ie) und/oder (IIe) bevorzugt sind.

**9.** Verbindungen gemäß mindestens einem der vorhergehenden Ansprüche, **dadurch gekennzeichnet, dass** die Verbindung mindestens eine der Strukturen der Formeln (If), (IIf) und/oder (IIIf) aufweist

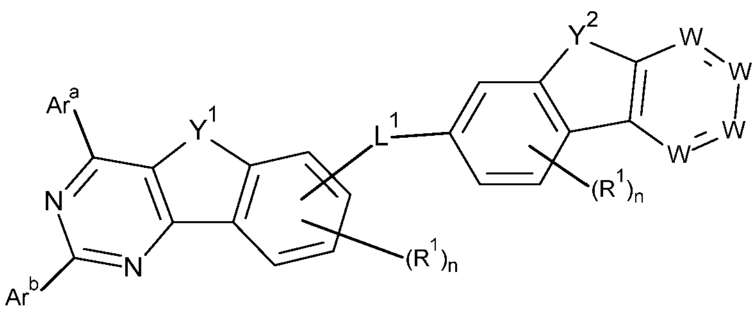

Formel (If)

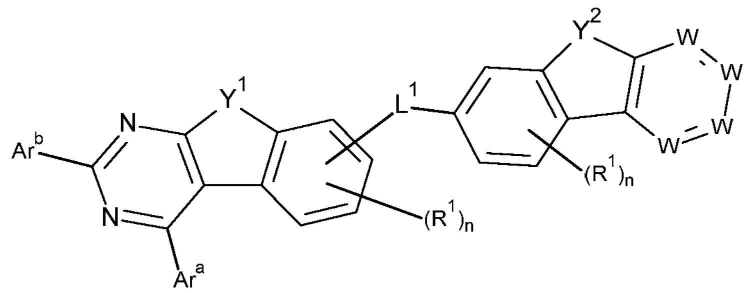

Formel (IIf)

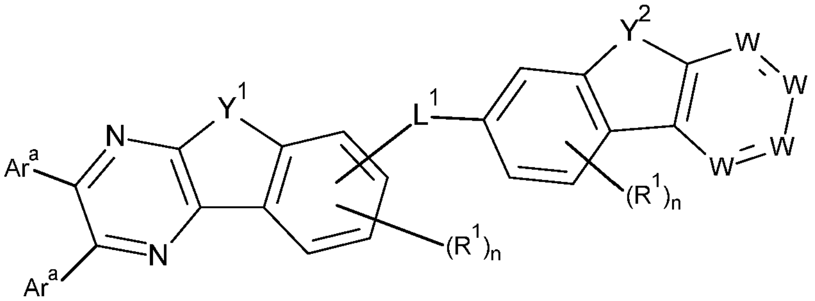

Formel (IIIf)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (If) und/oder (IIf) bevorzugt sind.

**10.** Verbindungen gemäß mindestens einem der vorhergehenden Ansprüche, **dadurch gekennzeichnet, dass** die Verbindung mindestens eine der Strukturen der Formeln (Ig), (IIg) und/oder (IIIg) aufweist

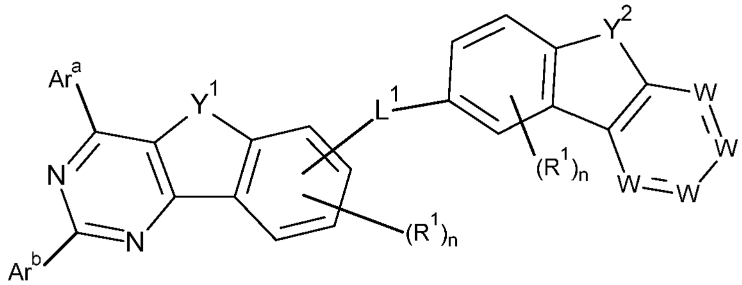

Formel (Ig)

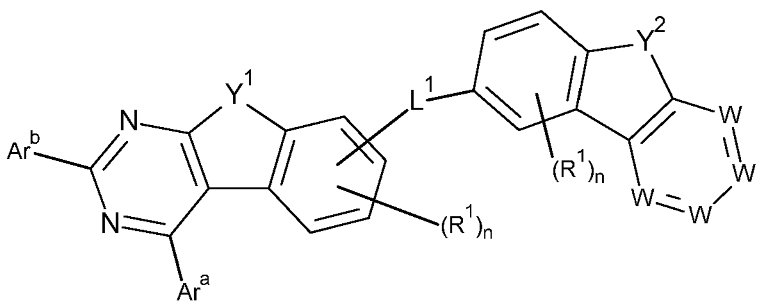

Formel (IIg)

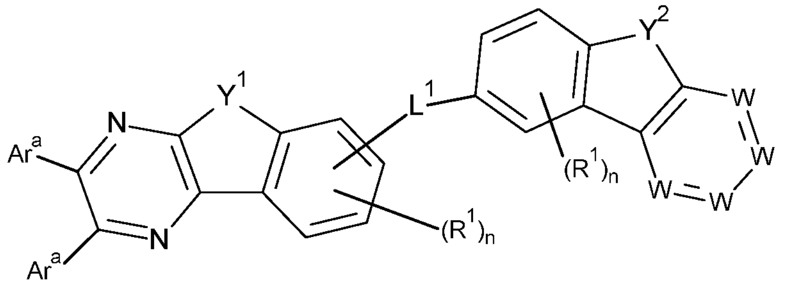

Formel (IIIg)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ig) und/oder (IIg) bevorzugt sind.

11. Verbindungen gemäß mindestens einem der vorhergehenden Ansprüche, **dadurch gekennzeichnet, dass** die Verbindung mindestens eine der Strukturen der Formeln (Ih), (IIh) und/oder (IIIh) aufweist

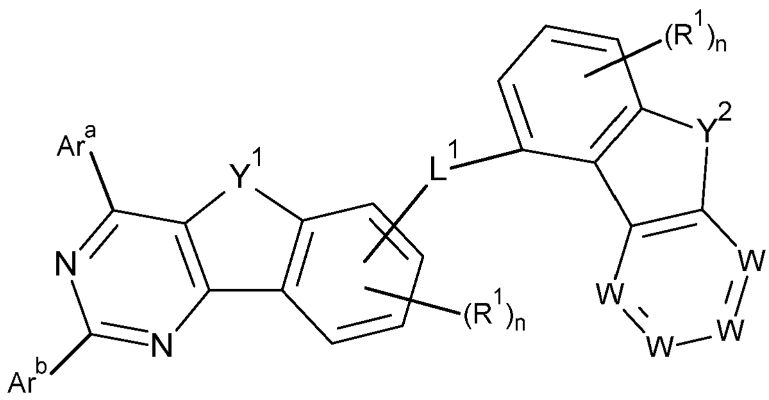

Formel (Ih)

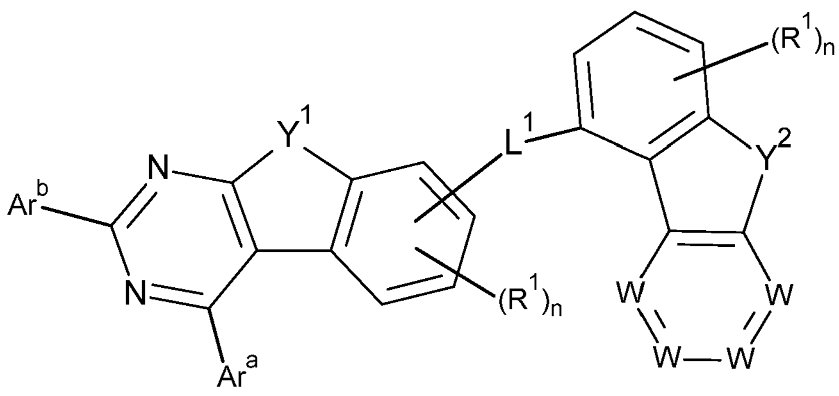

Formel (IIh)

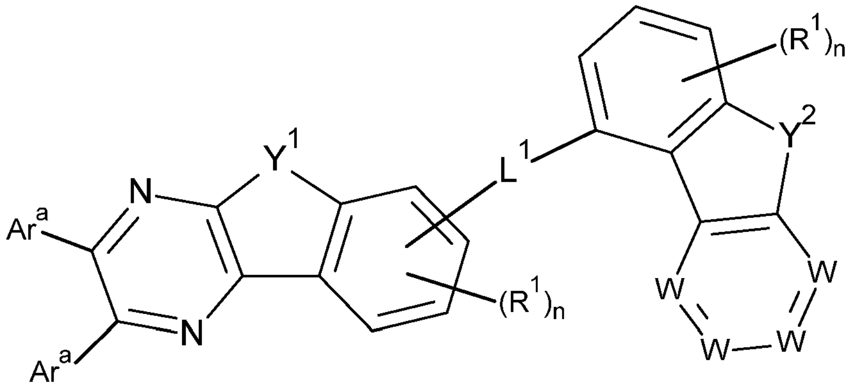

Formel (IIIh)

worin die Symbole $Ar^a$, $Ar^b$, $Y^1$, $L^1$, $Y^2$, $R^1$, n und W die in Anspruch 1 oder 2 dargelegte Bedeutung aufweisen, wobei Strukturen der Formel (Ih) und/oder (IIh) bevorzugt sind.

**12.** Verbindungen gemäß mindestens einem der vorhergehenden Ansprüche, **dadurch gekennzeichnet, dass** das Symbol $Y^2$ $C(R^1)$ ist und $R^1$ jeweils gleich oder verschieden ein aromatisches oder heteroaromatisches Ringsystem mit 5 bis 40 aromatischen Ringatomen ist, das jeweils durch einen oder mehrere Reste $R^2$ substituiert sein kann.

**13.** Verbindungen gemäß mindestens einem der vorhergehenden Ansprüche 1 bis 12, **dadurch gekennzeichnet, dass** das Symbol $Y^2$ für eine Gruppe der Formel ($Y^2$-2) steht

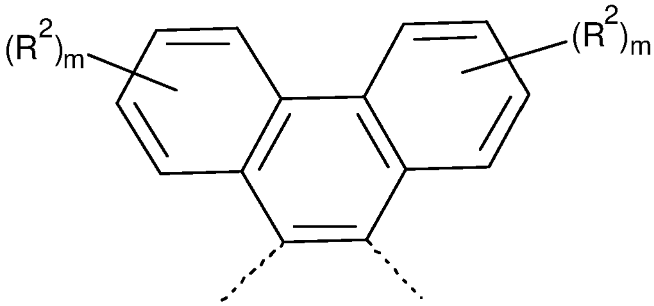

Formel ($Y^2$-2)

worin die gestrichelten Linien die Bindungen zu den benachbarten Atomen darstellen, $R^2$ die in Anspruch 1 genannte Bedeutung aufweist und m 0, 1, 2, 3 oder 4 ist.

**14.** Verbindung gemäß mindestens einem der vorhergehenden Ansprüche, **dadurch gekennzeichnet, dass** die Gruppe $Ar^b$ eine Gruppe der Formel ($Ar^b$-1) darstellt

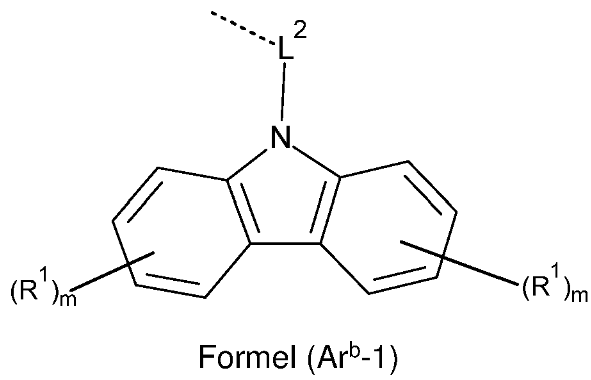

Formel ($Ar^b$-1)

worin $L^2$ eine Bindung oder ein aromatisches oder heteroaromatisches Ringsystem mit 5 bis 30 aromatischen Ringatomen ist, welches durch einen oder mehrere Reste $R^1$ substituiert sein kann, das Symbol $R^1$ die in Anspruch 1 genannte Bedeutung aufweist, m 0, 1, 2, 3 oder 4 ist und die gestrichelte Linie die Bindung darstellt.

**15.** Oligomer, Polymer oder Dendrimer enthaltend eine oder mehrere Verbindungen nach einem oder mehreren der Ansprüche 1 bis 14, wobei ein oder mehrere Bindungen der Verbindung zum Polymer, Oligomer oder Dendrimer vorhanden sind.

**16.** Zusammensetzung enthaltend wenigstens eine Verbindung nach einem oder mehreren der Ansprüche 1 bis 14 und/oder ein Oligomer, Polymer oder Dendrimer nach Anspruch 15 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**17.** Formulierung enthaltend mindestens eine Verbindung nach einem oder mehreren der Ansprüche 1 bis 14, ein Oligomer, Polymer oder Dendrimer nach Anspruch 15 und/oder wenigstens eine Zusammensetzung nach Anspruch 16 und mindestens ein Lösungsmittel.

**18.** Verfahren zur Herstellung einer Verbindung nach einem oder mehreren der Ansprüche 1 bis 14 oder eines Oligomers, Polymers oder Dendrimers nach Anspruch 15, **dadurch gekennzeichnet, dass** in einer Kupplungsreaktion eine Verbindung, umfassend mindestens eine Diazadibenzofuran- oder Diazadibenzothiophen-Gruppe, mit einer Gruppe, umfassend mindestens einen Carbazol-, Fluoren-, Phenanthren-, Benzofuran- und/oder Benzothiophen-Rest, verbunden wird.

**19.** Verwendung einer Verbindung nach einem oder mehreren der Ansprüche 1 bis 14, eines Oligomers, Polymers oder Dendrimers nach Anspruch 15 oder einer Zusammensetzung nach Anspruch 16 in einer elektronischen Vorrichtung als Hostmaterial, Matrixmaterial, Elektronentransportmaterial, Elektroneninjektionsmaterial und/oder Lochblockiermaterial.

**20.** Elektronische Vorrichtung, enthaltend mindestens eine Verbindung nach einem oder mehreren der Ansprüche 1 bis 14 oder ein Oligomer, Polymer oder Dendrimer nach Anspruch 15 oder eine Zusammensetzung nach Anspruch 16, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laser-dioden.

## Claims

**1.** Compound having the structure of the formula (A)

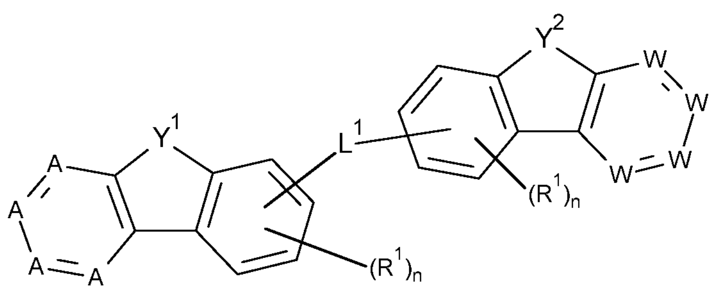

formula (A)

where the following applies to the symbols used:

$Y^1$ is O or S;

$Y^2$ is N(Ar), O or $C(R^1)_2$;
W is on each occurrence, identically or differently, N or $CR^1$, preferably $CR^1$, with the proviso that not more than two of the groups W in a ring stand for N;
$L^1$ is a bond or an aromatic or heteroaromatic ring system having 5 to 30 aromatic ring atoms, which may be substituted by one or more radicals $R^1$;
A is on each occurrence, identically or differently, N, $CAr^a$ or $CAr^b$, where precisely two A stand for N which are separated by at least one group $CAr^a$ or $CAr^b$, with the proviso that A stands for $CAr^b$ if two N are adjacent to this A;
Ar is on each occurrence, identically or differently, an aromatic or heteroaromatic ring system having 5 to 30 aromatic ring atoms, which may be substituted by one or more radicals $R^1$;
$Ar^a$ is on each occurrence, identically or differently, an aromatic or heteroaromatic ring system having 5 to 30 aromatic ring atoms, which may be substituted by one or more radicals $R^1$;
$Ar^b$ is on each occurrence, identically or differently, an aromatic or heteroaromatic ring system having 5 to 30 aromatic ring atoms, which may be substituted by one or more radicals $R^1$;
$R^1$ is on each occurrence, identically or differently, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)2$, $B(Ar^1)_2$, $Si(Ar^1)_3$, $Si(R^2)_3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$, or a combination of these systems; two or more substituents $R^1$ may also form a mono- or polycyclic, aliphatic or aromatic ring system with one another;
$Ar^1$ is on each occurrence, identically or differently, an aromatic or heteroaromatic ring system having 5 to 30 aromatic ring atoms, which may be substituted by one or more non-aromatic radicals $R^2$; two radicals $Ar^1$ that are bonded to the same Si atom, N atom, P atom or B atom may also be bridged to one another by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$;
$R^2$ is on each occurrence, identically or differently, H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^{13})_2$, $N(R^3)_2$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, $-C\equiv C-$, $Si(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more adjacent substituents $R^2$ may also form a mono- or polycyclic, aliphatic or aromatic ring system with one another;
$R^3$ is on each occurrence, identically or differently, H, D, F or an aliphatic, aromatic and/or heteroaromatic hydrocarbon radical having 1 to 20 C atoms, in which H atoms may also be replaced by F; two or more adjacent substituents $R^3$ may also form a mono- or polycyclic, aliphatic or aromatic ring system with one another;
n is 0, 1, 2 or 3;

with the proviso that,
if the group $Y^2$ represents N(Ar) or O, the radical $Ar^a$ does not contain a carbazole group, where this includes substituents $R^1$, $R^2$ and $R^3$ that may be bonded to the radical $Ar^a$.

**2.** Compound according to Claim 1, selected from the structures of the formulae (I), (II) and (III)

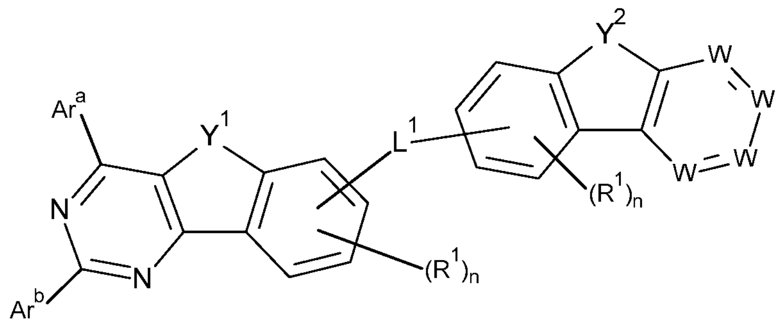

formula (I)

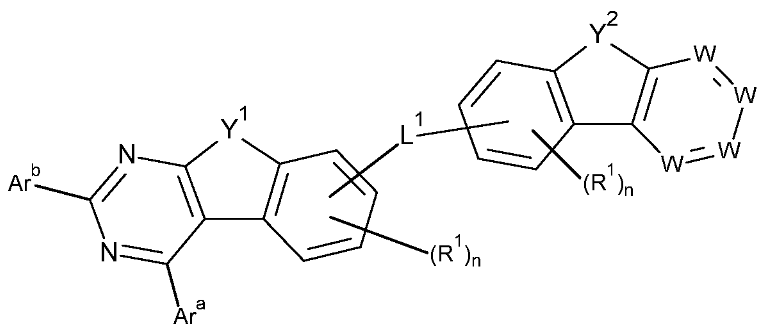

formula (II)

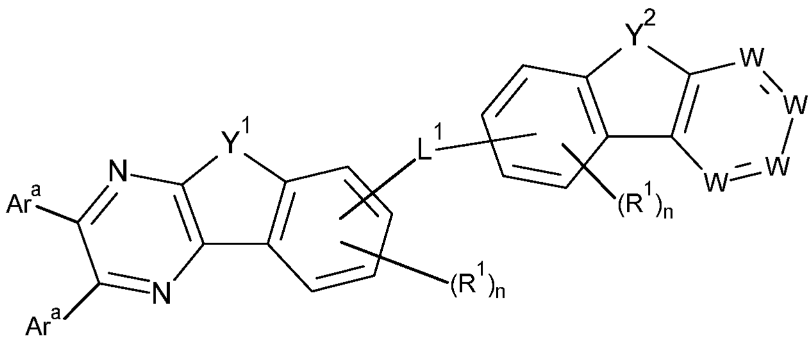

formula (III)

in which the symbols $Ar^a$, $Ar^b$, $Y^1$, $L^1$, $Y^2$, $R^1$, n and Whave the meaning indicated in Claim 1, where structures of the formula (I) and/or (II) are preferred.

**3.** Compound according to Claim 1 or 2, **characterised in that** two adjacent groups W each stand for $CR^1$ and together form a group of the formula (W-1)

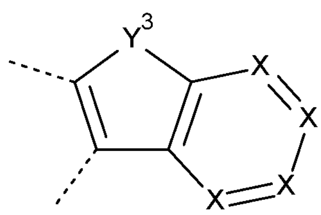

formula (W-1)

in which

$Y^3$ is N(Ar), O, S or $C(R^2)_2$, preferably $C(R^2)_2$,
X is on each occurrence, identically or differently, N or $CR^2$, preferably $CR^2$, with the proviso that not more than two of the groups X in a ring stand for N, where $R^2$ has the meaning given in Claim 1, and

the dashed lines represent the bonds to the adjacent atoms, where the compounds having structures of the formula (I), (II) or (III) preferably contain at most one group of the formula (W-1) per structure.

**4.** Compounds according to at least one of the preceding claims, **characterised in that** the compound has at least one of the structures of the formulae (Ia), (IIa) and/or (IIIa)

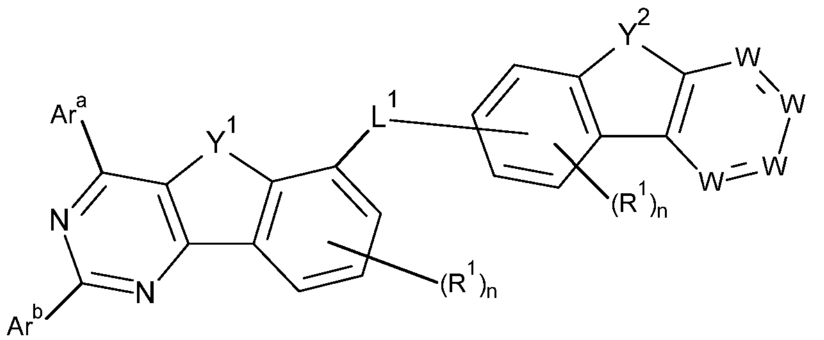

formula (Ia)

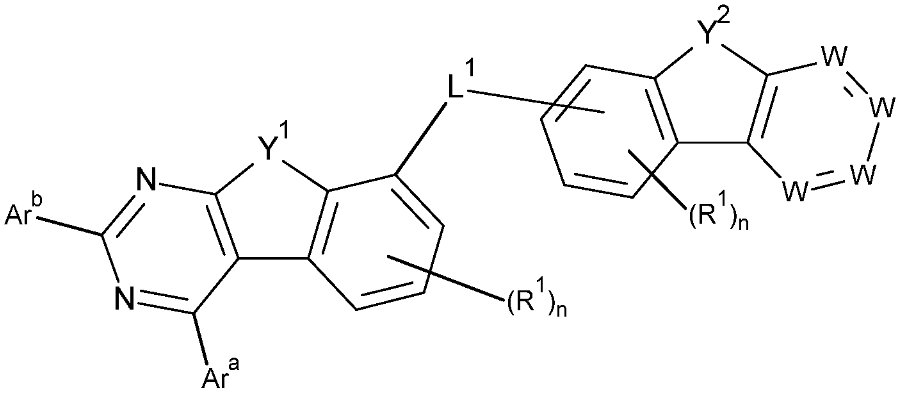

formula (IIa)

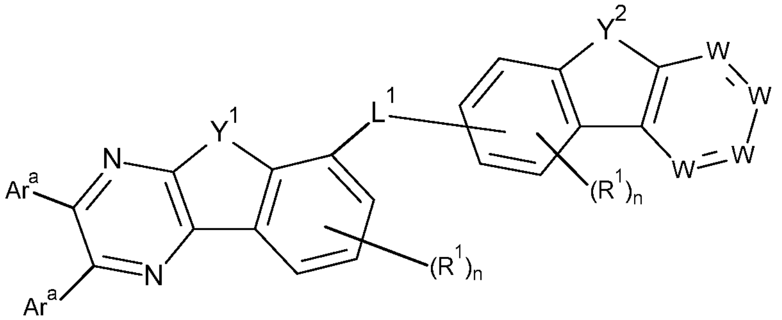

formula (IIIa)

in which the symbols $Ar^a$, $Ar^b$, $Y^1$, $L^1$, $Y^2$, $R^1$, n and Whave the meaning indicated in Claim 1 or 2, where structures of the formula (Ia) and/or (IIa) are preferred.

**5.** Compounds according to at least one of the preceding claims, **characterised in that** the compound has at least one of the structures of the formulae (Ib), (IIb) and/or (IIIb)

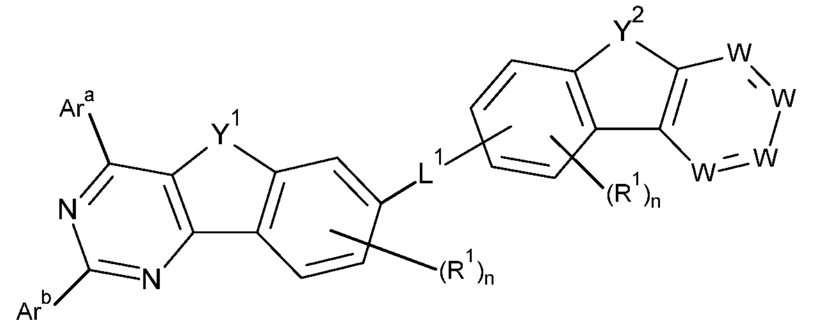

formula (Ib)

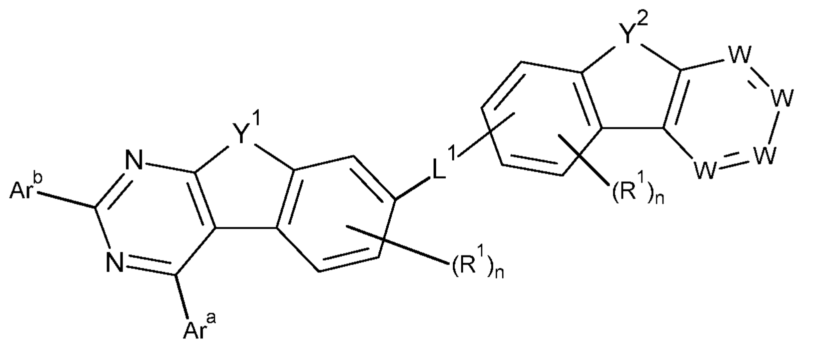

formula (IIb)

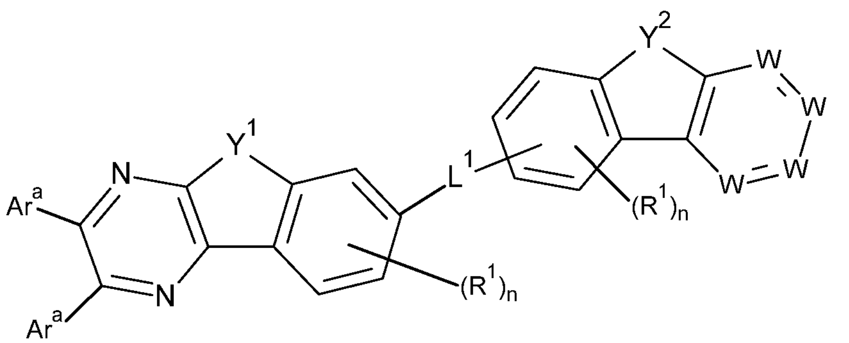

formula (IIIb)

in which the symbols $Ar^a$, $Ar^b$, $Y^1$, $L^1$, $Y^2$, $R^1$, n and Whave the meaning indicated in Claim 1 or 2, where structures of the formula (Ib) and/or (IIb) are preferred.

**6.** Compounds according to at least one of the preceding claims, **characterised in that** the compound has at least one of the structures of the formulae (Ic), (IIc) and/or (IIIc)

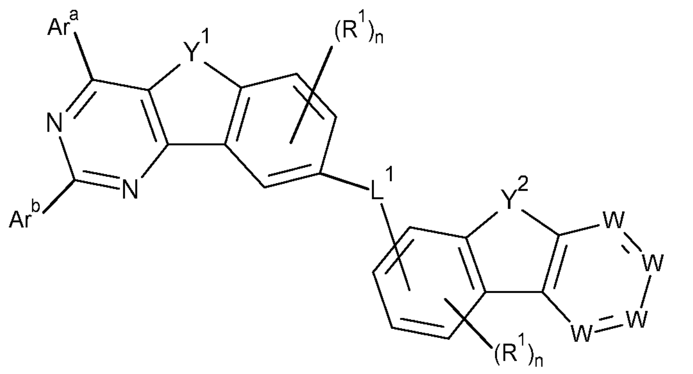

formula (Ic)

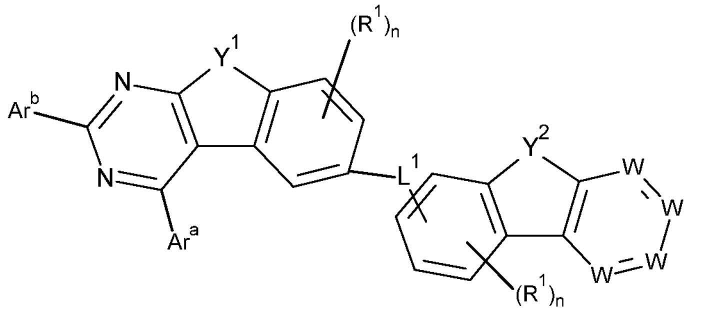

formula (IIc)

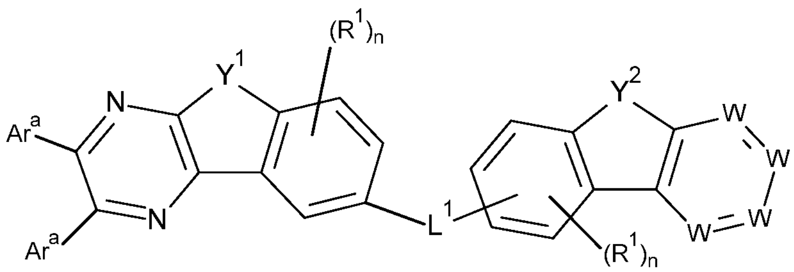

formula (IIIc)

in which the symbols $Ar^a$, $Ar^b$, $Y^1$, $L^1$,$Y^2$, $R^1$, n and Whave the meaning indicated in Claim 1 or 2, where structures of the formula (Ic) and/or (IIc) are preferred.

7. Compounds according to at least one of the preceding claims, **characterised in that** the compound has at least one of the structures of the formulae (Id), (lid) and/or (IIId)

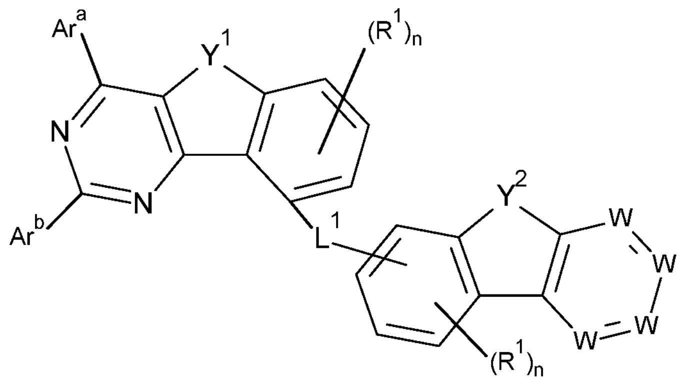

formula (Id)

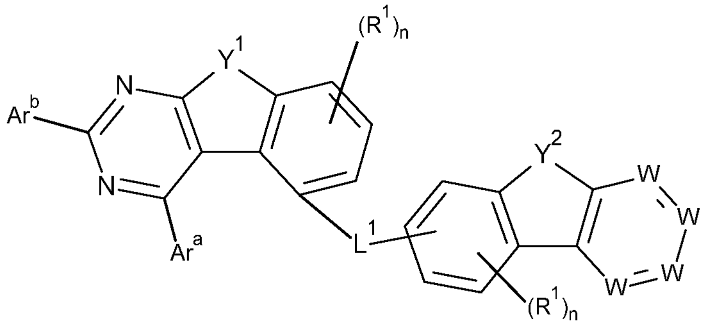

formula (IId)

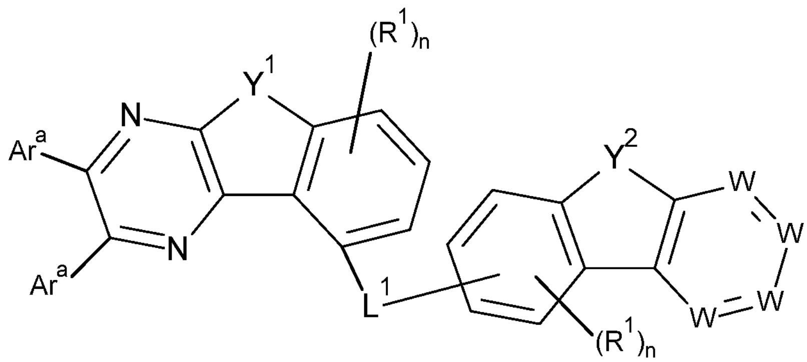

formula (IIId)

in which the symbols $Ar^a$, $Ar^b$, $Y^1$, $L^1$, $Y^2$, $R^1$, n and Whave the meaning indicated in Claim 1 or 2, where structures of the formula (Id) and/or (lid) are preferred.

8. Compounds according to at least one of the preceding claims, **characterised in that** the compound has at least one of the structures of the formulae (Ie), (lie) and/or (IIIe)

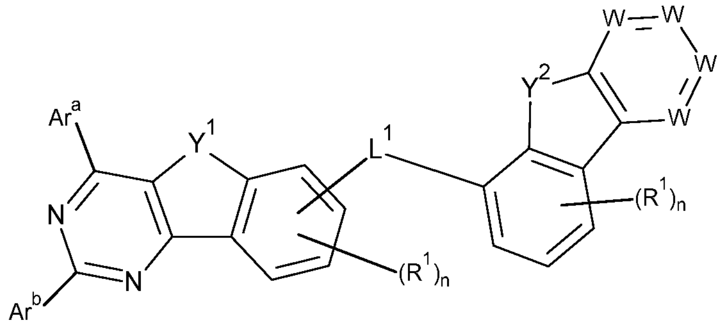

formula (Ie)

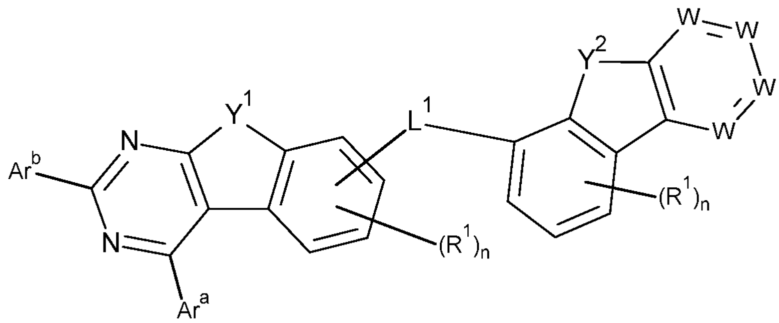

formula (IIe)

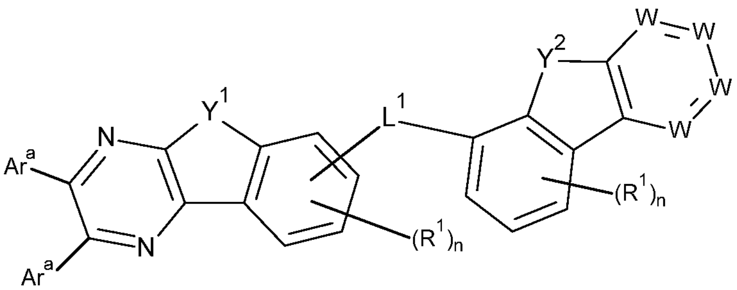

formula (IIIe)

in which the symbols $Ar^a$, $Ar^b$, $Y^1$, $L^1$, $Y^2$, $R^1$, n and Whave the meaning indicated in Claim 1 or 2, where structures of the formula (Ie) and/or (lie) are preferred.

9. Compounds according to at least one of the preceding claims, **characterised in that** the compound has at least one of the structures of the formulae (If), (IIf) and/or (IIIf)

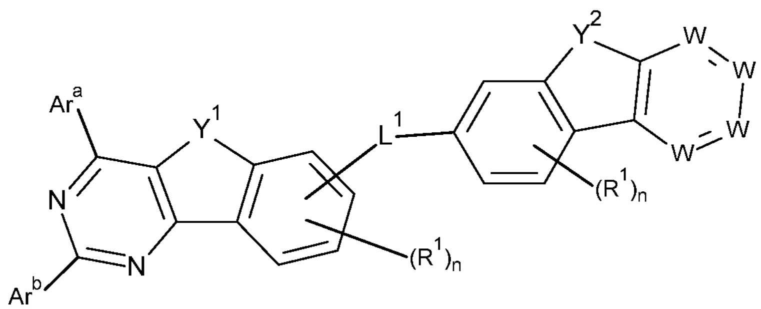

formula (If)

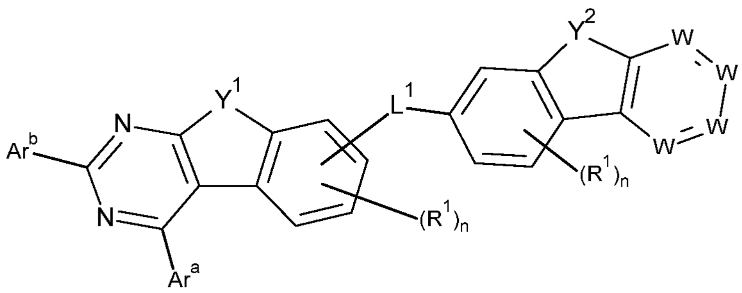

formula (IIf)

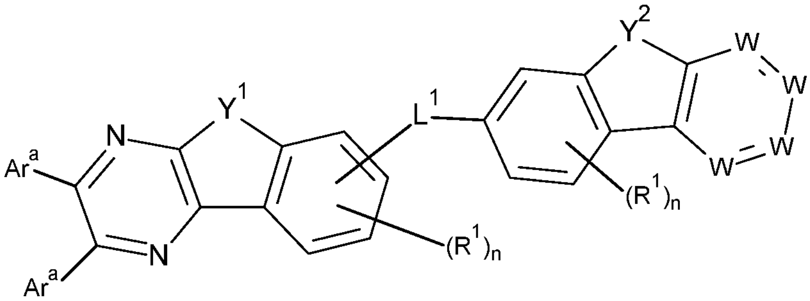

formula (IIIf)

in which the symbols $Ar^a$, $Ar^b$, $Y^1$, $L^1$, $Y^2$, $R^1$, n and Whave the meaning indicated in Claim 1 or 2, where structures of the formula (If) and/or (IIf) are preferred.

**10.** Compounds according to at least one of the preceding claims, **characterised in that** the compound has at least one of the structures of the formulae (Ig), (IIg) and/or (IIIg)

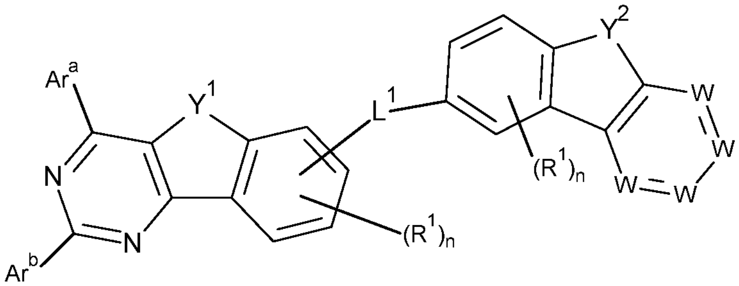

formula (Ig)

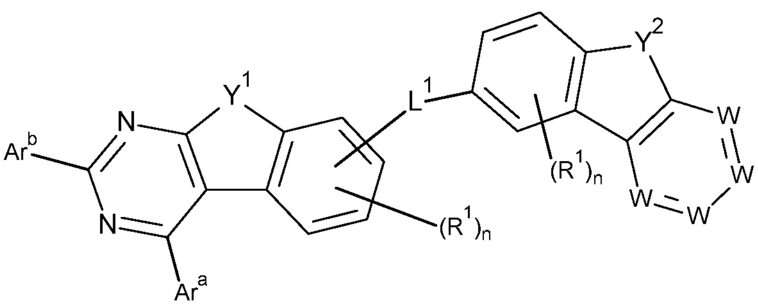

formula (IIg)

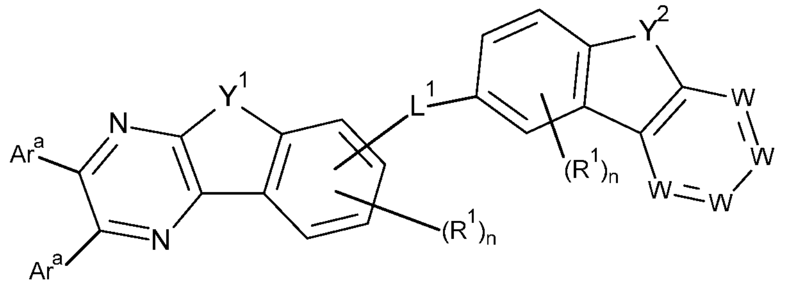

formula (IIIg)

in which the symbols $Ar^a$, $Ar^b$, $Y^1$, $L^1$, $Y^2$, $R^1$, n and Whave the meaning indicated in Claim 1 or 2, where structures of the formula (Ig) and/or (IIg) are preferred.

11. Compounds according to at least one of the preceding claims, **characterised in that** the compound has at least one of the structures of the formulae (Ih), (IIh) and/or (IIIh)

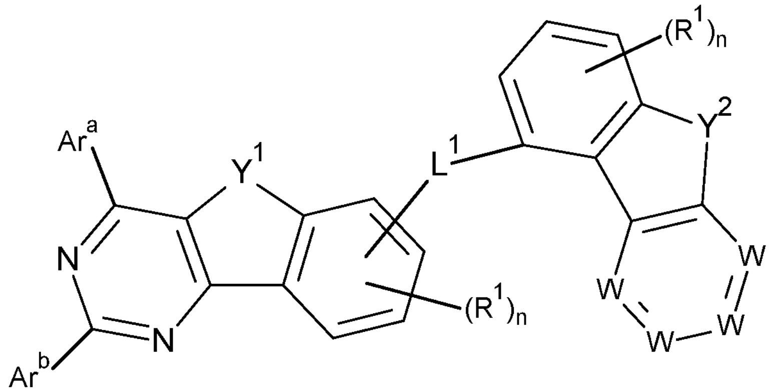

formula (Ih)

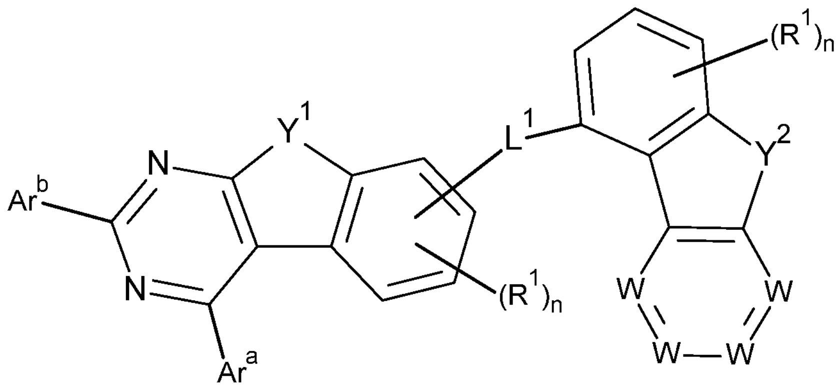

formula (IIh)

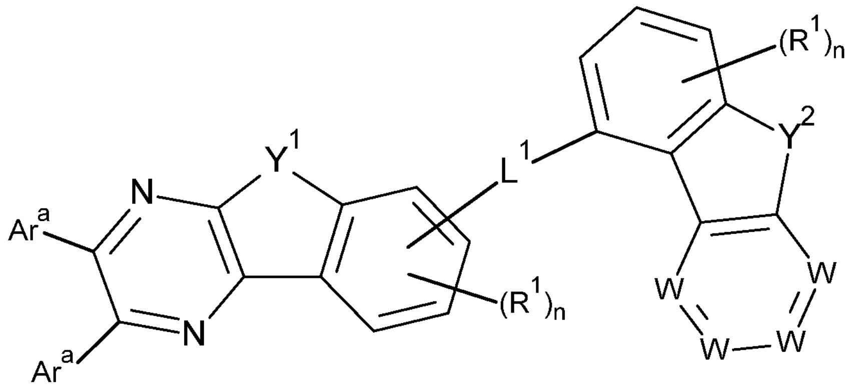

formula (IIIh)

in which the symbols $Ar^a$, $Ar^b$, $Y^1$, $L^1$, $Y^2$, $R^1$, n and Whave the meaning indicated in Claim 1 or 2, where structures of the formula (Ih) and/or (IIh) are preferred.

**12.** Compounds according to at least one of the preceding claims, **characterised in that** the symbol $Y^2$ is $C(R^1)$ and $R^1$ is in each case, identically or differently, an aromatic or heteroaromatic ring system having 5 to 40 aromatic ring atoms, which may in each case be substituted by one or more radicals $R^2$.

**13.** Compounds according to at least one of the preceding Claims 1 to 12, **characterised in that** the symbol $Y^2$ stands for a group of the formula ($Y^2$-2)

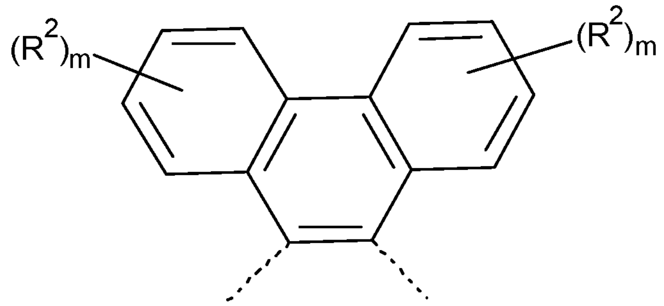

formula ($Y^2$-2)

in which the dashed lines represent the bonds to the adjacent atoms, $R^2$ has the meaning given in Claim 1 and m is 0, 1, 2, 3 or 4.

**14.** Compound according to at least one of the preceding claims, **characterised in that** the group $Ar^b$ represents a group of the formula ($Ar^b$-1)

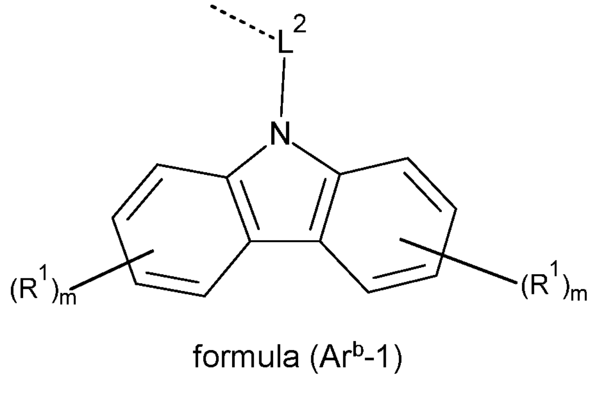

formula ($Ar^b$-1)

in which $L^2$ is a bond or an aromatic or heteroaromatic ring system having 5 to 30 aromatic ring atoms, which may be substituted by one or more radicals $R^1$, the symbol $R^1$ has the meaning given in Claim 1, m is 0, 1, 2, 3 or 4 and the dashed line represents the bond.

**15.** Oligomer, polymer or dendrimer containing one or more compounds according to one or more of Claims 1 to 14, where one or more bonds are present from the compound to the polymer, oligomer or dendrimer.

**16.** Composition comprising at least one compound according to one or more of Claims 1 to 14 and/or an oligomer, polymer or dendrimer according to Claim 15 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**17.** Formulation comprising at least one compound according to one or more of Claims 1 to 14, an oligomer, polymer or dendrimer according to Claim 15 and/or at least one composition according to Claim 16 and at least one solvent.

**18.** Process for the preparation of a compound according to one or more of Claims 1 to 14 or an oligomer, polymer or dendrimer according to Claim 15, **characterised in that** a compound containing at least one diazadibenzofuran or diazadibenzothiophene group is bonded to a group containing at least one carbazole, fluorene, phenanthrene, benzofuran and/or benzothiophene radical in a coupling reaction.

**19.** Use of a compound according to one or more of Claims 1 to 14, an oligomer, polymer or dendrimer according to Claim 15 or a composition according to Claim 16 in an electronic device as host material, matrix material, electron-transport material, electron-injection material and/or hole-blocking material.

**20.** Electronic device containing at least one compound according to one or more of Claims 1 to 14 or an oligomer, polymer or dendrimer according to Claim 15 or a composition according to Claim 16, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells and organic laser diodes.

**Revendications**

**1.** Composé présentant la structure de la formule (A)

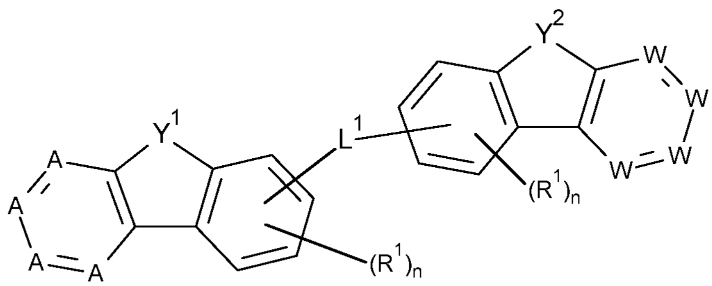

formule (A)

dans laquelle ce qui suit s'applique aux symboles qui sont utilisés :

$Y^1$ est O ou S ;
$Y^2$ est N(Ar), O ou $C(R^1)_2$ ;
W est pour chaque occurrence, de manière identique ou différente, N ou $CR^1$, de préférence $CR^1$, étant entendu que pas plus de deux des groupes W dans un cycle représentent N ;
$L^1$ est une liaison ou un système de cycle aromatique ou hétéroaromatique qui comporte de 5 à 30 atomes de cycle aromatique, lequel peut être substitué par un radical ou par plusieurs radicaux $R^1$ ;

A est pour chaque occurrence, de manière identique ou différente, N, $CAr^a$ ou $CAr^b$, où précisément deux A représentent N, lesquels sont séparés par au moins un groupe $CAr^a$ ou $CAr^b$, étant entendu que A représente $CAr^b$ si deux N sont adjacents à ce A;

Ar est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux $R^1$ ;

$Ar^a$ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux $R^1$ ;

$Ar^b$ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux $R^1$ ;

$R^1$ est pour chaque occurrence, de manière identique ou différente, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)2$, $B(Ar^1)_2$, $Si(Ar^1)_3$, $Si(R^2)_3$, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $-R^2C=CR^2-$, $-C\equiv C-$, $Si(R^2)_2$, C=O, C=S, $C=NR^2$, -C(=O)O-, $-C(=O)NR^2-$, $NR^2$, $P(=O)(R^2)$, -O-, -S-, SO ou $SO_2$ et où un ou plusieurs atome(s) de H peut/ peuvent être remplacé(s) par D, F, Cl, Br, I, CN ou $NO_2$, ou un système de cycle aromatique ou hétéroaromatique qui comporte de 5 à 40 atomes de cycle aromatique, lequel peut dans chaque cas être substitué par un radical ou par plusieurs radicaux $R^2$, ou un groupe aryloxy ou hétéroaryloxy qui comporte de 5 à 40 atomes de cycle aromatique, lequel peut dans chaque cas être substitué par un radical ou par plusieurs radicaux $R^2$, ou un groupe aralkyle ou hétéroaralkyle qui comporte de 5 à 40 atomes de cycle aromatique, lequel peut dans chaque cas être substitué par un radical ou par plusieurs radicaux $R^2$, ou une combinaison de ces systèmes ; deux substituants $R^1$ ou plus peuvent également former un système de cycle aliphatique ou aromatique mono- ou polycyclique l'un avec l'autre ou les uns avec les autres ;

$Ar^1$ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux non aromatique(s) $R^2$ ; deux radicaux $Ar^1$ qui sont liés au même atome de Si, au même atome de N, au même atome de P ou au même atome de B peuvent également être pontés l'un à l'autre au moyen d'une liaison simple ou d'un pont qui est sélectionné parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;

$R^2$ est pour chaque occurrence, de manière identique ou différente, H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^{13})_2$, $N(R^3)_2$, $NO_2$, $P(=O)(R^3)_2$, $OSO_2R^3$, $OR^3$, $S(=O)R^3$, $S(=O)_2R^3$, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 40 atomes de cycle aromatique, lequel peut dans chaque cas être substitué par un radical ou par plusieurs radicaux $R^3$, ou un groupe aryloxy ou hétéroaryloxy qui comporte de 5 à 40 atomes de cycle aromatique, lequel peut être substitué par un radical ou par plusieurs radicaux $R^3$, ou une combinaison de ces systèmes ; deux substituants $R^2$ adjacents ou plus peuvent également former un système de cycle aliphatique ou aromatique mono- ou polycyclique l'un avec l'autre ou les uns avec les autres ;

$R^3$ est pour chaque occurrence, de manière identique ou différente, H, D, F ou un radical hydrocarbone aliphatique, aromatique et/ou hétéroaromatique qui comporte de 1 à 20 atome(s) de C, où des atomes de H peuvent également être remplacés par F ; deux substituants $R^3$ adjacents ou plus peuvent également former un système de cycle aliphatique ou aromatique mono- ou polycyclique l'un avec l'autre ou les uns avec les autres ;

n est 0, 1, 2 ou 3 ;

étant entendu que,
si le groupe $Y^2$ représente N(Ar) ou O, le radical $Ar^a$ n'inclut pas un groupe carbazole, où celui-ci inclut des substituants $R^1$, $R^2$ et $R^3$ qui peuvent être liés au radical $Ar^a$.

**2.** Composé selon la revendication 1, sélectionné parmi les structures des formules (I), (II) et (III)

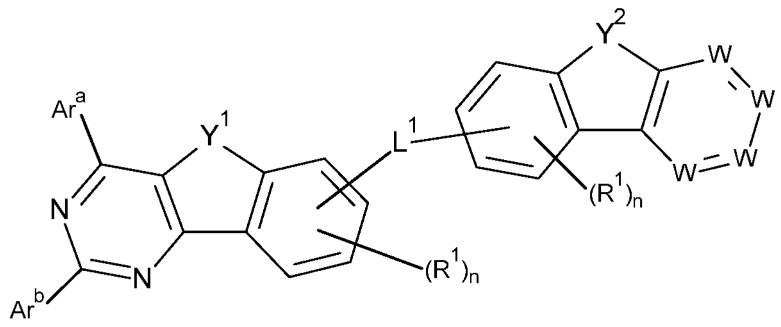

formule (I)

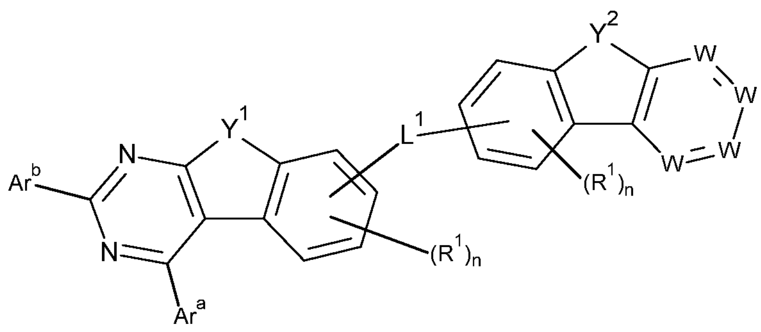

formule (II)

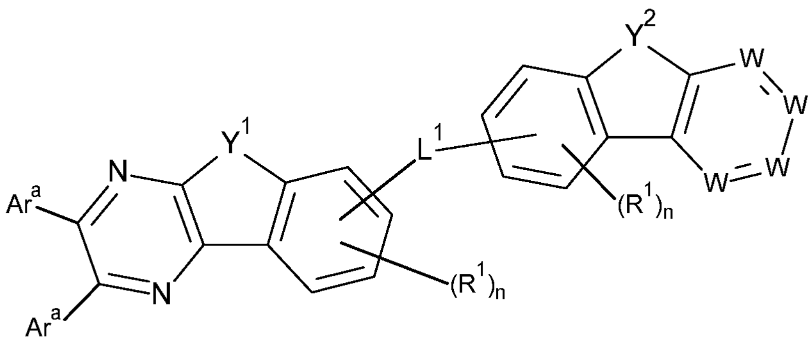

formule (III)

dans lesquelles les symboles $Ar^a$, $Ar^b$, $Y^1$, L 1, $Y^2$, $R^1$, n et W présentent la signification qui a été indiquée selon la revendication 1, où les structures des/de la formule(s) (I) et/ou (II) ont la préférence.

3. Composé selon la revendication 1 ou 2, **caractérisé en ce que** deux groupes adjacents W représentent chacun $CR^1$ et forment ensemble un groupe de la formule (W-1)

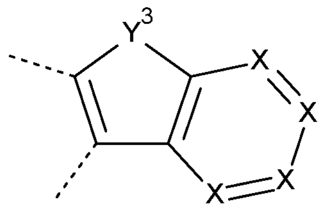

formule (W-1)

dans laquelle

$Y^3$ est N(Ar), O, S ou $C(R^2)_2$, de préférence $C(R^2)_2$,
X est pour chaque occurrence, de manière identique ou différente, N ou $CR^2$, de préférence $CR^2$, étant entendu que pas plus de deux des groupes X dans un cycle représentent N, où $R^2$ présente la signification qui a été donnée selon la revendication 1, et

les lignes en pointillés représentent les liaisons sur les atomes adjacents, où les composés qui présentent des structures de la formule (I), (II) ou (III) contiennent de préférence au moins un groupe de la formule (W-1) par structure.

4. Composé selon au moins l'une des revendications précédentes, **caractérisé en ce que** le composé comporte au moins l'une des structures des formules (Ia), (IIa) et/ou (IIIa)

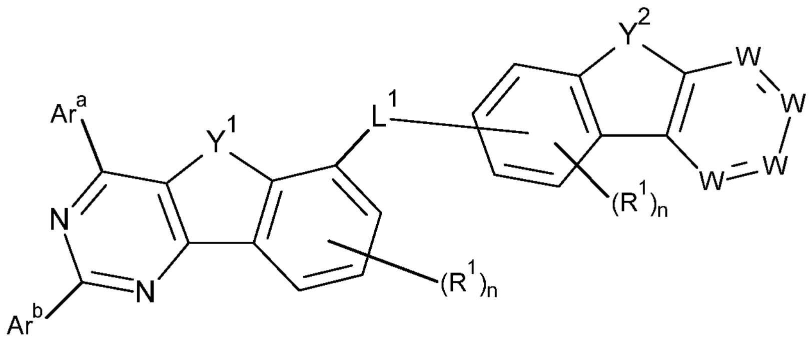

formule (Ia)

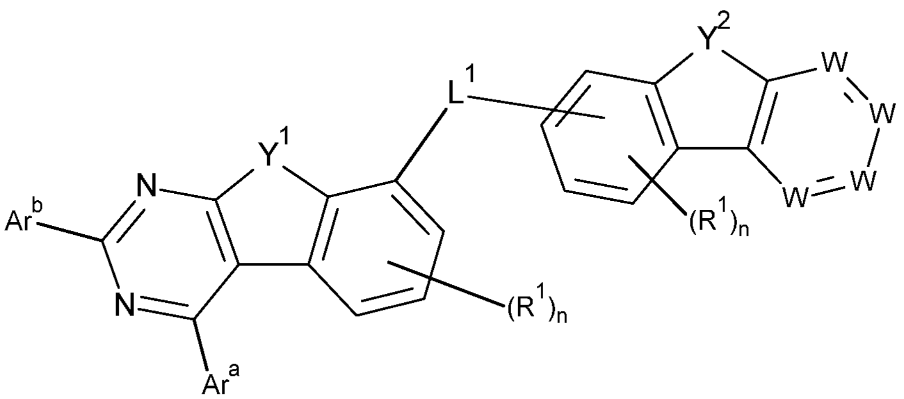

formule (IIa)

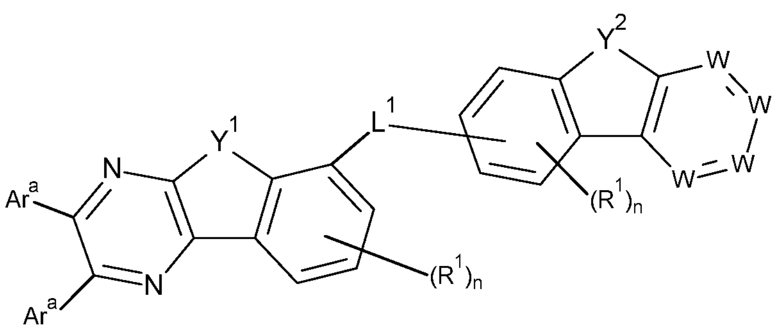

formule (IIIa)

dans lesquelles les symboles $Ar^a$, $Ar^b$, $Y^1$, L 1, $Y^2$, $R^1$, n et W présentent la signification qui a été indiquée selon la revendication 1 ou 2, où les structures des/de la formule(s) (Ia) et/ou (IIa) ont la préférence.

5. Composé selon au moins l'une des revendications précédentes, **caractérisé en ce que** le composé comporte au

moins l'une des structures des formules (Ib), (IIb) et/ou (IIIb)

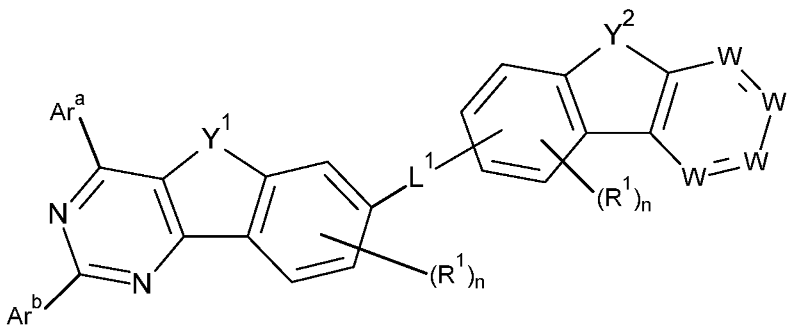

formule (Ib)

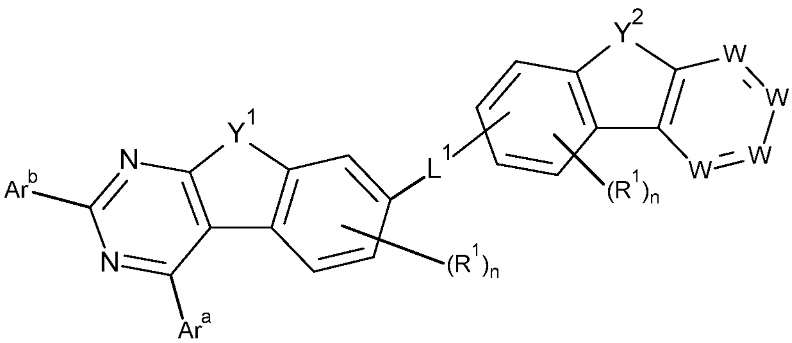

formule (IIb)

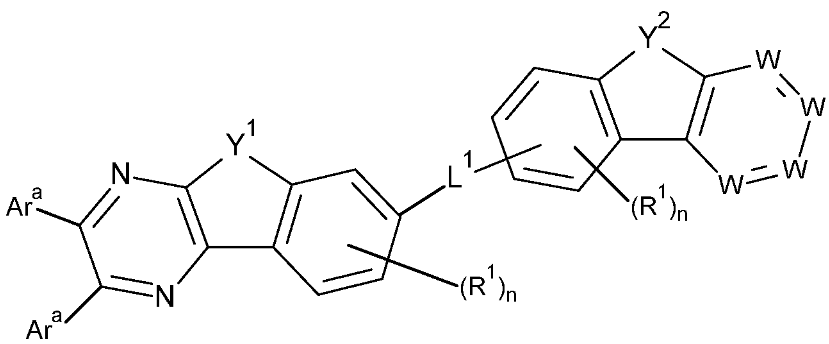

formule (IIIb)

dans lesquelles les symboles $Ar^a$, $Ar^b$, $Y^1$, L 1, $Y^2$, $R^1$, n et W présentent la signification qui a été indiquée selon la revendication 1 ou 2, où les structures des/de la formule(s) (Ib) et/ou (IIb) ont la préférence.

**6.** Composé selon au moins l'une des revendications précédentes, **caractérisé en ce que** le composé comporte au moins l'une des structures des formules (Ic), (IIc) et/ou (IIIc)

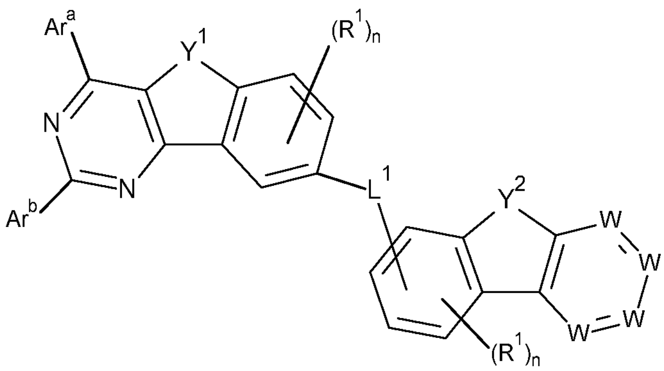

formule (Ic)

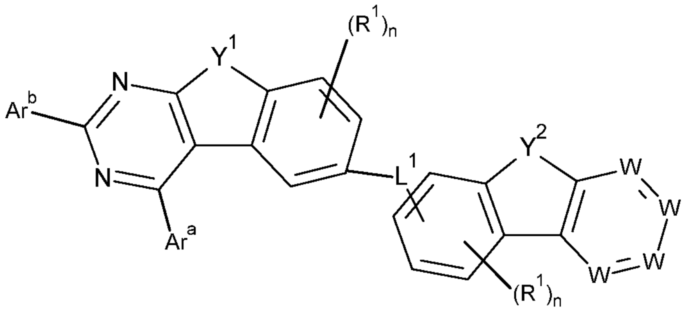

formule (IIc)

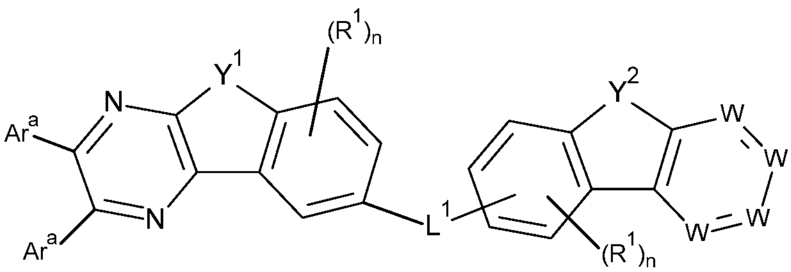

formule (IIIc)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule(s) (Ic) et/ou (IIc) ont la préférence.

7. Composé selon au moins l'une des revendications précédentes, **caractérisé en ce que** le composé comporte au moins l'une des structures des formules (Id), (IId) et/ou (IIId)

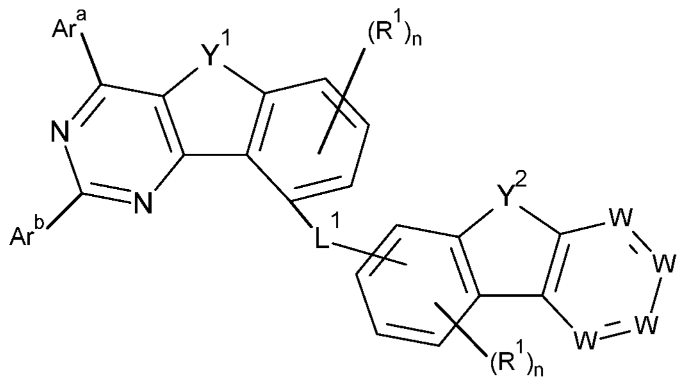

formule (Id)

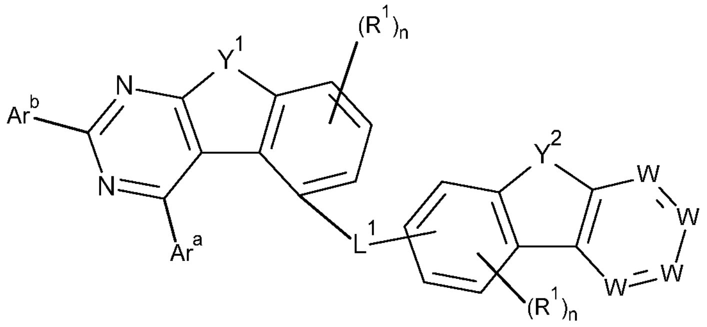

formule (IId)

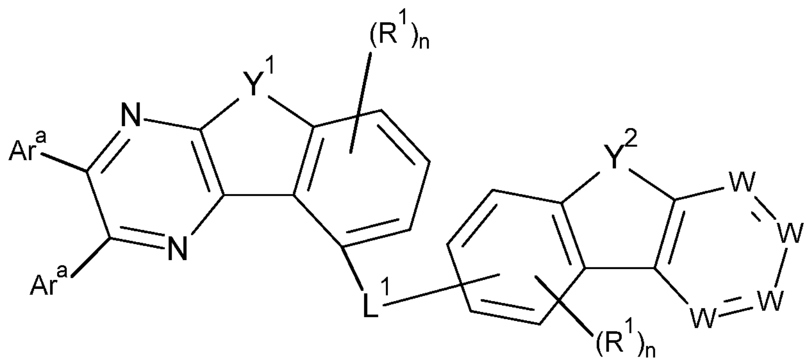

formule (IIId)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule(s) (Id) et/ou (IId) ont la préférence.

8. Composé selon au moins l'une des revendications précédentes, **caractérisé en ce que** le composé comporte au moins l'une des structures des formules (Ie), (IIe) et/ou (IIIe)

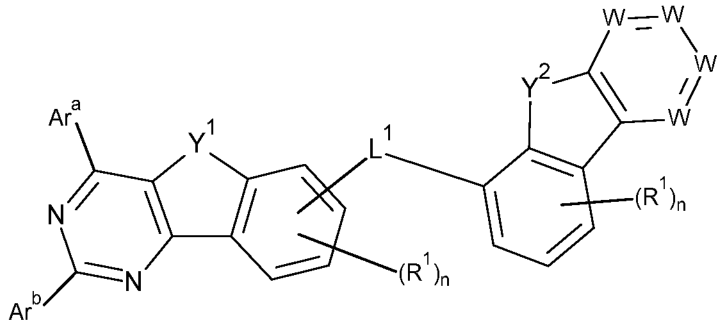

formule (Ie)

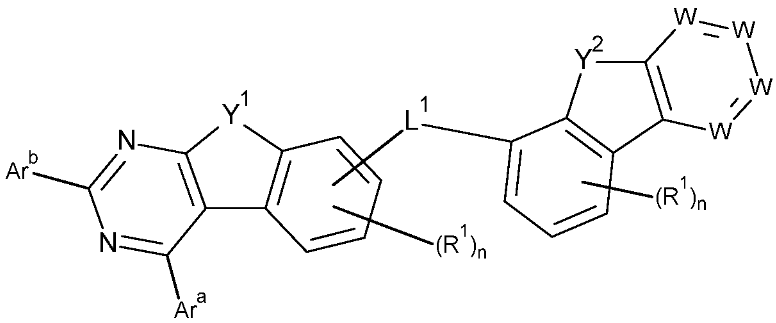

formule (IIe)

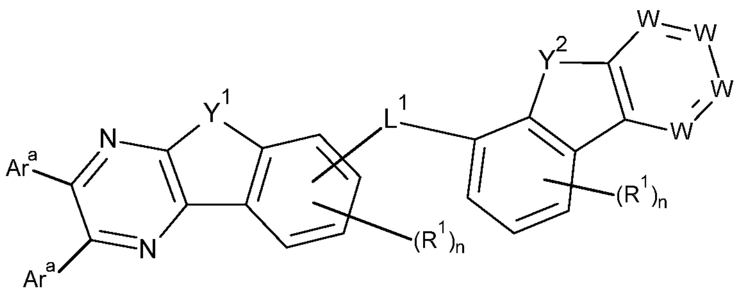

formule (IIIe)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule(s) (Ie) et/ou (IIe) ont la préférence.

9. Composé selon au moins l'une des revendications précédentes, **caractérisé en ce que** le composé comporte au moins l'une des structures des formules (If), (IIf) et/ou (IIIf)

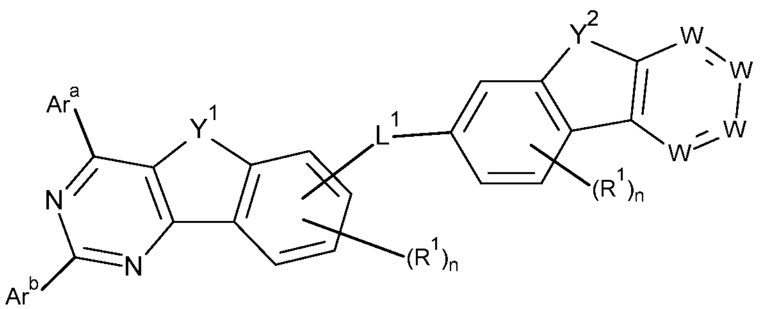

formule (If)

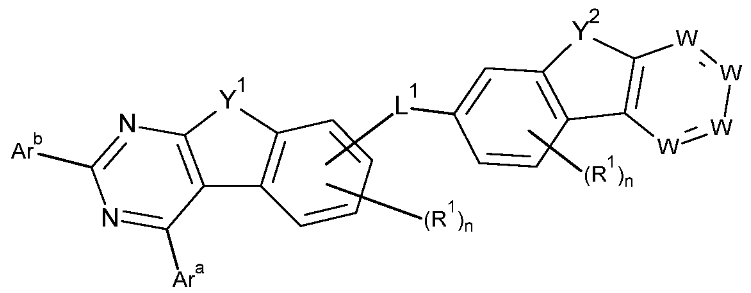

formule (IIf)

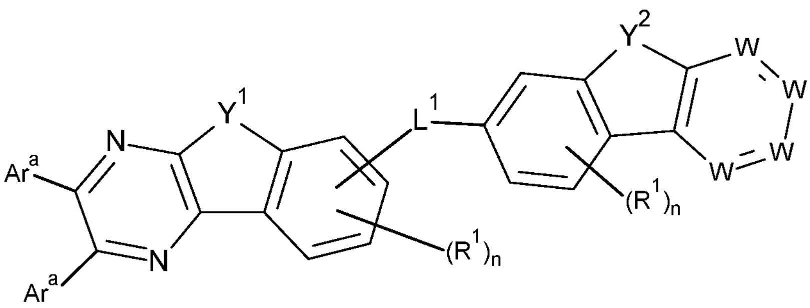

formule (IIIf)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule(s) (If) et/ou (IIf) ont la préférence.

**10.** Composé selon au moins l'une des revendications précédentes, **caractérisé en ce que** le composé comporte au moins l'une des structures des formules (Ig), (IIg) et/ou (IIIg)

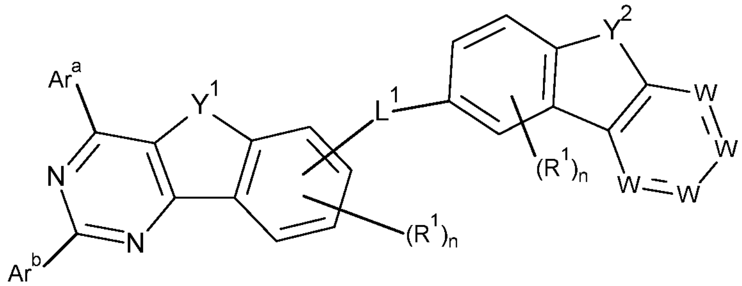

formule (Ig)

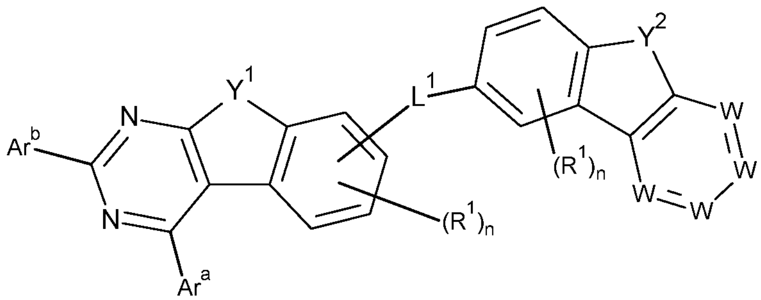

formule (IIg)

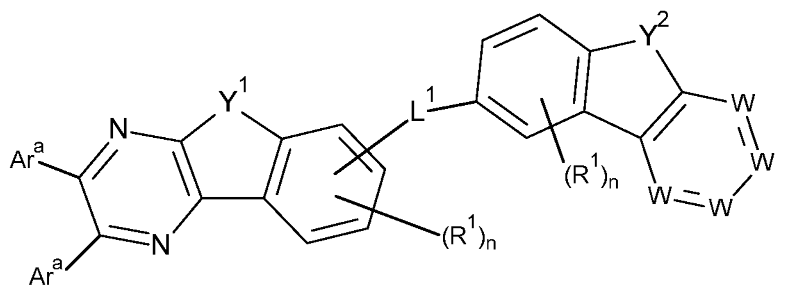

formule (IIIg)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule (Ig) et/ou (IIg) ont la préférence.

**11.** Composé selon au moins l'une des revendications précédentes, **caractérisé en ce que** le composé comporte au moins l'une des structures des formules (Ih), (IIh) et/ou (IIIh)

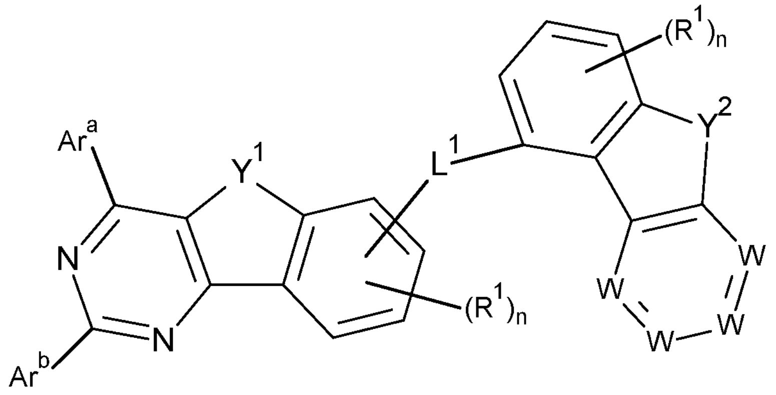

formule (Ih)

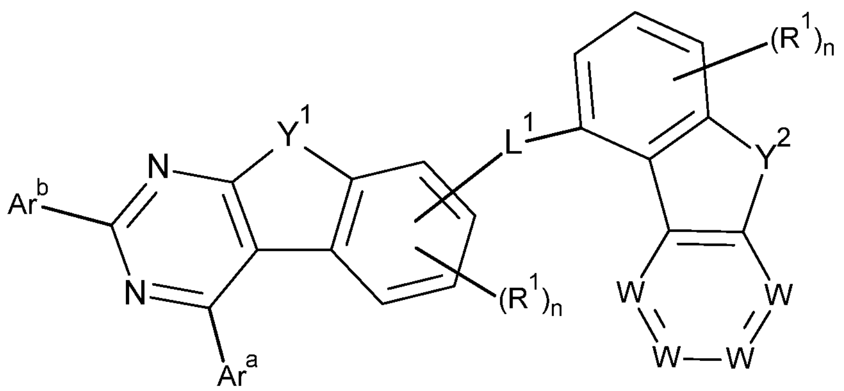

formule (IIh)

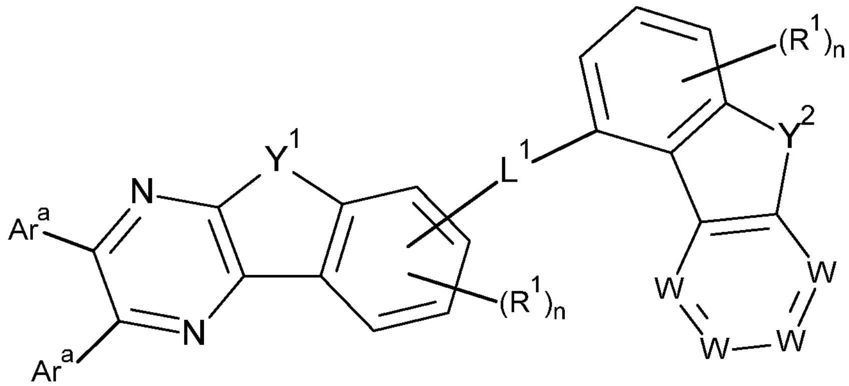

formule (IIIh)

dans lesquelles les symboles $Ar^a$, $Ar^b$, $Y^1$, $L^1$, $Y^2$, $R^1$, n et W présentent la signification qui a été indiquée selon la revendication 1 ou 2, où les structures des/de la formule(s) (Ih) et/ou (IIh) ont la préférence.

**12.** Composé selon au moins l'une des revendications précédentes, **caractérisé en ce que** le symbole $Y^2$ est $C(R^1)$ et $R^1$ est dans chaque cas, de manière identique ou différente, un système de cycle aromatique ou hétéroaromatique qui comporte de 5 à 40 atomes de cycle aromatique, lequel peut dans chaque cas être substitué par un radical ou par plusieurs radicaux $R^2$.

**13.** Composé selon au moins l'une des revendications précédentes 1 à 12, **caractérisé en ce que** le symbole $Y^2$ représente un groupe de la formule ($Y^2$-$_2$)

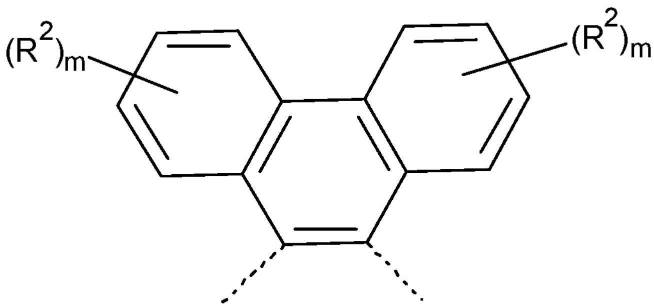

formule ($Y^2$-2)

dans laquelle les lignes en pointillés représentent les liaisons sur les atomes adjacents, $R^2$ présente la signification qui a été donnée selon la revendication 1 et m est 0, 1, 2, 3 ou 4.

**14.** Composé selon au moins l'une des revendications précédentes, **caractérisé en ce que** le groupe $Ar^b$ représente un groupe de la formule ($Ar^{b-1}$)

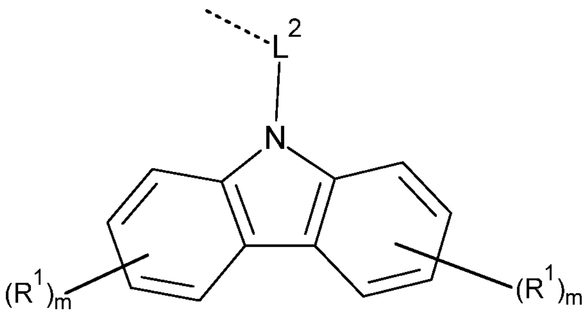

formule ($Ar^b$-1)

dans laquelle $L^2$ est une liaison ou un système de cycle aromatique ou hétéroaromatique qui comporte de 5 à 30 atomes de cycle aromatique, lequel peut être substitué par un radical ou par plusieurs radicaux $R^1$, le symbole $R^1$ présente la signification qui a été donnée selon la revendication 1, m est 0, 1, 2, 3 ou 4 et la ligne en pointillés représentent la liaison.

**15.** Oligomère, polymère ou dendrimère contenant un ou plusieurs composé(s) selon une ou plusieurs des revendications 1 à 14, dans lequel une ou plusieurs liaison(s) est/sont présente(s) depuis le composé jusqu'au polymère, jusqu'à l'oligomère ou jusqu'au dendrimère.

**16.** Composition comprenant au moins un composé selon une ou plusieurs des revendications 1 à 14 et/ou un oligomère, un polymère ou un dendrimère selon la revendication 15 et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

**17.** Formulation comprenant au moins un composé selon une ou plusieurs des revendications 1 à 14, un oligomère, un polymère ou un dendrimère selon la revendication 15 et/ou au moins une composition selon la revendication 16 et au moins un solvant.

**18.** Procédé pour la préparation d'un composé selon une ou plusieurs des revendications 1 à 14 ou d'un oligomère, d'un polymère ou d'un dendrimère selon la revendication 15, **caractérisé en ce qu'**un composé qui contient au moins un groupe diazadibenzofurane ou diazadibenzothiophène est lié à un groupe qui contient au moins un radical carbazole, fluorène, phénanthrène, benzofurane et/ou benzothiophène selon une réaction de couplage.

**19.** Utilisation d'un composé selon une ou plusieurs des revendications 1 à 14, d'un oligomère, d'un polymère ou d'un dendrimère selon la revendication 15 ou d'une composition selon la revendication 16 dans un dispositif électronique en tant que matériau hôte, matériau de matrice, matériau de transport d'électrons, matériau d'injection d'électrons et/ou matériau d'injection de trous et/ou matériau de blocage de trous.

**20.** Dispositif électronique contenant au moins un composé selon une ou plusieurs des revendications 1 à 14 ou un oligomère, un polymère ou un dendrimère selon la revendication 15 ou une composition selon la revendication 16, dans lequel le dispositif électronique est de préférence sélectionné parmi le groupe qui est constitué par les dispositifs électroluminescents organiques, les circuits intégrés organiques, les transistors à effet de champ organiques, les transistors à film mince organiques, les transistors à émission de lumière ou électroluminescents organiques, les cellules solaires organiques, les détecteurs optiques organiques, les photorécepteurs organiques, les dispositifs à extinction de champ organiques, les cellules électrochimiques à émission de lumière ou électroluminescentes et les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4539507 A
- US 5151629 A
- EP 0676461 A
- WO 9827136 A
- JP 5604848 B
- WO 2015182872 A1
- WO 2014157599 A1
- US 20150207082 A1
- JP 2011084531 A
- EP 3056498 A1
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- EP 13004411
- EP 14000345
- EP 14000417
- EP 14002623
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- EP 14002104
- WO 2010108579 A
- WO 2005053051 A
- WO 2009030981 A

**In der Beschreibung aufgeführte Nicht-Patentliteratur**


- **D. M. KOLLER et al.** *Nature Photonics,* 2008, 1-4
- **M. S. ARNOLD et al.** *Appl. Phys. Lett.,* 2008, vol. 92, 053301